# EUROPEAN PATENT APPLICATION

(11) **EP 4 786 487 A1**
(43) Date of publication of application: **05.08.2026**
(21) Application number: 24872502.0
(22) Date of filing: 27.09.2024
(51) Int. Cl.: C07K 7/08, A61K 38/10, A61K 38/12, A61P 21/00, A61P 43/00, C07K 7/64, C07K 14/47, C12Q 1/04

(54) **PEPTIDE**

(30) Priority: 29.09.2023 JP 2023170875
(71) Applicant: Peptidream Inc, Kawasaki-ku Kawasaki-shi, Kanagawa 210-0821 (JP)
(72) Inventor: OHUCHI, Masaki, Kawasaki-shi, Kanagawa 210-0821 (JP); KITAMURA, Hidetomo, Kawasaki-shi, Kanagawa 210-0821 (JP); KUWABARA, Naoyuki, Kawasaki-shi, Kanagawa 210-0821 (JP); KOGA, Hiroshi, Kawasaki-shi, Kanagawa 210-0821 (JP); SAWAI, Naoki, Kawasaki-shi, Kanagawa 210-0821 (JP); SHIMIZU, Hikaru, Kawasaki-shi, Kanagawa 210-0821 (JP); TAKUWA, Masatoshi, Kawasaki-shi, Kanagawa 210-0821 (JP); TAKEUCHI, Yui, Kawasaki-shi, Kanagawa 210-0821 (JP); FUNAKI, Yoichi, Kawasaki-shi, Kanagawa 210-0821 (JP); MASUDA, Yoshiaki, Kawasaki-shi, Kanagawa 210-0821 (JP); MASUYA, Keiichi, Kawasaki-shi, Kanagawa 210-0821 (JP); MURAKAMI, Masato, Kawasaki-shi, Kanagawa 210-0821 (JP); YAMADA, Masami, Kawasaki-shi, Kanagawa 210-0821 (JP)
(74) Representative: Mewburn Ellis LLP
(86) International application number: PCT/JP2024/034645
(87) International publication number: WO 2025/070720

(57) **Abstract**

The present invention relates to a peptide, a composition containing the peptide or the like. The present invention provides a peptide including the following amino acid sequence: X1-X2-X3-X4-X5-X6-X7-X8-X9-X10-X11-X12-X13-X14-X15 (SEQ ID NO: 2), wherein X1 is any amino acid or peptoid; X2 is any amino acid; X3 is an amino acid having an optionally substituted aromatic ring in a side chain; X4 is an amino acid having an optionally substituted aromatic ring in a side chain; X5 is an amino acid having an optionally substituted aromatic ring or optionally substituted cycloalkyl in a side chain; X6 is an amino acid having an alkyl group in a side chain; X7 is any secondary amino acid, N-alkylamino acid, or peptoid; X8 is any amino acid; X9 is an amino acid having an optionally substituted aromatic ring or an optionally substituted alkyl group or cycloalkyl group in a side chain; X10 is R; X11 is an amino acid having an optionally substituted aromatic ring or an optionally substituted alkyl group or cycloalkyl group in a side chain; X12 is an optionally substituted aliphatic amino acid or an amino acid having an optionally substituted alkyl chain in a side chain; X13 is any secondary amino acid, N-alkylamino acid, or peptoid; X14 is an amino acid having an optionally substituted aromatic ring in a side chain; and X15 is any amino acid.

## Description

### TECHNICAL FIELD

The present invention relates to a peptide, a composition containing the peptide, and use of the peptide.

### BACKGROUND ART

Myostatin is a secreted protein, namely a member of the transforming growth factor-β (TGF-β) superfamily, also called growth differentiation factor-8 (GDF-8). Like other members of the TGF-β family, myostatin is synthesized as a large precursor protein containing an N-terminal propeptide domain and a C-terminal domain, namely the active molecule. The two molecules of the myostatin precursor are covalently linked via a single disulfide bond present in the C-terminal growth factor domain. Active mature myostatin (a disulfide-linked homodimer consisting of C-terminal growth factor domains) is released from the myostatin precursor through multiple steps of proteolytic processing.

In the first step of the myostatin activation pathway, the peptide bond Arg266-Asp267 between the N-terminal propeptide domain and the C-terminal growth factor domain in both chains of the homodimer precursor is cleaved by a furin-type precursor protein convertase. However, the three resulting peptides (two propeptides and one mature myostatin (i.e., a disulfide-linked homodimer composed of growth factor domains)) remain associated, thereby forming a non-covalently linked, inactive complex called "latent myostatin". Mature myostatin can then be released from the latent myostatin via propeptide degradation. Members of the bone morphogenetic protein-1 (BMP-1) family of metalloproteinases cleave a peptide bond between Arg98 and Asp99 within the propeptide, concomitantly with the release of the active mature myostatin, which exists as a homodimer. In addition, the latent myostatin can also be activated *in vitro* by dissociating the complex with either acid or heat treatment (NPLs 2 and 3).

Myostatin is produced in striated muscle and is a factor that negatively regulates hypertrophy of striated muscle fibers. Myostatin knockout mice have been reported to have marked hypertrophy of skeletal muscle (NPL 1). The negative regulation of muscle hypertrophy is not only mediated by myostatin but also by GDF-11 (see NPL 4) and activin A (NPL 5), which also belong to the TGF-β superfamily. Myostatin exerts its effects through the transmembrane serine/threonine kinase heterotetrameric receptor family. Its activation is mediated by the serine/threonine kinase activity, which enhances receptor transphosphorylation. Activated forms of myostatin, GDF-11, and activin A, all bind to activin IIB-type receptor (ActRIIB) with high affinity. Next, low-affinity receptors such as activin-like kinase 4 (ALK4) or activin-like kinase 5 (ALK5) are in action and their transphosphorylation is activated. Subsequently, proteins Smad2 and Smad3 are phosphorylated in the cytoplasm, and after a complex is formed between them and Smad4, they migrate into the nucleus to express their target genes (NPL 6).

However, GDF-11 is known to be effective in improving muscle function as a rejuvenating factor (NPL 7), and activin acts on the pituitary gland to elicit an effect of promoting the production of follicle stimulating hormone (FSH) (NPL 8). Hypertrophy of striated muscle fibers is thus regulated by multiple factors with multiple functions. Therefore, it remains unclear which factor or factors regulate the control of muscle strength, which is an intrinsic function of striated muscle. Target molecules intended to ameliorate muscle weakness in muscular dystrophy, spinal muscular atrophy (SMA), sarcopenia, frailty, cachexia, stroke, and the like had also been unknown.

In particular, it has been reported that the blood concentration of GDF-11 increases with aging (NPL 9) and that it decreases (NPL 7). Thus, the role of GDF-11 in the regulation of muscle strength was not clear.

However, it has recently been demonstrated that an anti-latent myostatin antibody that specifically inhibits myostatin signaling exhibits a greater improvement in muscle strength in a Duchenne muscular dystrophy model mouse than antibodies or proteins that also inhibit GDF-11 and/or activin, and that administration of GDF-11 to a muscle atrophy model mouse resulted in an improvement in muscle strength. These experimental results suggest that the specific inhibition of myostatin by the anti-latent myostatin antibody is a useful therapeutic strategy in the reduced muscle strength conditions (NPL 10). Similarly, an anti-promyostatin antibody, which specifically inhibits myostatin signaling, had an additive effect on the improvement of muscle strength by using a low-molecular-weight drug that modifies SMN2 gene splicing in an SMA model mouse. This has indicated that selective inhibition of myostatin by anti-promyostatin antibody is useful for increasing muscle strength in SMA (NPL 11).

Based on these findings, selective myostatin signaling inhibitors have been created using antibodies such as those described above. However, IgG and other antibodies are known to be hardly distributed in muscle interstitial fluid (NPLs 12 and 13). The mode of action of myostatin in the body is thought to be mediated by autocrine, paracrine, and endocrine mechanisms, but mostly to autocrine and paracrine mechanisms in muscle tissue (NPL 16). It has also been reported that clathrin-dependent early endosomes are involved in TGF-β superfamily-mediated Smad signaling (NPLs 14 and 15).

However, no pharmaceutical composition that inhibits myostatin signaling not only extracellularly but also intracellularly has been developed.

### CITATION LIST

### NON PATENT LITERATURE

NPL 1: McPherron et al., Nature 1997; 387(6628): 83-90.
NPL 2: Szlama et al., FEBS J 280(16): 3822-3839 (2013)
NPL 3: Lee, PloS One 3(2): e1628 (2008)
NPL 4: Hammers et al., EMBO Mol Med. 2017; 9(4): 531-44.
NPL 5: Latres et al., Nat Commun.2017; 8: 15153.
NPL 6: Lee, J Clin Invest. 2021; 131(9): e148372.
NPL 7: Sinha et al., Science 2014; 344(6184): 649-52.
NPL 8: Bloise et al., Physiol Rev. 2019; 99(1): 739-80.
NPL 9: Egerman et al., Cell Metab. 2015; 22(1): 164-74.
NPL 10: Muramatsu et al., Sci Rep. 2021; 11(1): 2160.
NPL 11: Long et al., Hum Mol Genet. 2019; 28(7): 1076-89.
NPL 12: Jadhav et al., J Pharm Sci. 2017; 106(9): 2853-59.
NPL 13: Wiig et al., Am J Physiol Heart Circ Physiol. 2001; 280(4): H1505-12.
NPL 14: Derynck et al., Sci Signal. 2019; 12(570): 1-58.
NPL 15: Chen et al., Cell Res. 2009; 19: 58-70.
NPL 16: Lee et al., J Appl Physiol (1985). 2016; 120(6): 592-8.
NPL 17: Bulfield et al., Proc Natl Acad Sci U S A. 1984; 81: 1189-92.
NPL 18: Fukada et al., Am J Pathol. 2010; 176(5): 2414-24.
NPL 19: van Putten et al., FASEB J. 2019; 33(7): 8110-24.

### SUMMARY OF INVENTION

### TECHNICAL PROBLEM

If myostatin signaling can be selectively inhibited not only extracellularly but also intracellularly, for example, in both the muscle interstitial fluid and the cytoplasm, a higher inhibitory effect on myostatin signaling than that achieved by antibodies can be expected. If a compound exhibiting such an effect can be obtained, it is expected to have a greater muscle strength-enhancing effect than compounds or antibodies previously developed, and to serve as a therapeutic agent for the treatment of a disease or symptom associated with reduced muscle function, including, for example, muscular dystrophy and spinal muscular atrophy (SMA).

The present inventors have conducted intensive research to create a compound with myostatin inhibitory activity, and as a result, have conceived the present invention. The present invention provides a novel peptide with myostatin inhibitory activity, a composition containing the peptide, and use of the peptide.

### SOLUTION TO PROBLEM

Although not limited, the present application includes the following items of the invention.
[1] A peptide including the following amino acid sequence:
   X1-X2-X3-X4-X5-X6-X7-X8-X9-X10-X11-X12-X13-X14-X15 (SEQ ID NO: 2); or a pharmaceutically acceptable salt thereof,
   Wherein
   X1 is any amino acid or peptoid;
   X2 is any amino acid;
   X3 is an amino acid having an optionally substituted aromatic ring in a side chain;
   X4 is an amino acid having an optionally substituted aromatic ring in a side chain;
   X5 is an amino acid having an optionally substituted aromatic ring or optionally substituted cycloalkyl in a side chain;
   X6 is an amino acid having an alkyl group in a side chain;
   X7 is any secondary amino acid, N-alkylamino acid, or peptoid;
   X8 is any amino acid;
   X9 is an amino acid having an optionally substituted aromatic ring or an optionally substituted alkyl group or cycloalkyl group in a side chain;
   X10 is R;
   X11 is an amino acid having an optionally substituted aromatic ring or an optionally substituted alkyl group or cycloalkyl group in a side chain;
   X12 is an optionally substituted aliphatic amino acid or an amino acid having an optionally substituted alkyl chain in a side chain;
   X13 is any secondary amino acid, N-alkylamino acid, or peptoid;
   X14 is an amino acid having an optionally substituted aromatic ring in a side chain; and
   X15 is any amino acid.
[2] The peptide or pharmaceutically acceptable salt thereof according to [1], wherein the following one or two or more requirements are satisfied:
   X1 is an amino acid having an optionally substituted alkyl group in a side chain or a peptoid having an optionally substituted alkyl group in a side chain attached to amide nitrogen;
   X3 is optionally substituted F or W;
   X4 is optionally substituted F or Y, or naphthylalanine;
   X5 is optionally substituted F, Y or naphthylalanine, or optionally substituted cyclohexylalanine;
   X6 is an amino acid having a branched alkyl group, or an amino acid having a cycloalkyl group, wherein a carbon atom contained therein is optionally replaced by an oxygen atom;
   X8 is an amino acid having an optionally substituted aromatic ring or an optionally substituted alkyl group or cycloalkyl in a side chain;
   X9 is optionally substituted F or Y, or an amino acid containing an optionally substituted heterocycloalkyl group in a side chain, or alanine;
   X12 is an aliphatic amino acid containing three or more carbon atoms in a side chain, or an amino acid having an optionally substituted alkyl chain in a side chain;
   X13 is an N-alkylamino acid;
   X14 is optionally substituted F or Y; and
   X15 is an amino acid having a sulfur (S) element at a side chain terminus.
[3] The peptide or pharmaceutically acceptable salt thereof according to [1], wherein the following one or two or more requirements are satisfied:
   X1 is an amino acid having an optionally substituted alkyl group in a side chain or a peptoid having an optionally substituted alkyl group in a side chain attached to amide nitrogen;
   X3 is optionally substituted F or W;
   X4 is optionally substituted F or Y, or naphthylalanine;
   X5 is optionally substituted F, Y or naphthylalanine, or optionally substituted cyclohexylalanine;
   X6 is an amino acid having a branched alkyl group, or an amino acid having a cycloalkyl group, wherein a carbon atom contained therein is optionally replaced by an oxygen atom;
   X8 is an amino acid having an optionally substituted aromatic ring or an optionally substituted alkyl group or cycloalkyl in a side chain;
   X9 is optionally substituted F or Y, or an amino acid containing an optionally substituted heterocycloalkyl group in a side chain, or alanine;
   X12 is an aliphatic amino acid containing three or more carbon atoms in a side chain, or an amino acid having an optionally substituted alkyl chain in a side chain;
   X13 is an N-alkylamino acid;
   X14 is optionally substituted F or Y; and
   X15 is an aliphatic amino acid, and is further attached to an aliphatic amino acid or proline as X16.
[4] The peptide or pharmaceutically acceptable salt thereof according to [1], wherein the following one or two or more requirements are satisfied:
   X1 is A, MeA, Dap, ApG, CrpG, CmG, MeD, N, CrmG, MeopG, MeeG, AcapG, A4paa, KCOpipzaa, CeG, CrbG, K, KMe, Hly, E, Hgl, Orn, Dab, MeE, D, or MeHgl;
   X2 is R, Dap, K, E, A, da, S, G, de, dkCOpipzaa, ddab, or dorn;
   X3 is W, W7N, F44Py, Bph, W6N, F41dMeCmm4Pyz, W1Bn, W6H, W1Ph, Bph4ms, W1aa, W7Ph3C, W1Ph4OMe, W1Ph4COO, F42Py, or W6H1Ph4COO;
   X4 is Y, 3Py6NH2, YBn, Yae, Nal1, Yph, or mBph;
   X5 is MeF, MeY, MeF3Me, F, MeCha, MeYap, MeA1Ac4pip, MemBph, MeNal1, MeNal2, MeF4OMe, or MeF4C;
   X6 is I, Gcpe, or TMe;
   X7 is MeG, MeA, Meda, dp, EtG, or PrG;
   X8 is Y, F4COO, K, 4Py, F, F3aao, F3OMe, A4paa, KCOpipzaa, Bph2C, Bph2OMe, or pBph2aao;
   X9 is Y, F4COO, 3Py, 4Py, F4aao, F4CON, F3CON, F4F, or F4OMe;
   X11 is MeF, Me4Py, Me3Py, F, Cha, MeCha, MeK, MeKMe, MeF4am, MeD, MeE, MeF4COO, or F4am;
   X12 is L, Ahp, Ano, Ado, Ade, Aoc, SPent, HseBu, AhpOMe, HsePr, or Aun;
   X13 is MeA, MeE, MeKCOpipzaa, MeK, MeDap, or MeDab;
   X14 is MeF, MeF3Et, Me4Py, Me3Py, or MemBph; and
   X15 is C, MeC, dc, Medc, dhcy, Hcy, or de.
[5] The peptide or pharmaceutically acceptable salt thereof according to [1], wherein the following one or two or more requirements are satisfied:
   X1 is A, MeA, Dap, ApG, CrpG, CmG, MeD, N, CrmG, MeopG, MeeG, AcapG, A4paa, KCOpipzaa, CeG, CrbG, K, KMe, Hly, E, Hgl, Orn, Dab, MeE, D, or MeHgl;
   X2 is R, Dap, K, E, A, da, S, G, de, dkCOpipzaa, ddab, or dorn;
   X3 is W, W7N, F44Py, Bph, W6N, F41dMeCmm4Pyz, W1Bn, W6H, W1Ph, Bph4ms, W1aa, W7Ph3C, W1Ph4OMe, F42Py, W1Ph4COO, or W6H1Ph4COO;
   X4 is Y, 3Py6NH2, YBn, Yae, Nal1, Yph, or mBph;
   X5 is MeF, MeY, MeF3Me, F, MeCha, MeYap, MeA1Ac4pip, MemBph, MeNall, MeNal2, MeF4OMe, or MeF4C;
   X6 is I, Gcpe, or TMe;
   X7 is MeG, MeA, Meda, dp, EtG, or PrG;
   X8 is Y, F4COO, K, 4Py, F, F3aao, F3OMe, A4paa, KCOpipzaa, Bph2C, Bph2OMe, or pBph2aao;
   X9 is Y, F4COO, 3Py, 4Py, F4aao, F4CON, F3CON, F4F, or F4OMe;
   X11 is MeF, Me4Py, Me3Py, F, Cha, MeCha, MeK, MeKMe, MeF4am, MeD, MeE, MeF4COO, or F4am;
   X12 is L, Ahp, Ano, Ado, Ade, Aoc, SPent, HseBu, AhpOMe, HsePr, or Aun;
   X13 is MeA, MeE, MeKCOpipzaa, MeK, MeDap, or MeDab;
   X14 is MeF, MeF3Et, Me4Py, Me3Py, or MemBph; and
   X15 is Aeoac, Ape, dhgl, Hgl, MeHgl, Medhgl, MeAeoac, or MeApe, or X15 is G, da, A, or de and is attached to MeA, Meda, G, da, MeG, P, or A as X16.
[6] The peptide or pharmaceutically acceptable salt thereof according to [1], wherein the following one or two requirements are satisfied:
   X1 is A, MeA, Dap, ApG, CrpG, CmG, MeD, N, CrmG, MeopG, MeeG, AcapG, A4paa, KCOpipzaa, CeG, CrbG, K, KMe, Hly, E, Hgl, Orn, Dab, MeE, D, or MeHgl; and
   X15 is Aeoac, Ape, dhgl, Hgl, MeHgl, Medhgl, MeAeoac, or MeApe, or X15 is G, da, A, or de and is attached to MeA, Meda, G, da, MeG, P, or A as X16.
[7] The peptide or pharmaceutically acceptable salt thereof according to [1], the peptide including or consisting of an amino acid sequence set forth in any one of SEQ ID NOs: 2 to 368.
[8] A peptide including the following amino acid sequence:
   A-R-W-Y-MeF-I-MeG-Y-Y-R-MeF-L-MeA-MeF-C (SEQ ID NO: 1)
   Or
   an amino acid sequence having a substitution, addition, deletion, or insertion in 1 to 14 amino acid residues selected from the group consisting of amino acid residues at positions 1, 2, 3, 4, 5, 6, 7, 8, 9, 11, 12, 13, 14, and 15 in the above amino acid sequence; or a pharmaceutically acceptable salt thereof.
[9] The peptide or pharmaceutically acceptable salt thereof according to any one of [1] to [8], wherein the peptide is a cyclic peptide.
[10] The peptide or pharmaceutically acceptable salt thereof according to any one of [1], [2], [4], [6], [7], and [8], which has a cyclic structure in which a chloroacetylated amino acid is bonded to a cysteine residue included in the peptide.
[11] The peptide or pharmaceutically acceptable salt thereof according to any one of [1], [3], [5], [6], and [7], which has a cyclic structure in which an amino group of an amino acid residue at position 1 included in the peptide is bonded to a carboxy group of an amino acid residue at position 15.
[12] The peptide or pharmaceutically acceptable salt thereof according to any one of [1], [3], [5], [6], and [7], which has a cyclic structure in which an amino group of an amino acid residue at position 1 included in the peptide is bonded to a carboxy group of an amino acid residue at position 16.
[13] The peptide or pharmaceutically acceptable salt thereof according to [9], wherein the peptide further includes a crosslinking structure.
[14] The peptide or pharmaceutically acceptable salt thereof according to [9], which has a crosslinking structure between any of X1, X2, or X3 and X11 or X13.
[15] The peptide or pharmaceutically acceptable salt thereof according to [12], the peptide including or consisting of an amino acid sequence set forth in any one of SEQ ID NOs: 238-290, 326-329, 334-338, 348-354, 358-360, 362-366, and 368.
[16] The peptide or pharmaceutically acceptable salt thereof according to any one of [1] to [8], further including an additional amino acid residue.
[17] The peptide or pharmaceutically acceptable salt thereof according to any one of [1] to [8], including a linker at a C-terminus thereof.
[18] A pharmaceutical composition including the peptide or pharmaceutically acceptable salt thereof according to any one of [1] to [8].
[19] The pharmaceutical composition according to [18], having myostatin inhibitory activity.
[20] The pharmaceutical composition according to [18], for preventing or treating a myostatin-related disease or a disease or symptom associated with reduced muscle function.
[21] A method of inhibiting myostatin signaling, including using the peptide or pharmaceutically acceptable salt thereof according to any one of [1] to [8].
[22] A diagnostic composition including the peptide or pharmaceutically acceptable salt thereof according to any one of [1] to [8], for diagnosing a myostatin-related disease or a disease or symptom associated with reduced muscle function.
[23] A method of testing a peptide or a pharmaceutically acceptable salt thereof, including testing at least one of
   a) solubility in a solvent,
   b) myostatin inhibitory activity,
   c) cell and/or tissue toxicity, or
   d) toxicity to an experimental animal,
   wherein the peptide or pharmaceutically acceptable salt thereof is the peptide or pharmaceutically acceptable salt thereof according to any one of [1] to [8].

### ADVANTAGEOUS EFFECTS OF INVENTION

The peptide of the present invention has myostatin inhibitory activity, and is thus useful as a pharmaceutical composition for preventing or treating a disease or symptom associated with reduced muscle function, a diagnostic composition, and a research composition.

### BRIEF DESCRIPTION OF DRAWINGS

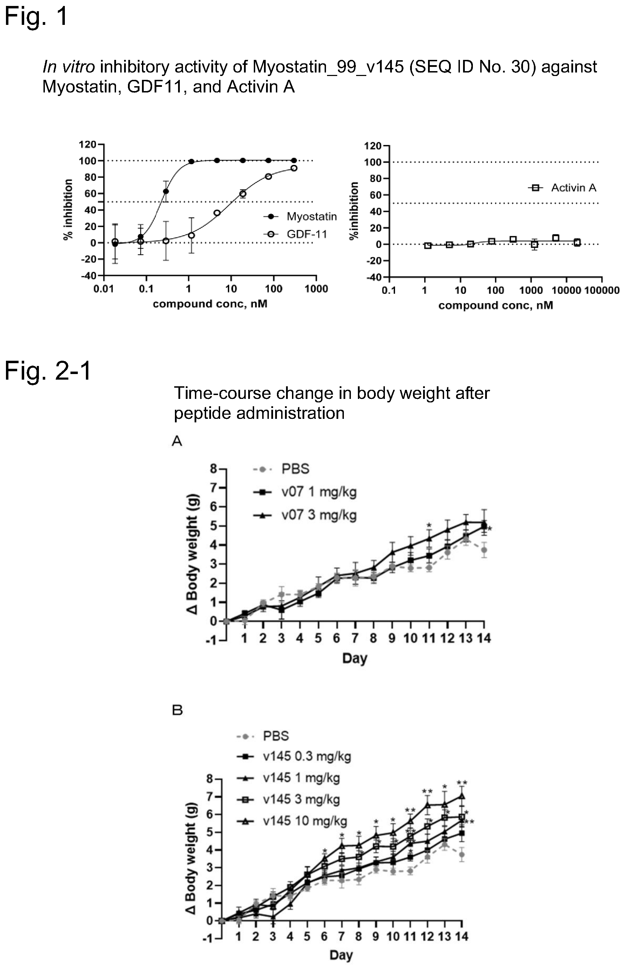

[Fig. 1] Fig. 1 is graphs showing the results of the *in vitro* inhibitory activity of Myostatin_99_v145 (SEQ ID NO: 30) against myostatin, GDF-11, and activin A as measured by a reporter gene assay for myostatin signaling in HEK-Blue (trademark) TGF-β Cells. (A) The results of inhibitory activity against myostatin and GDF-11, and (B) the results of the activity against activin A. Black circles indicate the results of activity against myostatin, white circles indicate the results of activity against GDF-11, and white squares indicate the results of activity against activin A. Note that the abscissa indicates the concentration of peptide (nM), and the ordinate indicates the % inhibition, where the unstimulated state is 0% inhibition and the maximum inhibitory activity exerted by the peptide is 100% inhibition.

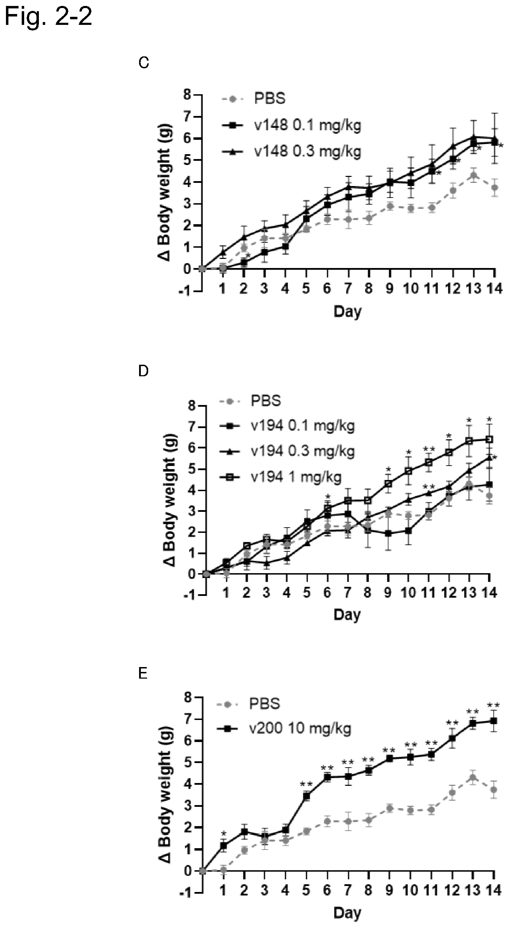

[Fig. 2-1] Fig. 2 is graphs showing the time-course changes in body weight of wild-type mice during the administration of each peptide of the present invention. The black squares in A show the results of administration of 1 mg/kg of Myostatin_99_Variant_07 (SEQ ID NO: 3), the black triangles show the results of administration of the same peptide at 3 mg/kg, and the gray circles show the results of administration of PBS as a control, respectively. The black squares, black triangles, white squares, and white triangles in B show the results of administration of the peptide Myostatin_99_v145 (SEQ ID NO: 30) at 0.3 mg/kg, 1 mg/kg, 3 mg/kg, and 10 mg/kg, respectively, and the gray circles show the results of administration of PBS as a control. The abscissa indicates the number of days after administration, and the ordinate indicates the change in body weight (grams) of the mice after administration.
[Fig. 2-2] Fig. 2 is graphs showing the time-course changes in body weight of wild-type mice during the administration of each peptide of the present invention. The black squares and black triangles in C show the results of administration of Myostatin_99_v148 (SEQ ID NO: 31) at 0.1 mg/kg and 0.3 mg/kg, respectively, and the gray circles show the results of administration of PBS as a control. The black squares, black triangles, and white squares in D show the results of administration of Myostatin_99_v194 (SEQ ID NO: 65) at 0.1 mg/kg, 0.3 mg/kg, and 1 mg/kg, respectively, and the gray circles show the results of administration of PBS as a control. The black squares in E show the results of administration of Myostatin_99_v200 (SEQ ID NO: 71) at 10 mg/kg, and the gray circles show the results of administration of PBS as a control. The abscissa indicates the number of days after administration, and the ordinate indicates the change in body weight (grams) of the mice after administration.

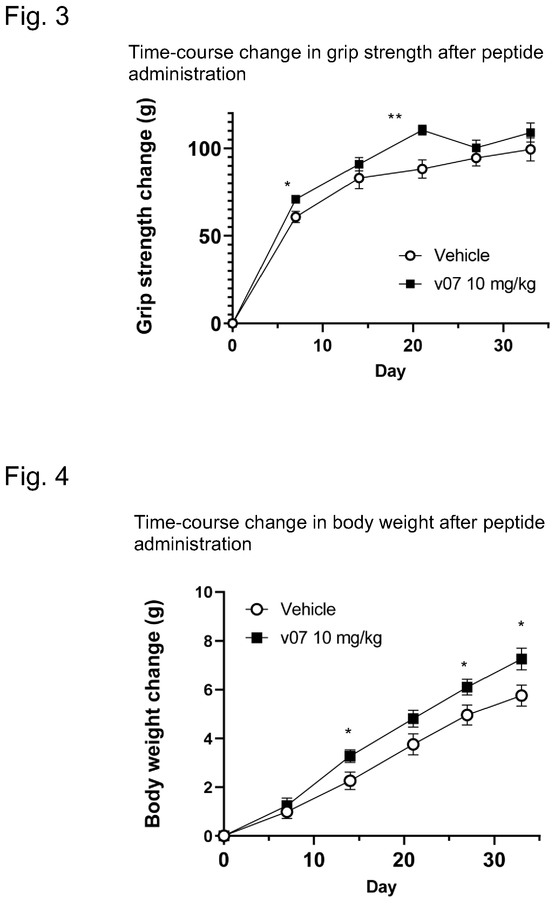

[Fig. 3] Fig. 3 is a graph showing the time-course improvements in grip strength and changes in body weight of B10 mdx mice during the administration of the peptide of the present invention. The black squares show the results of administration of Myostatin_99_Variant_07 (SEQ ID NO: 3) at 10 mg/kg, and the white circles show the results of administration of PBS as a control. The abscissa indicates the number of days after administration, and the ordinate indicates the change in grip strength.
[Fig. 4] Fig. 4 is a graph showing the time-course changes in body weight of B10 mdx mice during the administration of the peptide of the present invention. The black squares show the results of administration of Myostatin_99_Variant_07 (SEQ ID NO: 3) at 10 mg/kg, and the white circles show the results of administration of PBS as a control. The abscissa indicates the number of days after administration, and the ordinate indicates the change in body weight.

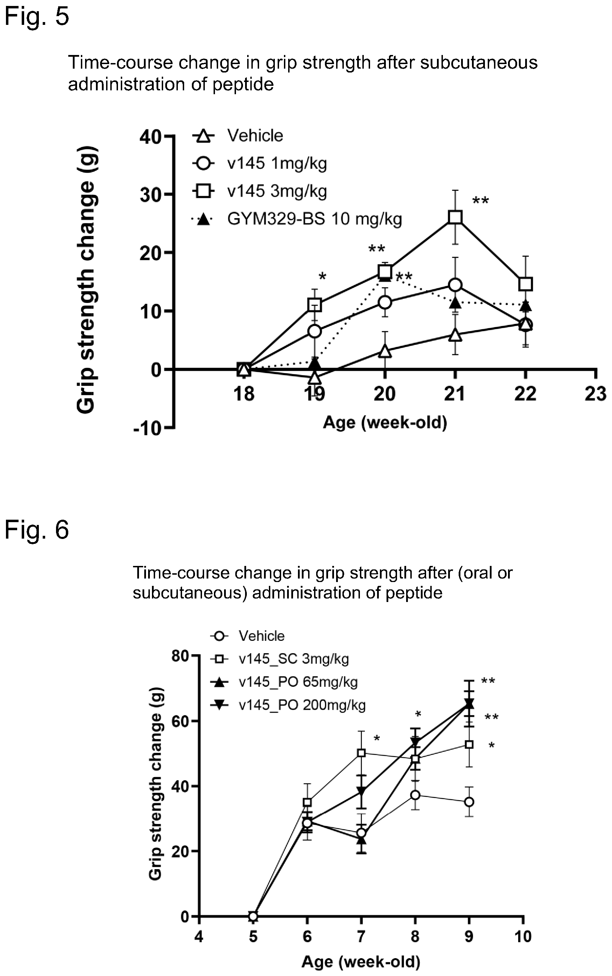

[Fig. 5] Fig. 5 is a graph showing the time-course changes in body weight of DBA/2 mdx mice during the administration of the peptide of the present invention or GYM329-BS. The white circles and the white squares show the results of administration of Myostatin_99_v145 (SEQ ID NO: 30) at 1 mg/kg and 3 mg/kg, respectively, the black triangles show the results of administration of GYM329-BS at 10 mg/kg, and the white triangles show the results of administration of PBS as a control. The abscissa indicates the age (week-old) of mice, and the ordinate indicates the change in grip strength.
[Fig. 6] Fig. 6 is a graph showing the time-course changes in body weight of DBA/2 mdx mice during the administration of the peptide of the present invention or GYM329-BS. The white circles show the results of subcutaneous administration of Myostatin_99_v145 (SEQ ID NO: 30) at 3 mg/kg, the black triangles show the results of oral administration of the same peptide at 65 mg/kg, and the black inverted triangles show the results of oral administration of the same peptide at 200 mg/kg. The abscissa indicates the age (week-old) of mice, and the ordinate indicates the change in grip strength.

### DESCRIPTION OF EMBODIMENTS

Although not limited, the present invention includes the following embodiments. Unless otherwise indicated herein, technical and scientific terms used herein have the same meanings as those ordinarily understood by those skilled in the art. The substances, materials, and examples disclosed herein are merely illustrative and not intended to be limiting. Reference to "in one embodiment" herein means that it is not limited to the embodiment, i.e., it is not limited.

### 1. Abbreviations

As used herein, the following abbreviations are used with the following meanings unless otherwise indicated.

### Abbreviations (general)

Å: angstrom (unit);
ClAc: chloroacetyl;
Cy5SAlk: sulfo-Cy5-alkyne;
DCM: dichloromethane;
TIPS: triisopropylsilyl;
tBu: tert-butyl;
DTT: dithiothreitol;
DMSO: dimethyl sulfoxide;
Trt: trityl;
Boc: *tert*-butoxycarbonyl;
DMF: N,N-dimethylformamide;
DIEA or DIPEA: N,N-diisopropylethylamine;
DIPCI or DIC: N,N'-diisopropylcarbodiimide;
Oxyma pure: ethyl cyano(hydroxyimino)acetate;
DODT: 3,6-dioxa-1,8-octane-dithiol;
Fmoc: 9-fluorenylmethyloxycarbonyl;
g: gram (unit);
HATU: O-(7-azabenzotriazol-1-yl)-N,N,N',N'-tetramethyluronium hexafluorophosphate;
HOSu: N-Hydroxysuccinimide;
HPLC: high-performance liquid chromatography;
LC-MS or LC/MS: liquid chromatography mass spectrometer;
mL: milliliter (unit);
M: molar (unit);
µL: microliter (unit);
mM: millimolar (unit);
µM: micromolar (unit);
mmol: millimole (unit);
mg: milligram (unit);
MeCN or CH₃CN: acetonitrile;
min: minute(s) (unit) min;
mm: millimeter (unit);
µm: micrometer (unit);
nm: nanometer (unit);
nM: nanomolar (unit);
OSu: succinimide;
PEG: polyethylene glycol;
rpm: revolutions per min (unit);
tBu: *tert*-butyl;
TFA: trifluoroacetic acid;
TIS: triisopropylsilane;
Trt or Tr: trityl group;
H-PEG4Me: 2,5,8,11-tetraoxatridecan-13-amine;
H-PEG8Me: 2,5,8,11,14,17,20,23-octaoxapentacosan-25-amine;
AA: amino acid;
PyAOP: 7-((azabenzotriazol-1-yloxy)tripyrrolidinophosphonium hexafluorophosphate;
CSA: 10-camphorsulfonic acid;
Fmoc-OSu: N-(9-fluorenylmethoxycarbonyloxy)succinimide;
THF: tetrahydrofuran;
ClAcOSu: N-(chloroacetoxy)succinimide;
Pd₂(dba)₃·CHCl₃: tris(dibenzylideneacetone)dipalladium(0)-chloroform complex;
SPhos: 2-dicyclohexylphosphino-2',6'-dimethoxybiphenyl;
EDCI·HCl: 1-(3-dimethylaminopropyl)-3-ethylcarbodiimide hydrochloride;
Pd(PPh₃)₄: tetrakis(triphenylphosphine)palladium(0);
ClAcOH: chloroacetic acid;
conc: concentration;
HFIP: 1,1,1,3,3,3-hexafluoro-2-propanol;
Pbf: 2,2,4,6,7-pentamethyldihydrobenzofuran-5-sulfonyl;
Alloc: allyloxycarbonyl;
Boc-NH-PEG9-NH₂: *tert*-butyl (29-amino-3,6,9,12,15,18,21,24,27-nonaoxanonacosyl)carbamate;
Biotin-OSu: 2,5-dioxopyrrolidin-1-yl 5-((3aS,4S,6aR)-2-oxohexahydro-1H-thieno[3,4-d]imidazol-4-yl)pentanoate; and
TEAA: triethylammonium acetate.

### Abbreviations (non-natural amino acids)

The abbreviations below for non-natural amino acids include those in which the amino group on the main chain is protected by a common protecting group such as a Boc or Fmoc group.
MeA: (2S)-2-(methylamino)propanoic acid (CAS 3913-67-5)
Dap: (2S)-2,3-diaminopropanoic acid (CAS 4033-39-0)
ApG: 2-[(3-aminopropyl)amino]acetic acid (CAS 2875-41-4)
CrpG: 4-((carboxymethyl)amino)butanoic acid (CAS 4386-04-3)
CmG: 2-[(2-carbamoylethyl)amino]acetic acid (CAS 34299-32-6)
MeD: (2S)-2-(methylamino)butanedioic acid (CAS 4226-18-0)
CrmG: 2,2'-iminodiacetic acid (CAS 142-73-4)
MeopG: 2-[(3-methoxypropyl)amino]acetic acid (CAS 807261-81-0)
MeeG: 2-[(2-methoxyethyl)amino]acetic acid (CAS 205124-55-6)
AcapG: 2-[(3-acetamidopropyl)amino]acetic acid (CAS 1862908-81-3)
A4paa: (S)-2-amino-3-(1-(carboxymethyl)piperidin-4-yl)propanoic acid 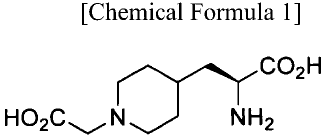
KCOpipzaa: (2S)-2-amino-6-{[4-(carboxymethyl)piperazin-1-carbonyl]amino}hexanoic acid 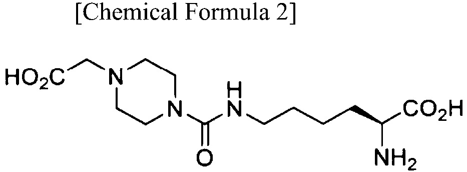
CeG: 3-((carboxymethyl)amino)propanoic acid (CAS 505-72-6)
CrbG: 5-((carboxymethyl)amino)pentanoic acid (CAS 72253-27-1)
KMe: (2S)-2-amino-6-(methylamino)hexanoic acid (CAS 1188-07-4)
Hly: (2S)-2,7-diaminoheptanoic acid (CAS 37689-89-7)
Hgl: (S)-2-aminohexanedioic acid (CAS 1118-90-7)
Orn: (2S)-2,5-diaminopentanoic acid (CAS 70-26-8)
Dab: (2S)-2,4-diaminobutanoic acid (CAS 1758-80-1)
MeE: (2S)-2-(methylamino)pentanedioic acid (CAS 6753-62-4)
da: D-alanine (CAS 338-69-2)
Aib: 2-amino-2-methylpropanoic acid (CAS 62-57-7)
de: D-glutamic acid (CAS 6893-26-1)
dkCOpipzaa: (2R)-2-amino-6-{[4-(carboxymethyl)piperazin-1-carbonyl]amino}hexanoic acid 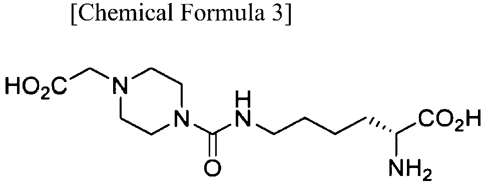
ddab: (2R)-2,4-diaminobutanoic acid (CAS 26908-94-1)
dorn: (2R)-2,5-diaminopentanoic acid (CAS 348-66-3)
MeW: (S)-3-(1H-indol-3-yl)-2-(methylamino)propanoic acid (CAS No. 526-31-8)
W7N: (S)-2-amino-3-(1H-pyrrolo[2,3-b]pyridin-3-yl)propanoic acid (CAS 49758-35-2)
W4C: (S)-2-amino-3-(4-chloro-1H-indol-3-yl)propanoic acid (CAS 52448-14-3)
Bph: (S)-3-([1,1'-biphenyl]-4-yl)-2-aminopropanoic acid (CAS 155760-02-4)
3Py6Ph: (S)-2-amino-3-(6-phenylpyridin-3-yl)propanoic acid 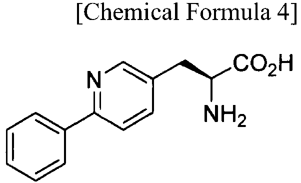
W6N: (S)-2-amino-3-(1H-pyrrolo[2,3-c]pyridin-3-yl)propanoic acid (CAS 149704-63-2)
F41dMeCmm4Pyz: (S)-2-amino-3-(4-(1-(2-(dimethylamino)-2-oxoethyl)-1H-pyrazol-4-yl)phenyl)propanoic acid 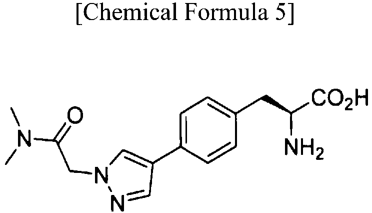
W1Bn: (2S)-2-amino-3-(1-benzyl-1H-indol-3-yl)propanoic acid (CAS 740-11-4)
W6H: (S)-2-amino-3-(6-hydroxy-1H-indol-3-yl)propanoic acid (CAS 13567-14-1)
Bph4ms: (S)-2-amino-3-(4'-(methylsulfonyl)-[1,1'-biphenyl]-4-yl)propanoic acid 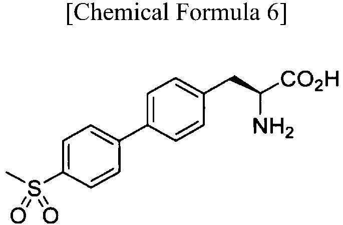
W1Ph: (2S)-2-amino-3-(1-phenyl-1H-indol-3-yl)propanoic acid (CAS 1224881-37-1)
W1aa: (2S)-2-amino-3-[1-(carboxymethyl)-1H-indole-3-yl]propanoic acid (CAS 773823-50-0)
W7Ph3C: (S)-2-amino-3-(7-(3-chlorophenyl)-1H-indol-3-yl)propanoic acid 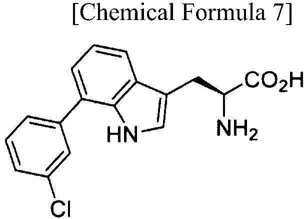
W1Ph4OMe: (2S)-2-amino-3-(1-(4-methoxyphenyl)-1H-indole-3-yl)propanoic acid 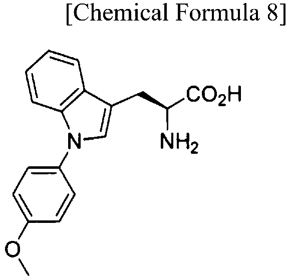
W1Ph4COO: 4-{3-[(2S)-2-amino-2-carboxyethyl]-1H-indole-1-yl}benzoic acid 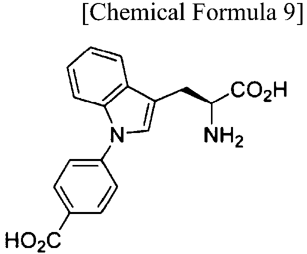
W6H1Ph4COO: (S)-4-(3-(2-amino-2-carboxyethyl)-6-(hydroxy-1H-indol-1-yl)benzoic acid 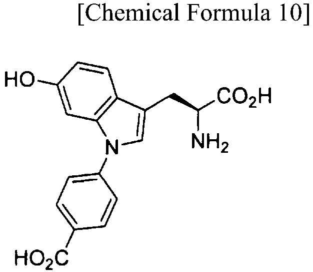
3Py6NH2: (S)-2-amino-3-(6-aminopyridin-3-yl)propanoic acid (CAS 1269968 -61-7)
Yph: (S)-2-amino-3-(4-phenoxyphenyl) propanoic acid (CAS 150351-64-7)
F4CON: (S)-2-amino-3-(4-carbamoylphenyl)propanoic acid (CAS 223593-04-2)
YBn: (S)-2-amino-3-(4-(benzyloxy)phenyl)propanoic acid (CAS 16652-64-5)
Yae: (S)-2-amino-3-(4-(2-aminoethoxy)phenyl)propanoic acid (CAS 1909283-20-0)
Nal1: (S)-2-amino-3-(naphthalen-1-yl)propanoic acid (CAS 55516-54-6)
mBph: (S)-3-([1,1'-biphenyl]-3-yl)-2-aminopropanoic acid (CAS 164172-96-7)
MeF: (2S)-2-(methylamino)-3-phenylpropanoic acid (CAS No. 2566-30-5)
MeY: (2S)-3-(4-hydroxyphenyl)-2-(methylamino)propanoic acid (CAS No. 537-49-5)
MeF3Me: (S)-2-(methylamino)-3-(meta-tolyl)propanoic acid 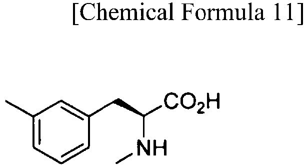
MeF4CON: (S)-3-(4-carbamoylphenyl)-2-(methylamino)propanoic acid 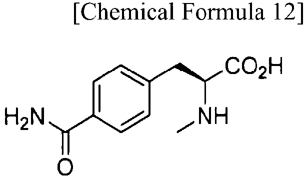
MeF3CON: (S)-3-(3-carbamoylphenyl)-2-(methylamino)propanoic acid 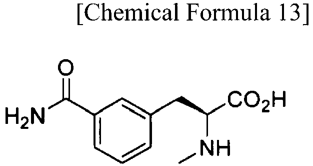
F3CON: (S)-2-amino-3-(3-carbamoylphenyl)propanoic acid (CAS 1217651-22-3)
MeCha: (S)-3-cyclohexyl-2-(methylamino)propanoic acid (CAS 2165389-05-7)
MeYap: (S)-3-(4-(3-aminopropoxy)phenyl)-2-(methylamino)propanoic acid 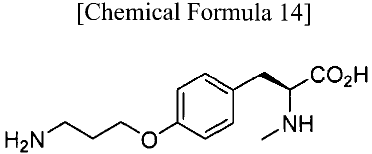
MeA1Ac4pip: (S)-3-(1-acetylpiperidin-4-yl)-2-(methylamino)propanoic acid 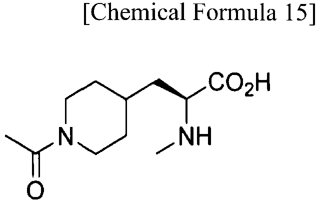
MemBph: (S)-3-([1,1'-biphenyl]-3-yl)-2-(methylamino)propanoic acid 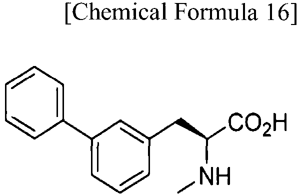
MeNall: (S)-2-(methylamino)-3-(naphthalen-1-yl)propanoic acid 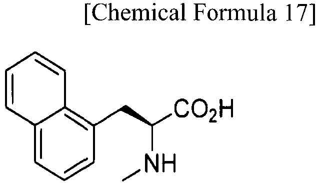
MeNal2: (S)-2-(methylamino)-3-(naphthalen-2-yl)propanoic acid 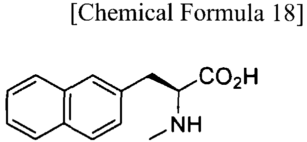
MeF4OMe: (S)-3-(4-methoxyphenyl)-2-(methylamino)propanoic acid (CAS 52939-33-0)
MeF4C: (S)-3-(4-chlorophenyl)-2-(methylamino)propanoic acid (CAS 347851-70-1)
Eva: (S)-2-amino-3-ethylpentanoic acid (CAS 14328-49-5)
Cbg: (S)-2-amino-2-cyclobutylacetic acid (CAS 49607-08-1)
Gcpe: (S)-2-amino-2-cyclopentylacetic acid (CAS 2521-84-8)
TMe: (2S,3R)-2-amino-3-methoxybutanoic acid (CAS 4144-02-9)
MeG: 2-(methylamino)acetic acid (CAS 107-97-1)
Meda: (2R)-2-(methylamino)propanoic acid (CAS 29475-64-7)
dp: D-proline (CAS 344-25-2)
EtG: 2-(ethylamino)acetic acid (CAS 627-01-0)
PrG: 2-(propylamino)acetic acid (CAS 25303-14-4)
F3aa: (S)-2-amino-3-(3-(carboxymethyl)phenyl)propanoic acid (CAS 1270107-31-7)
F3aao: (S)-2-amino-3-(3-(carboxymethoxy)phenyl)propanoic acid 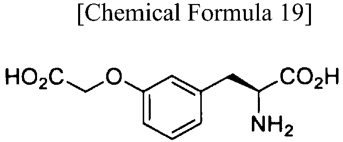
F4aa: (S)-2-amino-3-(4-(carboxymethyl)phenyl)propanoic acid (CAS 142348-77-4)
F4aao: (S)-2-amino-3-(4-(carboxymethoxy)phenyl)propanoic acid (CAS 24558-63-2)
F4C: (S)-2-amino-3-(4-chlorophenyl)propanoic acid (CAS 14173-39-8)
F3OMe: (S)-2-amino-3-(3-methoxyphenyl) propanoic acid (CAS 33879-32-2)
F3COO: (S)-3-(2-amino-2-carboxyethyl)benzoic acid (CAS No. 13861-02-4)
KAc: (2S)-2-amino-6-acetamidohexanoic acid (CAS 692-04-6)
Bph2C: (S)-2-amino-3-(2'-chloro-[1,1'-biphenyl]-4-yl)propanoic acid 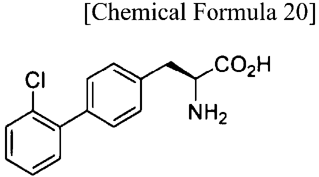
Bph2OMe: (S)-2-amino-3-(2'-methoxy-[1,1'-biphenyl]-4-yl)propanoic acid 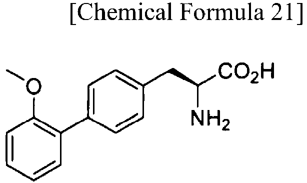
pBph2aao: (S)-2-amino-3-(2'-(carboxymethoxy)-[1,1'-biphenyl]-4-yl)propanoic acid 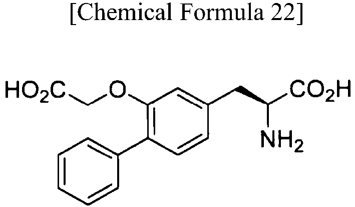
F4COO: (S)-4-(2-amino-2-carboxyethyl)benzoic acid (CAS 126109-42-0)
3Py: (S)-2-amino-3-(pyridin-3-yl)propanoic acid (CAS 64090-98-8)
4Py: (S)-2-smino-3-(pyridin-4-yl)propanoic acid (CAS 37535-49-2)
F4F: (S)-2-amino-3-(4-fluorophenyl) propanoic acid (CAS 1132-68-9)
F4OMe: (S)-2-amino-3-(4-methoxyphenyl)propanoic acid (CAS 6230-11-1)
Cha: (S)-2-amino-3-cyclohexylpropanoic acid (CAS 27527-05-5)
MeKMe: (2S)-2,6-bis(methylamino)hexanoic acid (CAS 51876-34-7)
MeF4am: (S)-3-(4-(aminomethyl)phenyl)-2-(methylamino)propanoic acid 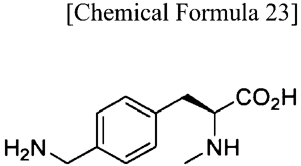
MeDap: (S)-3-amino-2-(methylamino)propanoic acid (CAS 904832-42-4)
MeF4COO: (S)-4-(2-carboxy-2-(methylamino)ethyl)benzoic acid 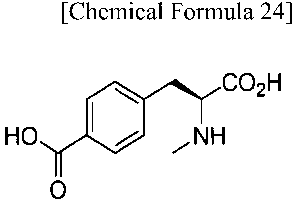
F4am: (S)-2-amino-3-(4-(aminomethyl)phenyl)propanoic acid (CAS 150338-20-8)
Ahp: (S)-2-aminoheptanoic acid (CAS No. 1115-90-8)
Ano: (S)-2-aminononanoic acid (CAS 133444-84-5)
Ado: (S)-2-aminododecanoic acid (CAS 169106-34-7)
Ade: (S)-2-aminodecanoic acid (CAS 84277-81-6)
Aoc: (S)-2-aminooctanoic acid (CAS 116783-26-7)
SPent: (2S)-2-amino-3-(pentyloxy)propanoic acid (CAS 1502644-74-7)
HseBu: (2S)-2-amino-4-butoxybutanoic acid (CAS 17673-71-1)
AhpOMe: (S)-2-amino-7-methoxyheptanoic acid 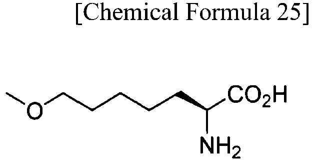
Aoc7r8dH: (2S)-2-amino-7,8-dihydroxyoctanoic acid 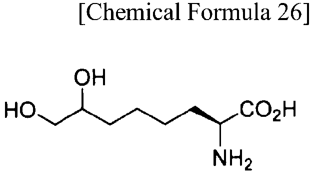
HsePr: (2S)-2-amino-4-propoxybutanoic acid (CAS 18312-28-2)
AhpOH: (S)-2-amino-7-hydroxyheptanoic acid (CAS 2136908-21-7)
Aun: (S)-2-aminoundecanoic acid (CAS 169106-34-7)
MeK: (2S)-6-amino-2-(methylamino)hexanoic acid (CAS 7431-89-2)
MeKCOpipzaa: (2S)-6-{[4-(carboxymethyl)piperazin-1-carbonyl]amino}-2-(methylamino)hexanoic acid 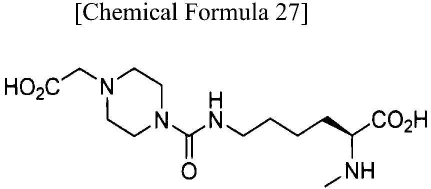
MeDab: (S)-4-amino-2-(methylamino)butanoic acid 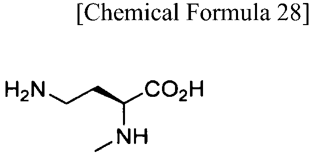
MeF3Et: (S)-3-(3-ethylphenyl)-2-(methylamino)propanoic acid 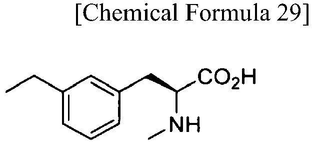
Me4Py: (S)-2-(methylamino)-3-(pyridin-4-yl)propanoic acid 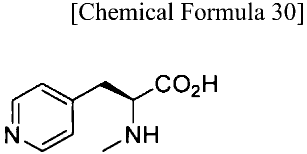
Me3Py: (S)-2-(methylamino)-3-(pyridin-3-yl)propanoic acid (CAS 2651172-69-7)
MeC: (2R)-2-(methylamino)-3-sulfanylpropanoic acid (CAS 4026-48-6)
dc: D-cysteine (CAS 921-01-7)
Medc: (2S)-2-(methylamino)-3-sulfanylpropanoic acid (CAS 95244-61-4)
dhcy: (2R)-2-amino-4-sulfanilbutanoic acid (CAS 6027-14-1)
Hcy:(2S)-2-amino-4-sulfanilbutanoic acid (CAS 6027-13-0)
Aeoac: 2-(2-aminoethoxy)acetic acid (CAS 10366-71-9)
Ape: 5-aminopentanoic acid (CAS 660-88-8)
dhgl: (R)-2-aminohexanedioic acid (CAS 7620-28-2)
Hgl: (S)-2-aminohexanedioic acid (CAS 1118-90-7)
MeHgl: (S)-2-(methylamino)hexanedioic acid (CAS 261943-13-9)
Medhgl: (R)-2-(methylamino)hexanedioic acid 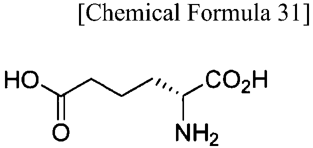
MeAeoac: 2-(2-(methylamino)ethoxy)acetic acid (CAS 98137-58-7)
MeApe: 5-(methylamino)pentanoic acid (CAS 21382-30-9)
pipzAc: 2-(piperazin-1-yl)acetic acid (CAS 37478-58-3)
pipAc: 2-(piperidin-4-yl)acetic acid (CAS 51052-78-9)
Pip4mAc: 3-(piperidin-4-yl)propanoic acid (CAS 1822-32-8)
Pic4: piperidin-4-carboxylic acid (CAS 498-94-2)
F42Py: (2S)-2-amino-3-[4-(pyridin-2-yl)phenyl]propanoic acid (CAS 1336207-47-6)
F44Py: (2S)-2-amino-3-[4-(pyridin-4-yl)phenyl]propanoic acid 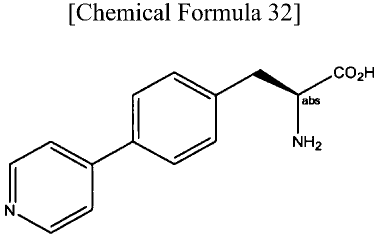
cC14COO: hexadecanedioic acid (CAS 505-54-4)
cC16COO: octadecanedioic acid (CAS 871-70-5)
PEG9NH2: 3,6,9,12,15,18,21,24,27-nonaoxanonacosan-1,29-diamine (CAS 474082-35-4)
Biotin: 5-((3aS,4S,6aR)-2-oxohexahydro-1H-thieno[3,4-d]imidazol-4-yl)pentanoic acid (CAS 58-85-5)
PEG12c: 1-amino-3,6,9,12,15,18,21,24,27,30,33,36-dodecaoxanonatriacontan-39-oic acid (CAS No. 1415408-69-3)

### 2. Peptide (A)

The present invention relates to a peptide or a pharmaceutically acceptable salt thereof. References herein to "peptide" also include references to a pharmaceutically acceptable salt thereof or a solvate thereof, unless otherwise indicated.

An aspect of the present invention provides a peptide including the following amino acid sequence:
A-R-W-Y-MeF-I-MeG-Y-Y-R-MeF-L-MeA-MeF-C (SEQ ID NO: 1)
or
an amino acid sequence having a substitution, addition, deletion, or insertion in 1 to 14 amino acid residues selected from the group consisting of amino acid residues at positions 1, 2, 3, 4, 5, 6, 7, 8, 9, 11, 12, 13, 14, and 15 in the above amino acid sequence.

### (Peptide (A))

A-R-W-Y-MeF-I-MeG-Y-Y-R-MeF-L-MeA-MeF-C (SEQ ID NO: 1) is a peptide confirmed to have myostatin inhibitory activity in the Examples herein.

The amino acid sequence may have a substitution, addition, deletion, or insertion in 1 to 14 amino acid residues selected from the group consisting of amino acid residues at positions 1, 2, 3, 4, 5, 6, 7, 8, 9, 11, 12, 13, 14, and 15 in SEQ ID NO: 1. The number of amino acid substitutions, deletions, additions, and/or insertions should be 1 or more and 14 or less and the lower limit is 1. The upper limit may be 14, 13, 12, 11, 10, 9, 8, 7, 6, 5, 4, 3, or 2, and the minimum is 1. Preferred is an amino acid "substitution". Such an amino acid substitution is preferably a conservative amino acid substitution.

The term "conservative amino acid substitution" means a substitution with a functionally equivalent or similar amino acid. A conservative amino acid substitution in a peptide causes a static change in the amino acid sequence of the peptide. For example, one or two or more amino acids with similar polarity act in a functionally equivalent manner to produce a static change in the amino acid sequence of such a peptide. In general, a substitution within a group can be considered structurally and functionally conservative. However, as obvious to those skilled in the art, the role played by a particular amino acid residue can be determined by its significance in the three-dimensional structure of the molecule containing that amino acid. For example, a cysteine residue can take a less polar, oxidized (disulfide) form when compared to the reduced (thiol) form. The long aliphatic portion of an arginine side chain can constitute a structurally and functionally important feature. In addition, an aromatic ring-containing side chain (of tryptophan, tyrosine, or phenylalanine) can contribute to an ion-aromatic or cation-pi interaction. In such cases, substitution of an amino acid having such a side chain with an amino acid belonging to an acidic or nonpolar group can be structurally and functionally conservative. A residue such as proline, glycine, or cysteine (disulfide form) can have a direct effect on the conformation of the main chain and often cannot be replaced without structural distortion.

The conservative amino acid substitution includes a specific substitution based on side chain similarity (e.g., Lehninger, Biochemistry, Revised 2nd edition, published 1975, pp. 73-75 (L. Lehninger, Biochemistry, 2nd edition, pp73-75, Worth Publisher, New York (1975))) and a typical substitution. In addition, the conservative amino acid substitution is preferably a substitution to an amino acid belonging to the same group as a group of amino acids as grouped on the basis of side chain properties common to those of the following natural amino acids.

Hydrophobic (also called nonpolar) amino acids: amino acids that are hydrophobic (nonpolar) and include alanine ("Ala" or simply "A"), glycine ("Gly" or simply "G"), valine ("Val" or simply "V"), leucine ("Leu" or simply "L"), isoleucine ("Ile" or simply "I"), proline ("Pro" or simply "P"), phenylalanine ("Phe" or simply "F"), tryptophan ("Trp" or simply "W"), tyrosine ("Tyr" or simply "Y"), and methionine ("Met" or simply "M").

Note that the hydrophobic amino acids can be further divided into the following groups.

Aliphatic amino acids: amino acids with fatty acid or hydrogen in the side chain, including Ala, Gly, Val, Ile, and Leu.

Aliphatic/branched-chain amino acids: amino acids with branched fatty acid in the side chain, including Val, Ile, and Leu.

Aromatic amino acids: amino acids with an aromatic ring in the side chain, including Trp, Tyr, and Phe.

Hydrophilic (also called polar) amino acids: amino acids that are hydrophilic (polar) and include serine (also "Ser" or simply "S"), threonine (also "Thr" or simply "T"), cysteine (also "Cys" or simply "C"), asparagine (also "Asn" or simply "N"), glutamine (also "Gln" or simply "Q"), aspartic acid (also "Asp" or simply "D"), glutamic acid ("Glu" or simply "E"), and lysine (also designated as "Lysine"; "Lys" or simply "K"), arginine ("Arg" or simply "R"), and histidine ("His" or simply "H").

Note that the hydrophilic amino acids can be further divided into the following groups.

Acidic amino acids: amino acids with an acidic side chain, including Asp and Glu. Basic amino acids: amino acids with a basic side chain, including Lys, Arg, and His.

Neutral amino acids: amino acids with a neutral side chain, including Ser, Thr, Asn, Gln, and Cys.

Gly and Pro can also be classified as "amino acids affecting the direction of the main chain", and amino acids containing a sulfur molecule in the side chain, namely Cys and Met, can be classified as "sulfur-containing amino acids".

As used herein, the "amino acids" include not only natural amino acids but also non-natural amino acids. Examples of the non-natural amino acids include N-alkylamino acids, in which the above-described natural amino acid is N-alkylated, and those in which the nitrogen forming a peptide bond is modified with a branched or unbranched lower (e.g., C1-C5, preferably C1-C3, more preferably C1) alkyl group. The N-alkylamino acids are preferably N-ethylamino acids, N-butylamino acids, or N-methylamino acids, and more preferably N-methylamino acids. In addition, examples of the non-natural amino acids include D-form amino acids (also called D-amino acids), β-amino acids, γ-amino acids, amino acid mutants, chemically modified amino acids such as amino acid derivatives, and amino acids, such as norleucine and ornithine, which are not used as building blocks of proteins *in vivo.* Further, examples include amino acids in which the side chain of a natural amino acid further contains a functional group or is substituted by another functional group (e.g., amino acids with, for example, a side chain arylene or alkylene group moiety with substitution and/or addition, amino acids with an increased number of carbon atoms in an arylene, alkylene or alkyl group in the side chain, amino acids with a substituted aromatic ring in a side chain, heterocyclized or cyclocondensed amino acids).

Note that a nature amino acid side chain may contain or be substituted with a structure such as a functional group. This can impart a property different from that of a natural amino acid. For example, Dap is an amino acid with an amino group in the side chain of alanine. Here, the amino group is added to exhibit a property of basic, polar amino acid, which differs from alanine belonging to a nonpolar amino acid group. In other words, non-natural amino acids with similar side chain properties can be included in the above-described groups obtained by classifying natural amino acids based on their common side chain properties. For example, N-methylarginine (MeR), an amino acid in which the main chain nitrogen atom of arginine, which belongs to basic amino acids, is methylated, is a non-natural amino acid, but is basic. Thus, it can be classified as a basic amino acid. As such, non-natural amino acids that exhibit side-chain properties similar to those of a certain amino acid can also be included as targets for conservative amino acid substitution. Note that D-amino acids such as de (D-glutamic acid) can be classified as D-amino acids or can also be classified according to the property of their side chain. N-methylamino acids can be classified as N-alkylamino acids or classified according to the nature of the side chain of the original amino acid that is not N-methylated.

Although not limited, non-natural amino acids include N-methylamino acids, MeA, Dap, ApG, CrpG, CmG, MeD, CrmG, MeopG, MeeG, AcapG, A4paa, KCOpipzaa, CeG, CrbG, KMe, Hly, Hg, Orn, Dab, MeE, da, Aib, de, dkCOpipzaa, ddab, dor, MeW, W7N, W4C, Bph, 3Py6Ph, W6N, F41dMeCmm4Pyz, W1Bn, W6H, Bph4ms, W1Ph, W1aa, W7Ph3C, W1Ph4OMe, W1Ph4COO, W6H1Ph4COO, 3Py6NH2, Yph, F4CON, YBn, Yae, Nal1, F42Py, mBph, MeF, MeY, MeF3Me, MeF4CON, MeF3CON, F3CON, MeCha, MeYap, MeA1Ac4pip, MemBph, MeNall, MeNal2, MeF4OMe, MeF4C, Eva, Cbg, Gepe, TMe, MeG, Meda, dp, EtG, PrG, F3aa, F3aao, F4a, F4aao, F4C, F3OMe, F3COO, KAc, Bph2C, Bph2OMe, pBph2aao, F4COO, 3Py, 4Py, F4F, F4OMe, Cha, MeKMe, MeF4am, MeDap, MeF4COO, Ahp, Ano, Ado, Ade, Aoc, SPent, HseBu, AhpOMe, Aoc7r8dH, HsePr, AhpOH, Aun, MeK, MeKCOpipzaa, MeDab, MeF3Et, Me4Py, Me3Py, MeC, dc, Medc, dhcy, Hcy, Aeoac, Ape, dhgl, Hgl, MeHgl, Medhgl, MeAeoac, MeApe, pipzAc, pipAc, Pip4mAc, Pic4, F42Py, and F44Py.

Peptide (A) substituted at any of the 1 to 14 amino acid residues may be a peptide included in the scope of peptide (B) described below.

In one embodiment, the peptide of the present invention is a peptide including the following amino acid sequence:
A-R-W-Y-MeF-I-MeG-Y-Y-R-MeF-L-MeA-MeF-C (SEQ ID NO: 1)
or a peptide including an amino acid sequence containing not more than 14 amino acid substitutions selected from the following (1-i) to (1-xii):
(1-i) substitution where position 1 of the amino acid sequence set forth in SEQ ID NO: 1 is replaced by any amino acid or peptoid;
(1-ii) substitution where position 2 of the amino acid sequence set forth in SEQ ID NO: 1 is replaced by any amino acid;
(1-iii) substitution where position 3 of the amino acid sequence set forth in SEQ ID NO: 1 is replaced by an amino acid having an optionally substituted aromatic ring in a side chain;
(1-iv) substitution where position 4 of the amino acid sequence set forth in SEQ ID NO: 1 is replaced by an amino acid having an optionally substituted aromatic ring in a side chain;
(1-v) substitution where position 5 of the amino acid sequence set forth in SEQ ID NO: 1 is replaced by an amino acid having an optionally substituted aromatic ring or optionally substituted cycloalkyl in a side chain;
(1-vi) substitution where position 6 of the amino acid sequence set forth in SEQ ID NO: 1 is replaced by an amino acid having an alkyl group in a side chain;
(1-vii) substitution where position 7 of the amino acid sequence set forth in SEQ ID NO: 1 is replaced by any secondary amino acid, N-alkyl amino acid, or peptoid;
(1-viii) substitution where position 8 of the amino acid sequence set forth in SEQ ID NO: 1 is replaced by any amino acid;
(1-ix) substitution where position 9 of the amino acid sequence set forth in SEQ ID NO: 1 is replaced by an amino acid containing an optionally substituted aromatic ring or an optionally substituted alkyl or cycloalkyl group in a side chain;
(1-x) substitution where position 11 of the amino acid sequence set forth in SEQ ID NO: 1 is replaced by an amino acid containing an optionally substituted aromatic ring or an optionally substituted alkyl or cycloalkyl group in a side chain;
(1-xi) substitution where position 12 of the amino acid sequence set forth in SEQ ID NO: 1 is replaced by an optionally substituted aliphatic amino acid or an amino acid having an optionally substituted alkyl chain in a side chain;
(1-xii) substitution where position 13 of the amino acid sequence set forth in SEQ ID NO: 1 is replaced by any secondary amino acid, N-alkyl amino acid, or peptoid;
(1-xiii) substitution where position 14 of the amino acid sequence set forth in SEQ ID NO: 1 is replaced by an amino acid having an optionally substituted aromatic ring in a side chain; or
(1-xiv) substitution where position 15 of the amino acid sequence set forth in SEQ ID NO: 1 is replaced by any amino acid.

Although not limited, the peptide of the present invention may be a peptide containing an amino acid sequence having 14 or less, 13 or less, 12 or less, 11 or less, 10 or less, 9 or less, 8 or less, 7 or less, 6 or less, 5 or less, 4 or less, 3 or less, 2 or less, or 1 amino acid substitution selected from (1-i) to (1-xiv) above in the amino acid sequence set forth in SEQ ID NO: 1. Substitutions (1-i) to (1-xii) include any combination thereof.

The "pharmaceutically acceptable salt thereof" means a salt of any of the peptides. Examples of the pharmaceutically acceptable salt include salts of mineral acids (e.g., sulfuric acid, hydrochloric acid, phosphoric acid), salts of organic acids (e.g., acetic acid, oxalic acid, lactic acid, tartaric acid, fumaric acid, maleic acid, methanesulfonic acid, benzenesulfonic acid), salts of amines (e.g., trimethylamine methylamine), or salts of metal ions (e.g., sodium, potassium, or calcium ions). For compounds that come to contain moisture over time, such moisture is also included in the pharmaceutically acceptable salt thereof.

The peptide may contain an additional amino acid residue(s) in addition to the amino acid sequence of SEQ ID NO: 1. The "additional amino acid residue(s)" is described in detail in "3. Peptide (B)".

The above peptide may, in one embodiment, contain a linker preferably at the C-terminus. The "linker" is described in detail in "3. Peptide (B)".

The above peptide is, in one embodiment, a cyclic peptide. The "cyclic peptide" is described in detail in "3. Peptide (B)".

The above peptide preferably has myostatin inhibitory activity. The details of "having myostatin inhibitory activity" are described in "3. Peptide (B)".

The matters described in other sections also apply to this section, unless otherwise noted.

### 3. Peptide (B)

The present invention relates to a peptide or a pharmaceutically acceptable salt thereof. References herein to "peptide" also include references to a pharmaceutically acceptable salt thereof, unless otherwise indicated.

The matters described in "2. Peptide (A)" also apply to this section unless there is any particular problem.

One embodiment of the present invention provides a peptide including the following amino acid sequence:
X1-X2-X3-X4-X5-X6-X7-X8-X9-X10-X11-X12-X13-X14-X15 (SEQ ID NO: 2), wherein
X1 is any amino acid or peptoid;
X2 is any amino acid;
X3 is an amino acid having an optionally substituted aromatic ring in a side chain;
X4 is an amino acid having an optionally substituted aromatic ring in a side chain;
X5 is an amino acid having an optionally substituted aromatic ring or optionally substituted cycloalkyl in a side chain;
X6 is an amino acid having an alkyl group in a side chain;
X7 is any secondary amino acid, N-alkylamino acid, or peptoid;
X8 is any amino acid;
X9 is an amino acid having an optionally substituted aromatic ring or an optionally substituted alkyl group or cycloalkyl group in a side chain;
X10 is R;
X11 is an amino acid having an optionally substituted aromatic ring or an optionally substituted alkyl group or cycloalkyl group in a side chain;
X12 is an optionally substituted aliphatic amino acid or an amino acid having an optionally substituted alkyl chain in a side chain;
X13 is any secondary amino acid, N-alkylamino acid, or peptoid;
X14 is an amino acid having an optionally substituted aromatic ring in a side chain; and
X15 is any amino acid.

### (Peptide (B))

One embodiment of the present invention provides a peptide satisfying the following one or two or more requirements.
X1 is an amino acid having an optionally substituted alkyl group in a side chain or a peptoid having an optionally substituted alkyl group in a side chain attached to amide nitrogen;
X3 is optionally substituted F or W;
X4 is optionally substituted F or Y, or naphthylalanine;
X5 is optionally substituted F, Y or naphthylalanine, or optionally substituted cyclohexylalanine;
X6 is an amino acid having a branched alkyl group, or an amino acid having a cycloalkyl group, wherein a carbon atom contained therein is optionally replaced by an oxygen atom;
X8 is an amino acid having an optionally substituted aromatic ring or an optionally substituted alkyl group or cycloalkyl in a side chain;
X9 is optionally substituted F or Y, or an amino acid containing an optionally substituted heterocycloalkyl group in a side chain, or alanine;
X12 is an aliphatic amino acid containing three or more carbon atoms in a side chain, or an amino acid having an optionally substituted alkyl chain in a side chain;
X13 is an N-alkylamino acid;
X14 is optionally substituted F or Y; and
X15 is an amino acid having a sulfur (S) element at a side chain terminus.

### (Peptide (B)-1)

The above options for X1 to X15 in one of the above embodiments may be selected in any combination. Although not limited, the requirements include 1 or more, 2 or more, 3 or more, 4 or more, 5 or more, 6 or more, 7 or more, 8 or more, 9 or more, 10 or more, 11 or more, 12 or more, 13 or more, or 14 or more requirements described above. Preferably, all of the above 15 requirements are satisfied.

One embodiment of the present invention provides a peptide satisfying the following one or two or more requirements.
X1 is an amino acid having an optionally substituted alkyl group in a side chain or a peptoid having an optionally substituted alkyl group in a side chain attached to amide nitrogen;
X3 is optionally substituted F or W;
X4 is optionally substituted F or Y, or naphthylalanine;
X5 is optionally substituted F, Y or naphthylalanine, or optionally substituted cyclohexylalanine;
X6 is an amino acid having a branched alkyl group, or an amino acid having a cycloalkyl group, wherein a carbon atom contained therein is optionally replaced by an oxygen atom;
X8 is an amino acid having an optionally substituted aromatic ring or an optionally substituted alkyl group or cycloalkyl in a side chain;
X9 is optionally substituted F or Y, or an amino acid containing an optionally substituted heterocycloalkyl group in a side chain, or alanine;
X12 is an aliphatic amino acid containing three or more carbon atoms in a side chain, or an amino acid having an optionally substituted alkyl chain in a side chain;
X13 is an N-alkylamino acid;
X14 is F or Y having a optionally substituted side chain; and
X15 is an aliphatic amino acid, and is attached to an aliphatic amino acid or proline as X16.

### (Peptide (B)-2)

The above options for X1 to X15 in one of the above embodiments may be selected in any combination. Although not limited, the requirements include 1 or more, 2 or more, 3 or more, 4 or more, 5 or more, 6 or more, 7 or more, 8 or more, 9 or more, 10 or more, 11 or more, 12 or more, 13 or more, or 14 or more requirements described above. Preferably, all of the above 15 requirements are satisfied.

Peptide (B)-2 differs from peptide (B)-1 in X15. In one embodiment, the peptide of the present invention may have, but is not limited to, as many as 15 or 16 amino acid residues (when X16 is present) contained in the cyclic structure.

Any amino acid is not particularly limited and may be any known amino acid. Note that examples of any amino acid include, but are not limited to, L-/D-form amino acids, N-alkylamino acids, and peptoids.

As used herein, the term "optionally substituted" means either unsubstituted or substituted with any number of any substituents.

Examples of the "amino acid having an optionally substituted aromatic ring in a side chain" include natural amino acids belonging to aromatic amino acids, or non-natural amino acids such as N-acetylated aromatic amino acids, or natural or non-natural amino acids with an aromatic ring added to or substituted on the side chain thereof, provided that the aromatic ring thereof may be a heterocycle. Note that examples include a heterocycle where the side chain indole ring of Trp has a heteroatom-containing ring or a heterocycle where the side chain benzyl group of Phe has a heteroatom. Note that one or more substitutions may be allowed.

The "amino acid having a substituted aromatic ring" means an amino acid in which a carbon atom constituting the aromatic ring or a functional group attached to the aromatic ring is substituted. The "optionally substituted aromatic ring in a side chain" may be those having a condensed ring structure (e.g., a benzene ring, an indole ring, a naphthalene ring) where a carbon atom thereof, for example, is replaced by another atom (e.g., oxygen, nitrogen) and/or replaced by an atom further having another molecule or functional group. Note that it may contain a heteroaromatic ring. In addition, examples of the amino acid having an optionally substituted aromatic ring in a side chain may include an amino acid in which a side chain hydroxyl group of tyrosine is replaced by another functional group. The kind and/or position of the substituted functional group is not particularly limited, and may be optionally selected from, for example, an alkyl group, a phenyl group, a cycloalkyl group, a hydroxy group, or a halogen. Examples of the amino acid having an optionally substituted aromatic ring in a side chain include tyrosine, tryptophan, histidine, or phenylalanine. The amino acid having an optionally substituted aromatic ring in a side chain may be any natural or non-natural amino acid, and examples may include 4Py, 3Py, F4aao, W7N, Bph, F44Py, F4F, or F4C.

Examples of the "optionally substituted naphthylalanine" include naphthylalanine and amino acids in which a carbon atom in the naphthyl group of the side chain of naphthylalanine is replaced by another atom such as nitrogen, and/or is replaced by an atom further having another molecule or functional group. Note that one or more substitutions may be allowed.

Examples of the "amino acid having optionally substituted cycloalkyl in a side chain" include an amino acid having a cycloalkyl group (also called a cyclic alkyl group) in a side chain, e.g., an amino acid having a C₃₋₈ cycloalkyl group. In addition, examples of the "amino acid having a substituted cycloalkyl group" include an amino acid having a side chain in which some carbon atoms in the cycloalkyl group are replaced by other atoms such as oxygen or nitrogen, or by atoms further having other molecules or functional groups. In other words, examples of the "amino acid having a substituted cycloalkyl group" include an amino acid having a heterocycloalkyl group. Note that one or more substitutions may be allowed.

Examples of the "amino acid having a heterocycloalkyl group in a side chain" include an amino acid having a ring in which some carbon atoms in the cycloalkyl group mentioned above are replaced by other atoms such as oxygen or nitrogen, or by atoms further having other molecules or functional groups. Note that one or more substitutions may be allowed. In addition, examples of the "amino acid having a substituted heterocycloalkyl group" include an amino acid having a side chain heterocycle in which some molecules in the heterocycloalkyl group are replaced by other molecules or functional groups. Examples include an amino acid in which some carbon atoms in the heterocycloalkyl group are replaced with ethyl group-containing nitrogen (CH₃-CH₂-N). Note that one or more substitutions may be allowed.

The "amino acid having an alkyl group in a side chain" means an amino acid having an alkyl group in a side chain, and examples include, but are not limited to, alanine, proline, isoleucine, leucine, and valine, provided that the alkyl group may be either linear or cyclic.

The "amino acid having an optionally substituted alkyl group in a side chain" means an amino acid having a chain-like alkyl group in a side chain, and examples include, but are not limited to, alanine, isoleucine, leucine, or valine wherein the side chain of the amino acid having a chain-like alkyl group in a side chain is further substituted with a substituent such as an alkyl group or a polar group, examples of which include, but are not limited to, a carboxy group, an amide group, or a methanesulfonyl group. Note that one or more substitutions may be allowed.

Examples of the "peptoid" include an amino acid in which a side chain is attached to amide nitrogen contained in the amino acid. The side chain is not particularly specified, and may be any known amino acid side chain structure.

The "peptoid having an optionally substituted alkyl group in a side chain attached to amide nitrogen" means an amino acid having an amide nitrogen-attached side chain wherein the side chain of the amino acid having a chain-like alkyl group is further substituted with a substituent such as an alkyl group or a polar group, examples of which include, but are not limited to, a carboxy group, an amide group, or a methanesulfonyl group. In addition, examples of the "peptoid having an optionally substituted alkyl group attached to amide nitrogen" also include, but are not limited to, alanine or glycine having a C₁₋₆ alkyl group attached to amide nitrogen. Note that one or more substitutions may be allowed.

Examples of the "optionally substituted aliphatic amino acid" include amino acids classified as aliphatic amino acids and amino acids having a linear or branched alkyl group in a side chain in which some molecules of the linear or branched alkyl group in a side chain are replaced by other molecules or functional groups. Examples include an amino acid in which the methyl group CH₃- in the side chain of alanine is replaced with a pentyl group (CH₃CH₂CH₂-), and an amino acid (e.g., AhpOMe, Ado) in which a carbon atom in the alkyl group is replaced with oxygen. Note that one or more substitutions may be allowed.

The "amino acid containing a sulfur element (S) at a side chain terminus" refers to an amino acid containing a sulfur atom at a side chain terminus, and examples include, but are not limited to, cysteine (Cys), N-alkylated cysteine, and D-form cysteine. Examples include, but are not limited to, C, MeC, dc, Medc, dhcy or Hcy.

Examples of the "optionally substituted F", "optionally substituted Y", or "optionally substituted W" include an amino acid containing a substituent in the benzene ring of the side chain of Phe, in the phenol group of the side chain of Tyr, or in the indole ring of the side chain of Trp, an amino acid having a heteroatom-containing ring, or an amino acid having a functional group attached thereto. Examples may include an amino acid in which a carbon atom in the indole ring of Trp is further replaced by nitrogen, or an amino acid having a side chain biphenyl group in which a carbon atom in the benzene ring of Phe is further substituted with a carbon atom having a benzene ring. In addition, examples include an amino acid in which a carbon atom in the biphenyl group is further replaced by another atom or an atom having another functional group, and an amino acid in which the hydroxyl group in the phenol group of Tyr is replaced by another atom such as fluorine or an atom having another functional group. Note that one or more substitutions may be allowed.

The "amino acid having a branched alkyl group or cycloalkyl group, in which a carbon atom contained therein is optionally replaced by an oxygen atom" means an amino acid in which a carbon atom contained in a branched alkyl group or cycloalkyl group in a side chain is replaced by an oxygen atom. Examples include, but are not limited to, isoleucine or TMe. Note that one or more substitutions may be allowed.

Examples of the "aliphatic amino acid having 3 or more carbon atoms in a side chain or amino acid having an optionally substituted alkyl chain in a side chain" include amino acids classified as aliphatic amino acids and amino acids having an alkyl group in a side chain, provided that the alkyl group in a side chain contains 3 or more carbon atoms, and the carbon atom in the side chain alkyl group is replaced by oxygen or another molecule or functional group. Examples include Ahp, Ano, AhpOMe, Ado, HsePr, SPent, or Leu.

The "alanine or glycine having a C₁₋₆ alkyl group attached to amide nitrogen" refers to an amino acid further having a C₁₋₆ alkyl group, preferably a C₁₋₃ alkyl group, attached to amide nitrogen of alanine or glycine. Note that alanine having a C₁ alkyl group attached to amide nitrogen is also called N-methylalanine.

In addition, examples of the N-alkylamino acid include, for each amino acid or its derivative, an amino acid having an N-alkyl group attached to amide nitrogen. Examples include N-methylamino acid.

### (One embodiment of peptide (B)-1)

One embodiment of the present invention provides a peptide satisfying the following one or two or more requirements.
X1 is A, MeA, Dap, ApG, CrpG, CmG, MeD, N, CrmG, MeopG, MeeG, AcapG, A4paa, KCOpipzaa, CeG, CrbG, K, KMe, Hly, E, Hgl, Orn, Dab, MeE, D, or MeHgl;
X2 is R, Dap, K, E, A, da, S, G, de, dkCOpipzaa, ddab, or dorn;
X3 is W, W7N, F44Py, Bph, W6N, F41dMeCmm4Pyz, W1Bn, W6H, W1Ph, Bph4ms, W1aa, W7Ph3C, W1Ph4OMe, W1Ph4COO, F42Py, or W6H1Ph4COO;
X4 is Y, 3Py6NH2, YBn, Yae, Nal1, Yph, or mBph;
X5 is MeF, MeY, MeF3Me, F, MeCha, MeYap, MeA1Ac4pip, MemBph, MeNall, MeNal2, MeF4OMe, or MeF4C;
X6 is I, Gcpe, or TMe;
X7 is MeG, MeA, Meda, dp, EtG, or PrG;
X8 is Y, F4COO, K, 4Py, F, F3aao, F3OMe, A4paa, KCOpipzaa, Bph2C, Bph2OMe, or pBph2aao;
X9 is Y, F4COO, 3Py, 4Py, F4aao, F4CON, F3CON, F4F, or F4OMe;
X11 is MeF, Me4Py, Me3Py, F, Cha, MeCha, MeK, MeKMe, MeF4am, MeD, MeE, MeF4COO, or F4am;
X12 is L, Ahp, Ano, Ado, Ade, Aoc, SPent, HseBu, AhpOMe, HsePr, or Aun;
X13 is MeA, MeE, MeKCOpipzaa, MeK, MeDap, or MeDab;
X14 is MeF, MeF3Et, Me4Py, Me3Py, or MemBph; and
X15 is C, MeC, dc, Medc, dhcy, or Hcy.

### (One embodiment of peptide (B)-2)

Peptide (B)-2 differs from peptide (B)-1 in X15. In one embodiment, the peptide of the present invention may have as many as 15 or 16 amino acid residues (when X16 is present) contained in the cyclic structure.

The above options for X1 to X15 in one of the above embodiments may be selected in any combination. Although not limited, the requirements include 1 or more, 2 or more, 3 or more, 4 or more, 5 or more, 6 or more, 7 or more, 8 or more, 9 or more, 10 or more, 11 or more, 12 or more, 13 or more, or 14 or more requirements described above. Preferably, all of the above 15 requirements are satisfied.

One embodiment of the present invention provides a peptide satisfying the following one or two or more requirements.

The requirements are as follows: X1 is A, MeA, Dap, ApG, CrpG, CmG, MeD, N, CrmG, MeopG, MeeG, AcapG, A4paa, KCOpipzaa, CeG, CrbG, K, KMe, Hly, E, Hgl, Orn, Dab, MeE, D, or MeHgl or de;
X2 is R, Dap, K, E, A, da, S, G, de, dkCOpipzaa, ddab, or dorn;
X3 is W, W7N, F44Py, Bph, W6N, F41dMeCmm4Pyz, W1Bn, W6H, W1Ph, Bph4ms, W1aa, W7Ph3C, W1Ph4OMe, F42Py, W1Ph4COO, or W6H1Ph4COO;
X4 is Y, 3Py6NH2, YBn, Yae, Nal1, Yph, or mBph;
X5 is MeF, MeY, MeF3Me, F, MeCha, MeYap, MeA1Ac4pip, MemBph, MeNall, MeNal2, MeF4OMe, or MeF4C;
X6 is I, Gcpe, or TMe;
X7 is MeG, MeA, Meda, dp, EtG, or PrG;
X8 is Y, F4COO, K, 4Py, F, F3aao, F3OMe, A4paa, KCOpipzaa, Bph2C, Bph2OMe, or pBph2aao;
X9 is Y, F4COO, 3Py, 4Py, F4aao, F4CON, F3CON, F4F, or F4OMe;
X11 is MeF, Me4Py, Me3Py, F, Cha, MeCha, MeK, MeKMe, MeF4am, MeD, MeE, MeF4COO, or F4am;
X12 is L, Ahp, Ano, Ado, Ade, Aoc, SPent, HseBu, AhpOMe, HsePr, or Aun;
X13 is MeA, MeE, MeKCOpipzaa, MeK, MeDap, or MeDab;
X14 is MeF, MeF3Et, Me4Py, Me3Py, or MemBph; and
X15 is Aeoac, Ape, dhgl, Hgl, MeHgl, Medhgl, MeAeoac, or MeApe, or X15 is G, da, A, or de and is attached to MeA, Meda, G, da, MeG, P, or A as X16.

One embodiment of the present invention provides a peptide satisfying the following one or two requirements.

X1 is A, MeA, Dap, ApG, CrpG, CmG, MeD, N, CrmG, MeopG, MeeG, AcapG, A4paa, KCOpipzaa, CeG, CrbG, K, KMe, Hly, E, Hgl, Orn, Dab, MeE, D, or MeHgl; and
X15 is Aeoac, Ape, dhgl, Hgl, MeHgl, Medhgl, MeAeoac, or MeApe, or X15 is G, da, A, or de and is attached to MeA, Meda, G, da, MeG, P, or A as X16.

The above peptides (peptide (A) and peptide (B)) may further contain Dap, de, A4paa, KCOpipzaa, pipzAc, pipAc, or Pip4mAc at the C-terminus.

The above peptide is, in one embodiment, a cyclic peptide. The "cyclic peptide" means a peptide in which two amino acids are bonded and all or part of which is cyclic. Examples of the above-mentioned peptide include those in which amino acids in a peptide form a crosslinking structure, those in which a cyclic structure is formed by lactam ring formation or macrocyclization reaction, or those having a lasso peptide-like structure. In other words, the cyclic peptide may have a part with a cyclic structure and may have a linear chain part.

Peptides generally have poor metabolic stability *in vivo,* and their large size makes it difficult for them to permeate the cell membrane. To address such issues, a peptide cyclization technique has been adopted. It has been suggested that the peptide cyclization increases protease resistance and thus increases their metabolic stability and restricts conformational changes, thereby increasing their rigidity and improving membrane permeability and affinity for a target protein.

In one embodiment, the peptide has a cyclic structure in which a chloroacetylated amino acid is bonded to a cysteine residue included in the peptide. In one embodiment, the peptide has a cyclic structure in which an N-terminal amino acid (amino acid residue at position 1) is bonded to a cysteine residue included in the peptide. In one embodiment, the peptide has a cyclic structure in which an N-terminal amino acid (amino acid residue at position 1) is bonded to a cysteine residue at position 15 included in the peptide. In one embodiment, the peptide has a cyclic structure in which a chloroacetylated N-terminal amino acid (amino acid residue at position 1) is bonded to a cysteine residue at position 15 included in the peptide. The "chloroacetylation" may also be "halogen acetylation" using another halogen. Further, the "acetylation" may also be "acylation" by an acyl group other than the acetyl group.

In this specification, some amino acids may be modified for cyclization of a peptide. The present invention also encompasses amino acids that have been partially modified as such. For example, as described above, a chloroacetyl group may be added to the N-terminal amino acid, and may be bonded to a cysteine residue in the peptide to form a ring. Such various (natural/non-natural) amino acids containing a chloroacetyl group are also included in the amino acids herein.

In addition, in one embodiment, the peptide has a cyclic structure in which an N-terminal amino acid (amino acid residue at position 1: X1) and a C-terminal amino acid (amino acid residue at position 15: X15 or amino acid in the most C-terminal portion if X16 or the like is added as an additional amino acid residue) are bonded. In one embodiment, the peptide has a cyclic structure in which an amino group of an N-terminal amino acid (amino acid residue at position 1) is bonded to a carboxyl group of an amino acid at position 15 included in the peptide. In addition, in one embodiment, an additional amino acid residue is further added to an amino acid residue at position 15, and a cyclic structure may be formed in which an N-terminal amino acid (amino acid residue at position 1) may be bonded to, for example an amino acid residue at position 16 or 17 obtained by adding a further additional amino acid residue(s) to the amino acid residue at position 15. In one embodiment, the peptide has a cyclic structure in which an amino group of an amino acid residue at position 1 included in the peptide is bonded to a carboxyl group of an amino acid residue at position 16.

In one embodiment, the peptide having a cyclic structure may have an additional crosslinking structure between amino acid residues in the cyclic structure. In one embodiment, a crosslinking structure is formed in which a side chain of an amino acid residue in the cyclic structure is linked to a side chain of another amino acid residue in the cyclic structure. For example, non-limitingly, an amino group at the end of the side chain of K, namely an amino acid residue in the cyclic structure, may be bonded to a carboxyl group at the end of the side chain of MeE, namely another amino acid residue in the same cyclic structure, to form a crosslinking structure within the cyclic structure. In addition, in one embodiment, a crosslinking structure is formed between one of an N-terminal amino acid residue (amino acid residue at position 1: X1) or an amino acid residue at position 2 (X2) or 3 (X3) and an amino acid residue at position 11 (X11) or 13 (X13). In the above-mentioned peptide having a cyclic structure with a further crosslinking structure, there may be one or more crosslinking structures. For example, the peptide having a cyclic structure with one more crosslinking structure is also called a bicyclic peptide.

In one embodiment, the peptide having a cyclic structure with a further crosslinking structure has a crosslinking structure in which a side chain of the N-terminal amino acid residue (X1) and a side chain of the amino acid residue at position 11 (X11) are linked. In another embodiment, a crosslinking structure is formed in which a side chain of the N-terminal amino acid residue (X1) is linked to a side chain of the amino acid residue at position 13 (X13). In another embodiment, a crosslinking structure is formed in which a side chain of the amino acid residue at position 2 (X2) is linked to a side chain of the amino acid residue at position 11 (X11). In another embodiment, a crosslinking structure is formed in which a side chain of the amino acid residue at position 3 (X3) is linked to a side chain of the amino acid residue at position 11 (X11).

In one embodiment, the peptide having a cyclic structure with a further crosslinking structure may be: a peptide having a cyclic structure with a further crosslinking structure in which a side chain of one of amino acids including, but not limited to, E, D, MeE, CeG, CrpG, CrbG, K, KMe, Hly, Dap, Hgl, Orn, or Dab is linked to a side chain of one of amino acids MeF4am, MeK, MeKMe, MeD, MeE, MeF4COO, and F4am, or a further crosslinking structure in which a side chain of one of amino acids D, CeG, CrpG, Dap, and E is linked to a side chain of one of amino acids MeDab, MeDap, and MeE; a peptide having a cyclic structure with a further crosslinking structure in which a side chain of one of amino acids ddab, and dorn is linked to a side chain of either amino acid MeD or MeE; or a peptide having a cyclic structure with a further crosslinking structure in which a side chain of W1aa is linked to a side chain of MeK. In addition, the above peptide may be: a peptide having a cyclic structure with a further crosslinking structure in which a side chain of one of amino acids MeE, MeHgl, E, and Hgl is linked to a side chain of MeF4am; or a peptide having a cyclic structure with a further crosslinking structure in which a side chain of either amino acid CrpG or CeG is linked to a side chain of MeDap. Furthermore, a combination of the above amino acids may be allowed.

Examples of the peptide with a crosslinking structure inside the cyclic portion include SEQ ID NOs: 238-290, 326-329, 334-338, 348-354, 358-360, 362-366, and 368. One embodiment of the present invention includes or consists of the amino acid sequence set forth in any one of SEQ ID NOs: 238-290, 326-329, 334-338, 348-354, 358-360, 362-366, and 368.

In one embodiment, the peptide includes or consists of the amino acid sequence set forth in any one of SEQ ID NOs: 2-314 or 326-368. In one embodiment, the peptide is a cyclic peptide including or consisting of the amino acid sequence set forth in any one of SEQ ID NOs: 2-314 or 326-368. The peptide is a peptide consisting of the amino acid sequence set forth in any one of SEQ ID NOs: 2-314 or 326-368, or a peptide consisting of an amino acid sequence having 1 to 14 (preferably 1 to 8, 1 to 7, 1 to 6, 1 to 5, 1 to 4, 1 to 3, 2, or 1) amino acid residue substitutions, deletions, insertions, or additions therein. Note that for a sequence to which amino acid residues are added, a sequence having many more amino acid residues (e.g., 1 to 14 residues) may be added to the sequence, as long as the added portion is a linker. In addition, the peptide consisting of an amino acid sequence having the amino acid residue substitutions, deletions, insertions, or additions is preferably those having myostatin inhibitory activity.

In one embodiment, the peptide does not include SEQ ID NO: 3 or SEQ ID NOs: 110-140. References to "SEQ ID NOs: 2-314" may herein refer to "SEQ ID NOs: 2, 4-109, and 141-314" excluding SEQ ID NOs: 3 and 110-140.

In one embodiment, the peptide includes or consists of the amino acid sequence set forth in any one of SEQ ID NOs: 326-354.

The peptide may further include an additional amino acid residue(s). The peptide may contain, without limitation, an additional amino acid residue(s) in addition to the amino acid sequence of any of SEQ ID NOs: 2-314 and 326-368.

The "additional amino acid residue(s)" may be included in the peptide forming the cyclic structure, or the additional amino acid residue(s) may be added like a linker from the cyclic peptide. The peptide and the number of amide bonds (number and length of amino acids) in the peptide moiety are not limited.

In addition, an additional linker may also be added to the cyclic peptide. Examples of the linker include the above-described amino acid linker (peptide linker), a chemical linker, a fatty acid linker, a nucleic acid linker, or a sugar chain linker. The linker may also be a complex of, for example, a chemical linker and a peptide linker. The chemical linker may be, for example, a PEG linker consisting of 1-24 ethylene glycol units. The linker may also be a fatty acid linker containing a divalent chemical moiety derived from a fatty acid. The amino acid (peptide) linker may be a linker containing at least one amino acid. It is possible to use, for example, a glycine-rich peptide such as a peptide having the sequence [Gly-Gly-Gly-Gly-Gly-Ser]ₙ (where n is 1, 2, 3, 4, 5, or 6) as described in U.S. Patent No. 7,271,149 or a serine-rich peptide linker described in U.S. Patent No. 5,525,491. In addition, it is possible to use, without limitation, a peptide linker containing the amino acid sequence set forth in any of SEQ ID NOs: 315-325. Note that in the peptide linker, the bond between amino acids or between an amino acid and a chemical linker may be linked through the side chain of the amino acid. A linker may be added without limitation to change the physical properties (e.g., solubility) of the peptide.

The linker may be added at any position. For example, it may be linked to an amino acid located at the C-terminal side, or to an amino acid contained in a cyclic peptide. In one embodiment, the above peptide contains a linker at the C-terminus. Preferably, it is linked to Cys located at the C-terminal side, or to the side chain of an amino acid contained in a cyclic peptide.

Further, the peptide may form a multimer via, for example, a linker. The number of peptides in the multimer is not limited. In one embodiment, the multimer is a dimer, trimer, tetramer, pentamer, hexamer, octamer, or more. The multimer may contain multiple copies of the same peptide or multiple different peptides.

In one embodiment, the above peptide preferably has myostatin inhibitory activity.

In one embodiment, the above peptide preferably has GDF-8 inhibitory activity.

Myostatin, also called GDF-8 (Growth Differentiation Factor-8), is a protein and a member of the TGF-β (Transforming Growth Factor-β) superfamily.

The term "myostatin" as used herein refers to the natural-type myostatin possessed by mammals, preferably rodents such as mice and primates such as humans, unless otherwise specified.

Note that the term "human myostatin" herein refers to the natural-type myostatin possessed by humans (e.g., Gene ID: 2660). Incidentally, it is known that the amino acid sequences of human, mouse, and rat myostatin are almost identical.

Unless otherwise specified, the "myostatin" and "human myostatin" herein include unprocessed myostatin, processed myostatin, and mutants thereof. Myostatin is also known to be glycosylated and dimerized, and active myostatin (also called mature myostatin) is a homodimer consisting of two C-terminal growth factor domains, which are disulfide-linked.

Myostatin inhibition refers to the inhibition, prevention, antagonism, or competitive inhibition of myostatin signaling. The myostatin signaling refers to intracellular signaling in which myostatin binds to the type II activin receptor (ActRII: Gene ID:92), causing formation of a complex with the type I activin receptor (ActRI: Gene ID:90). That is, the inhibition of myostatin signaling includes, for example, inhibition of the binding of myostatin to ActRII by binding of a peptide to myostatin or to ActRII; inhibition of formation of an ActRI-ActRII complex by binding to the ActR1; and inhibition of a signaling pathway from the ActRI-ActRII complex, including, for example, inhibition of a signaling pathway mediated via Smad, PI3K, Ras, or TAK1. Note that the inhibition of a signaling pathway means reduction, modulation, or inhibition of any of the signaling.

The "myostatin inhibitory activity" can be evaluated using known methods. For example, as shown in the Examples herein, the activity can be evaluated by a system for measuring an *in vitro* myostatin inhibitory activity while using a reporter gene, or by measuring the body weight and muscle strength of mice. Alternatively, the myostatin inhibitory activity may be evaluated by the methods disclosed in, for example, JP-A-2020-011965 and JP-A-2022-101593.

Although not limited, the myostatin inhibitory activity may be expressed as IC50, for example, in a system for measuring an *in vitro* myostatin inhibitory activity while using a reporter gene.

In one embodiment, the peptide is myostatin-selective (specific). In addition, the peptide more preferably has low inhibitory activity against GDF-11 activity.

It is known that many molecules with myostatin inhibitory activity inhibit not only the activity of myostatin, i.e., GDF-8, but also the activity of GDF-11, which belongs to the same TGF- *β* superfamily. The amino acid sequences of the above active myostatin and GDF-11 have high identity, and furthermore, myostatin, GDF-11, and activin are all ActR-mediated signal transducers. Therefore, molecules such as antibodies with myostatin inhibitory activity often inhibit the activity of all three growth factors: myostatin, GDF-11, and activin. However, preferably, GDF-11 should not be inhibited, especially in children and young adults, because of its muscle function-enhancing effects as a rejuvenating factor. In one embodiment, preferred is a myostatin inhibitor that specifically inhibits only myostatin and not myostatin signal transducers (e.g., GDF-11) other than myostatin. Incidentally, GDF-11 is also highly homologous among mice, humans, and rats. References herein to "GDF-11" are preferably to human GDF-11 (Gene ID: 10220).

Note that the wording "does not inhibit GDF-11" means that the inhibitory activity against GDF-11 signaling is lower than that against myostatin signaling. For example, it means that the IC50 for GDF-11 is 2, 3, 4, 5, 10, 20, 30, 40, 50, 100, 200, or more times higher than the IC50 for myostatin in the system for measuring an *in vitro* myostatin inhibitory activity while using a reporter gene.

In one embodiment, the peptide of the present invention has inhibitory activity against myostatin derived from multiple species. The above peptide preferably has inhibitory activity against human-derived myostatin.

The present invention provides a novel peptide with myostatin inhibitory activity. The peptide with myostatin inhibitory activity is preferably a peptide that does not inhibit GDF-11 when the GDF-11 inhibitory activity is compared with myostatin inhibitory activity. This has been demonstrated by, for example, Examples 3 and 4 herein, suggesting that while the peptide has inhibitory activity against myostatin, the inhibitory activity against GDF-11 is much lower than that against myostatin.

The peptide with myostatin inhibitory activity is effective in the treatment and prevention of various BMP-mediated diseases. The peptide with myostatin inhibitory activity is effective in various experiments on various myostatin-mediated diseases.

As used herein, the "peptide" includes both "2. Peptide (A)" and "3. Peptide (B)" unless otherwise indicated.

The matters described in other sections also apply to this section, unless otherwise noted.

### 4. Production of Peptide

The peptide of the present invention can be produced by any known method for peptide production. Examples include the following.

The matters described in other sections also apply to this section, unless otherwise noted.

A chemical synthesis method such as a liquid-phase method, a solid-phase method, a hybrid method in which liquid-phase and solid-phase methods are combined; and a genetic recombinant method.

In the solid-phase method, for example, the hydroxyl group of a hydroxylated resin is esterified with the carboxy group of the first amino acid, α-amino group of which is protected by a protecting group (usually the C-terminal amino acid of the peptide of interest). The esterification catalyst used may be a known dehydration-condensation agent such as 1-mesitylene sulfonyl-3-nitro-1,2,4-triazole (MSNT), dicyclohexylcarbodiimide (DCC), or diisopropylcarbodiimide (DIC).

Next, the protecting group of the α-amino group of the first amino acid is eliminated and a second amino acid in which all functional groups other than the carboxy group of the main chain are protected is added to activate the carboxy group, and the first and second amino acids are coupled. Further, the α-amino group of the second amino acid is deprotected and a third amino acid in which all functional groups other than the carboxy group of the main chain are protected is added to activate the carboxy group, and the second and third amino acids are coupled. This procedure is repeated until a peptide of the desired length is synthesized, and all the functional groups are then deprotected.

Examples of the resin for solid-phase synthesis include Merrifield resin, MBHA resin, Cl-Trt resin, SASRIN resin, Wang resin, Rink amide resin, HMFS resin, Amino-PEGA resin (Merck), or HMPA-PEGA resin (Merck). These resins may be washed with a solvent (e.g., dimethylformamide (DMF), 2-propanol, methylene chloride) before use.

Examples of the protecting group for α-amino group include a benzyloxycarbonyl (Cbz or Z) group, a tert-butoxycarbonyl (Boc) group, a 9-fluorenylmethyloxycarbonyl (Fmoc) group, a benzyl group, an allyl group, or an allyloxycarbonyl (Alloc) group. The Cbz group may be deprotected, for example, with hydrofluoric acid or by hydrogenation, the Boc group may be deprotected with trifluoroacetic acid (TFA), and the Fmoc group may be deprotected by treatment with piperidine or pyrrolidine.

The α-carboxy group may be protected using, for example, methyl ester, ethyl ester, allyl ester, benzyl ester, *tert-butyl* ester, or cyclohexyl ester.

The carboxy group may be activated using a condensing agent. Examples of the condensing agent include dicyclohexylcarbodiimide (DCC), diisopropylcarbodiimide (DIC), 1-ethyl-3-(3-dimethylaminopropyl)carbodiimide (EDC or WSC), (1H-benzotriazol-1-yloxy)tris(dimethylamino)phosphonium hexafluorophosphate (BOP), or 1-[bis(dimethylamino)methyl]-1H-benzotriazolium-3-oxide hexafluorophosphate (HBTU).

The peptide chain may be cleaved from the resin by treatment with an acid such as TFA or hydrogen fluoride (HF).

The peptide may be produced by genetic recombination method (translation-synthesis system) while using a nucleic acid encoding the peptide described above. The nucleic acid encoding the peptide may be DNA or RNA.

The nucleic acid encoding the peptide may be prepared by a known method or an equivalent method. For example, the nucleic acid can be synthesized with an automatic synthesizer. The resulting DNA may contain a restriction enzyme recognition site(s) for vector insertion. Alternatively, a nucleotide sequence encoding an amino acid sequence may be incorporated, so that the resulting peptide chain can be cut out by enzyme or other means.

To inhibit degradation by host-derived proteases, a chimeric protein expression method, in which a target peptide is expressed as a chimeric peptide with another peptide, can also be used. In this case, it is possible to use, as the above nucleic acid, a nucleic acid encoding the target peptide and a peptide fused to the target peptide.

Then, an expression vector is prepared using the nucleic acid encoding the peptides. The nucleic acid can be inserted downstream of the promoter of the expression vector either as is or by digestion with restriction enzyme(s) or by adding a linker. Examples of the vector include: an E. coli-derived plasmid (e.g., pBR322, pBR325, pUC12, pUC13, pUC18, pUC19, pUC118, pBluescript II); *a Bacillus subtilis-derived* plasmid (e.g., pUB110, pTP5, pC1912, pTP4, pE194, pC194); a yeast-derived plasmid (e.g., pSH19, pSH15, YEp, YRp, YIp, YAC); a bacteriophage (e.g., e-phage, M13 phage); a virus (e.g., retrovirus, vaccinia virus, adenovirus, adeno-associated virus (AAV), cauliflower mosaic virus, tobacco mosaic virus, baculovirus); or a cosmid.

The promoter can be selected, if appropriate, according to the type of host. If the host is an animal cell, for example, an SV40 (simian virus 40)-derived promoter or a CMV (cytomegalovirus)-derived promoter can be used. If the host is *E. coli,* the trp promoter, T7 promoter, or lac promoter, for example, can be used.

The expression vector may have, for example, a DNA replication origin (ori), a selection marker (e.g., antibiotic resistance, nutrient requirement), an enhancer, a splicing signal, a poly-A addition signal, and/or a tag (e.g., FLAG, HA, GST, GFP)-encoding nucleic acid.

Next, an appropriate host cell is transfected with the expression vector. The host may be selected, if appropriate, in view of the relation to the vector. Examples of the host used include *E. coli, Bacillus subtilis,* a bacterium belonging to the genus *Bacillus,* yeast, an insect or insect cell, or an animal cell. Examples of the animal cell used include an HEK293T cell, a CHO cell, a COS cell, a myeloma cell, a HeLa cell, or a Vero cell. The transfection may be performed according to the type of host by a known method such as lipofection, a calcium phosphate method, electroporation, microinjection, particle gun, or other known methods. The transformants may be cultured according to a routine procedure to express a peptide of interest.

The peptide may be purified from the transformant culture by collecting the cultured cells, by suspending the cells in an appropriate buffer solution, by, for instance, sonication or freeze-thawing to destroy the cells, and then by centrifugation or filtration to obtain a crude extract. If the peptide is secreted into the culture medium, the supernatant is collected.

Purification from the crude extract or culture supernatant can also be performed by a known method or an equivalent method (e.g., chlorination, dialysis, ultrafiltration, gel filtration, SDS-PAGE, ion exchange chromatography, affinity chromatography, reverse-phase high-performance liquid chromatography).

A known method or an equivalent method may be used to convert the resulting peptide in a free form to a salt, or the peptide in a salt form to a free form.

In one embodiment, the translation synthesis system may be a cell-free translation system. The cell-free translation system generally allows an expressed product to be obtained in a highly pure form without purification. The cell-free translation system contains, for example, ribosomal proteins, aminoacyl-tRNA synthetase (ARS), ribosomal RNA, amino acids, rRNA, GTP, ATP, translation initiation factor (IF), elongation factor (EF), termination factor (RF), and ribosome regeneration factor (RRF), and other factors necessary for translation. *E. coli* extract or wheat germ extract may be added to increase expression efficiency. In addition, rabbit red blood cell extract or insect cell extract may also be added.

Energy may be continuously supplied to the system containing them by using dialysis to produce, in a non-limiting manner, several hundred µg to several mg/mL of the protein. The system may also include RNA polymerase for transcription from the gene DNA. As a commercially available cell-free translation system that can be used, examples of the E. coli-derived system include Roche Diagnostics' RTS-100 (registered trademark), Gene Frontier's PURESYSTEM, or NEW ENGLAND Biolabs PURExpress *In Vitro* Protein Synthesis Kit; and examples of the system using wheat germ extract include those from ZOEGENE Corporation and CellFree Sciences Co., Ltd..

In the cellular translation system, instead of aminoacyl-tRNA synthesized by natural aminoacyl-tRNA synthetase, an artificial aminoacyl-tRNA in which the desired amino acid or hydroxy acid is linked (acylated) to the tRNA may be used. Such aminoacyl-tRNA can be synthesized using an artificial ribozyme.

Examples of such a ribozyme include flexizyme (H. Murakami, H. Saito, and H. Suga, (2003), Chemistry & Biology, Vol.10, 655-662; and WO2007/066627 or others). The flexizyme is also known as and called the original Flexizyme (Fx) or a modified form such as dinitrobenzylflexizyme (dFx), enhanced Flexizyme (eFx), or aminoflexizyme (aFx).

By using the tRNA, generated by flexizyme, to which the desired amino acid or hydroxy acid is linked, the desired codon can be associated with the desired amino acid or hydroxy acid for translation. A special amino acid may be used as the desired amino acid. For example, the non-natural amino acid required for the cyclization described above can also be introduced into the peptide linked by this method.

The peptide may be chemically synthesized using various methods routinely used in the art. Examples of the method include stepwise solid-phase synthesis, semi-synthesis of peptide fragment via conformationally assisted re-ligation, or chemical ligation. The synthesis of the peptide is a chemical synthesis using various solid-phase techniques as described, for example, in K. J. Jensen, P. T. Shelton, S. L. Pedersen, Peptide Synthesis and Applications, 2nd Edition, Springer, 2013. The preferred strategy is based on a combination of a Fmoc group that temporarily protects the α-amino group and allows for selective removal by a base, and a protecting group that temporarily protects the side chain functional group and is stable under de-Fmoc conditions. Such general peptide side chain selection is described in the above Peptide Synthesis and Applications, 2nd edition and G. B. Fields, R. L. Noble, Solid Phase Peptide Synthesis Utilizing 9-Fluorenylmethoxycarbonyl Amino Acids, Int. J. Peptide Protein Res. 35, 1990, 161-214. Examples of the preferred peptide side chain protecting group include: a benzyl, *tert-butyl,* or trityl (Trt) group for the hydroxy group of serine or threonine; a 2-bromobenzyloxycarbonyl or *tert-*butyl group for the hydroxy group of tyrosine; a Boc, methyltetrazole thiol (Mtt), Alloc, or ivDde group for the amino group of the lysine side chain; a Trt or Boc group for the imidazole group of histidine; a 2,2,4,6,7-pentamethyldihydrobenzofuran-5-sulfonyl (Pbf) group for the guanidyl group of arginine; a *tert-*butyl*,* allyl, or 3-methylpentane (Mpe) group for the carboxyl group of, for instance, glutamic acid or aspartic acid; a Trt group for the carboxamide group of glutamine or asparagine; or a Trt or monomethoxytrityl (Mmt) group for the thiol group of cysteine.

The peptide can be synthesized in a stepwise manner on the solid-phase resin described above. The α-amino protecting groups of the C-terminal amino acid used and all amino acids and the peptide used in the synthesis should be selectively removed in the synthetic process. Preferably, the above-described solid-phase resin is used to initiate the process. The C-terminal carboxyl group of the peptide with the N-terminus appropriately protected with a Fmoc group or the like or the C-terminal carboxyl group of a Fmoc group-protected amino acid is made into an activated ester with an appropriate reagent and then coupled to the amino group on the solid-phase resin. Subsequent elongation of the peptide chain can be achieved by sequentially repeating the removal of the N-terminal protecting group (Fmoc group), followed by condensation with a protected amino acid derivative, according to the amino acid sequence of the peptide of interest. Note that the peptide of interest can be released at the final stage. For example, as the release conditions, TFA solution containing water/silylhydride/thiol as a scavenger in TFA, as described in, for instance, Teixeira, W. E. Benckhuijsen, P. E. de Koning, A. R. P. M. Valentijn, J. W. Drijfhout, Protein Pept. Lett. 2002, 9, 379-385, can be used for the release. A typical example is TFA/Water/TIS/DODT (volume ratio 92.5:2.5:2.5:2.5).

The peptide described herein may be synthesized using a single or multichannel peptide synthesizer, such as CEM's Liberty Blue synthesizer or Biotage's Syro I synthesizer or their successors.

The carboxy group may be activated using a condensing agent. Examples of the condensing agent include dicyclohexylcarbodiimide (DCC), diisopropylcarbodiimide (DIPCDI), 1-ethyl-3-(3-dimethylaminopropyl)carbodiimide (EDC or WSC), (1H-benzotriazol-1-yloxy)tris(dimethylamino)phosphonium hexafluorophosphate (BOP), or 1-[bis(dimethylamino)methyl]-1H-benzotriazolium-3-oxide hexafluorophosphate (HBTU).

The peptide can be cyclized according to a known procedure. Although not limited, for example, the peptide may be designed to contain two or more cysteine residues, so that a cyclic structure can be formed through a disulfide bond after translation. In addition, Goto's method (Y. Goto, et al., ACS Chem. Biol., 3, 120-129 (2008)) may be used to synthesize a peptide with a chloroacetyl group at the N-terminus by genetic code reprogramming technique. In the peptide, a cysteine residue containing a sulfur molecule may be placed for cyclization. This causes the spontaneous nucleophilic attack of the mercapto group on the chloroacetyl group after translation, and the peptide is cyclized through a thioether bond. Another combination of amino acids that are bonded to form a ring may be placed, for cyclization, within the peptide by genetic code reprogramming technique. Alternatively, the peptide may be cyclized by placing an L-2-amino adipic acid residue in the peptide and bonding it to the N-terminal main chain amino group. In this way, any known cyclization method can be used without restriction.

The peptide may further adopt a bicyclic structure in which crosslinking is formed within the ring. The formation of bicyclic structure may be performed by known methods. For example, although not limited, a peptide with a bicyclic structure can be obtained by linking two side chains of amino acids in a cyclic structure, with or without any further amino acid(s) or the like.

### 5. Composition, etc.

The present invention relates to a composition containing a peptide of the present invention or a pharmaceutically acceptable salt thereof. The composition is not limited, and may be a pharmaceutical composition (a composition for medical use), a diagnostic composition, or a therapeutic composition.

The matters described in other sections also apply to this section, unless otherwise noted.

### Pharmaceutical Composition

In one embodiment, the present invention relates to a pharmaceutical composition containing the above peptide or a pharmaceutically acceptable salt thereof. The target disease of the pharmaceutical composition is not particularly limited to any disease if the above-mentioned peptide itself is effective.

In one embodiment, the above pharmaceutical composition has myostatin inhibitory activity.

In one embodiment, the pharmaceutical composition is a pharmaceutical composition for preventing or treating a myostatin-related disease or a disease or symptom associated with reduced muscle function.

The wording "myostatin-related disease or a disease or symptom associated with reduced muscle function" includes known myostatin-related diseases or diseases or symptoms associated with reduced muscle function. For example, the diseases include, without limitation, diseases originating from muscle tissue, such as myopathy and myogenic muscular atrophy, as well as diseases originating from the nervous system, such as neurogenic muscular atrophy.

In one embodiment, the term "myostatin-related diseases" includes muscular dystrophies (including Duchenne muscular dystrophy (DMD), facioscapulohumeral muscular dystrophy (FSHD), limb-girdle muscular dystrophy (LGMD), Becker muscular dystrophy (BMD), and the like), myopathies (including congenital myopathy, inflammatory myopathy, metabolic myopathy, mitochondrial myopathy, endocrine myopathy, myofibrillar myopathy, distal myopathy, and the like), neurogenic muscular atrophy, amyotrophic lateral sclerosis (ALS), muscular atrophy, spinal muscular atrophy (SMA), and the like.

In one embodiment, the pharmaceutical composition of the present invention is for preventing a decrease in the amount of muscle fibers. In one embodiment, the pharmaceutical composition of the present invention is for increasing the amount of muscle fibers. In one embodiment, the pharmaceutical composition of the present invention is for increasing the strength of muscle fibers. In one embodiment, the pharmaceutical composition of the present invention suppresses necrosis of muscle fibers. In one embodiment, the pharmaceutical composition of the present invention suppresses fibrosis and adipose tissue formation secondary to necrosis of muscle fibers. Without limitation, the "disease or symptom associated with reduced muscle function" includes a decrease in the amount of muscle fibers, necrosis of muscle fibers, and fibrosis and adipose tissue formation secondary to necrosis of muscle fibers.

In one embodiment, the pharmaceutical composition is administered to an individual having a myostatin-related disease or a disease or symptom associated with reduced muscle function. The "individual" is preferably a human.

The pharmaceutical composition may contain the peptide itself, and may contain a pharmaceutically acceptable salt of the peptide, or a solvate thereof. As used herein, the "peptide" may include a pharmaceutically acceptable salt thereof, or a solvate thereof, unless otherwise specified. The pharmaceutical composition preferably contains an effective amount of the peptide as an active ingredient.

The dosage form of the pharmaceutical composition herein is not particularly limited and may be administered orally or parenterally. Examples of the parenteral administration include an injection (e.g., an intramuscular, intravenous, or subcutaneous injection) or transdermal or transmucosal (nasal, oral, ocular, transpulmonary, transvaginal, rectal) administration.

The pharmaceutical composition may be modified in various ways in view of the easy-to-metabolize or easy-to-excrete characteristics of the peptide. For example, polyethylene glycol (PEG) or sugar chain may be added to the polypeptide to increase the residence time in blood and decrease the antigenicity. In addition, for example, a biodegradable polymer compound (e.g., polylactic acid glycol (PLGA)), porous hydroxyapatite, liposome, surface-modified liposome, an emulsion prepared with unsaturated fatty acid, nanoparticles, or nanospheres may be used as a sustained release base material, and the polypeptide can be encapsulated in them. For transdermal administration, a weak electric current can be applied to the skin surface for stratum corneum permeation (iontophoresis method).

The pharmaceutical composition may be made into a preparation by using the active ingredient as it is, or by adding a pharmaceutically acceptable carrier, excipient, and/or additive. Examples of the dosage form include liquid (e.g., an injection), dispersion, suspension, tablets, rounds, powder, suppositories, pulvis, fine particles, granules, capsules, syrup, lozenges, inhalant, ointment, eye drops, nose drops, ear drops, or cataplasm. The preparation can be made by a common procedure using, for example, an excipient, a binder, a disintegrant, a lubricant, a dissolving agent, a dissolution aid, a colorant, an odor correcting agent, a stabilizer, an emulsifier, an absorption enhancer, a surfactant, a pH modifier, a preservative, and/or an antioxidant as appropriate.

Examples of the component used in the preparation include, but are not limited to, purified water, saline solution, phosphate buffer, dextrose, glycerol, ethanol or other pharmaceutically acceptable organic solvents, animal or vegetable oil, lactose, mannitol, glucose, sorbitol, crystalline cellulose, hydroxypropyl cellulose, starch, cornstarch, silicic anhydride, magnesium aluminum silicate, collagen, polyvinyl alcohol, polyvinyl pyrrolidone, carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthan gum, gum arabic, tragacanth, casein, agar, polyethylene glycol, diglycerin, glycerin, propylene glycol, vaseline, paraffin, octyldodecyl myristate, isopropyl myristate, higher alcohols, stearyl alcohol, stearic acid, or human serum albumin.

In view of the fact that peptides are generally difficult to be absorbed transmucosally, the pharmaceutical composition may contain an absorption enhancer for improving absorption of a poorly absorbable drug. Examples of such an absorption enhancer include: a surfactant (e.g., a polyoxyethylene lauryl ether compound, sodium lauryl sulfate, saponin); a salt of bile acid (e.g., glycolic acid, deoxycholic acid, taurocholic acid); a chelator (e.g., EDTA, salicylic acid); a fatty acid compound (e.g., caproic acid, capric acid, lauric acid, oleic acid, linoleic acid, a mixed micelle); or an enamine derivative, an N-acyl collagen peptide, N-acylamino acid, a cyclodextrin compound, a chitosan compound, or a nitric oxide donor.

If the pharmaceutical composition is a round(s) or a tablet(s), it may be coated with a sugar coating or a gastric-soluble or enteric-soluble substance.

If the pharmaceutical composition is an injection, it may contain distilled water for injection, physiological saline, propylene glycol, polyethylene glycol, vegetable oil, and/or an alcohol compound. In addition, it is possible to add a wetting agent, an emulsifier, a dispersant, a stabilizer, a dissolving agent, a dissolution aid, and/or a preservative.

The pharmaceutical composition may be targeted not only to humans but also to non-human mammals or birds. Examples of the non-human mammals include non-human primates (e.g., monkeys, chimpanzees, gorillas), domestic animals (e.g., pigs, cows, horses, sheep), or dogs, cats, rats, mice, guinea pigs, or rabbits.

In particular, the dose when administered to a human, varies depending on the symptoms, age, sex, weight, and sensitivity of the patient as well as the administration method, the administration interval, the type of active ingredient, and the type of preparation. The dose is not limited and the dose of, for example, from 30 µg to 100 g, from 100 µg to 500 mg, or from 100 µg to 100 mg can be administered in one or several separate portions. For injection, 1 µg/kg to 3000 µg/kg or 3 µg/kg to 1000 µg/kg may be administered once or in several separate doses depending on the patient's body weight.

In one embodiment, the present invention relates to a method of preventing or treating a myostatin-related disease or a disease or symptom associated with reduced muscle function, the method including administering a peptide of the present invention.

In one embodiment, the present invention relates to use of a peptide of the present invention for preventing or treating a myostatin-related disease or a disease or symptom associated with reduced muscle function.

In one embodiment, the present invention relates to use of a peptide of the present invention for the manufacture of a pharmaceutical composition for preventing or treating a myostatin-related disease or a disease or symptom associated with reduced muscle function.

In one embodiment, the present invention relates to use of a peptide of the present invention as a pharmaceutical composition for preventing or treating a myostatin-related disease or a disease or symptom associated with reduced muscle function.

In one embodiment, the present invention relates to a peptide of the present invention, the peptide having myostatin inhibitory activity.

In one embodiment, the present invention relates to a peptide of the present invention for use in a method of preventing or treating a myostatin-related disease or a disease or symptom associated with reduced muscle function.

In one embodiment, the present invention relates to a peptide of the present invention for use as a pharmaceutical composition for preventing or treating a myostatin-related disease or a disease or symptom associated with reduced muscle function.

In one embodiment, the present invention relates to a method of inhibiting myostatin signaling by using a peptide of the present invention. Examples of the method of the present invention include an *in vivo, ex vitro,* or *ex vivo* method.

### Diagnostic Composition

The present invention also relates to a diagnostic composition including a peptide of the present invention or a pharmaceutically acceptable salt thereof for diagnosing a myostatin-related disease or a disease or symptom associated with reduced muscle function.

The peptide of the present invention may also be used as a composition for diagnosing a myostatin-related disease or a disease or symptom associated with reduced muscle function. This diagnostic agent may be a detection agent for diagnosing whether a subject is affected by a myostatin-related disease or a disease or symptom associated with reduced muscle function, and, when used as a detection agent, the peptide may be detectably labeled.

The peptide may be detectably labeled. The peptide may be labeled with an enzyme (e.g., peroxidase, alkaline phosphatase), a radioactive substance (e.g., ¹²⁵I, ¹³¹I, ³⁵S, ³H), a fluorescent material (e.g., fluorescein isothiocyanate, rhodamine, dansyl chloride, phycoerythrin, tetramethylrhodamine isothiocyanate, a near infrared fluorescent material), or an antibody labeled with a luminescent substance (e.g., luciferase, luciferin, aequorin). In addition, an antibody labeled with nanoparticles such as gold colloids and quantum dots may be used for detection. For example, a complex may be formed between the peptide and an antibody that binds to a protein involving a myostatin-related disease or a disease or symptom associated with reduced muscle function; a complex having the antibody or the peptide labeled may be prepared; and, by administering and detecting the labeled complex, the severity and the like of the myostatin-related disease or the disease or symptom associated with reduced muscle function can be detected. Also, in immunoassay, the peptide may be labeled with biotin and detected by binding the biotin to avidin or streptavidin labeled with, for example, an enzyme.

Among immunoassays, an enzymatically linked ELISA is preferred because of its simplicity and rapid antigen measurement. For example, an antibody is immobilized on a solid-phase carrier, a sample is added and reacted, and then the labeled peptide is added and reacted. After washing, by reacting with an enzyme substrate to develop color and measuring absorbance, the severity and the like of a myostatin-related disease or a disease or symptom associated with reduced muscle function can be detected. After the reaction of the sample with the antibody immobilized on the solid-phase carrier, an unlabeled peptide may be added, and the antibody against the peptide may be enzyme-labeled and added further. The antibody may be immobilized on or inside the surface of the solid-phase carrier.

The enzyme substrate used may be, for instance, 3,3'-diaminobenzidine (DAB), 3, 3', 5, 5'-tetramethylbenzidine (TMB), or o-phenylenediamine (OPD) in the case where the enzyme is peroxidase and may be, for instance, p-nitrophenyl phosphate (pNPP) in the case of alkaline phosphatase.

The "solid-phase carrier" herein is not limited as long as the carrier can be used to immobilize the antibody. Examples include a glass, metal, or resin-made microtiter plate, substrate, or bead, or a nitrocellulose, nylon, or PVDF membrane. The target substance may be immobilized on the solid-phase carrier in accordance with a known procedure.

The present invention also relates to a diagnostic kit including the peptide of the present invention. The diagnostic kit includes a reagent and a tool (including, but is not limited to, any or all of the peptide of the present invention, an antibody, a solid-phase carrier, buffer solution, enzyme reaction stopping solution, and/or a microplate reader) necessary for the above detection.

The present invention relates to a method of diagnosing a myostatin-related disease or a disease or symptom associated with reduced muscle function, the method including using a peptide of the present invention. The "diagnosis method" include *in vivo* or *in vitro* diagnosis methods. Preferred is an *in vitro* diagnosis method. The present invention also relates to a method of detecting a disease by using the peptide of the present invention. The disease can be detected by, for instance, laboratory technicians or researchers other than physicians, at research institutes (including educational institutions such as universities), companies, or others. In one embodiment, the method of detecting a disease does not include medical treatment.

The present invention relates to use of a peptide of the present invention for diagnosing a myostatin-related disease or a disease or symptom associated with reduced muscle function.

The present invention relates to use of a peptide of the present invention for the manufacture of a diagnostic composition for diagnosing a myostatin-related disease or a disease or symptom associated with reduced muscle function.

The present invention relates to use of a peptide of the present invention as a diagnostic composition for diagnosing a myostatin-related disease or a disease or symptom associated with reduced muscle function.

The present invention relates to a peptide of the present invention for use in a method of diagnosing a myostatin-related disease or a disease or symptom associated with reduced muscle function.

The present invention relates to a peptide of the present invention for use as a diagnostic composition for diagnosing a myostatin-related disease or a disease or symptom associated with reduced muscle function.

The present invention relates to a tester including a peptide of the present invention. The present invention relates to a diagnostic or detection tester including a peptide of the present invention.

### Research Composition

The present invention also relates to a research composition including a peptide of the present invention. The "research composition" includes those used by, for instance, researchers, technicians, students, or doctors in research institutes (including educational institutions such as universities), companies, hospitals, or others.

The above research composition may be used, for example, for detection of myostatin or detection of a myostatin-related disease or a disease or symptom associated with reduced muscle function.

The carrier for immobilizing the peptide is not limited herein. Examples include a glass, metal, or resin-made microtiter plate, substrate, or bead, or a nitrocellulose, nylon, or PVDF membrane.

The present invention includes a method of detecting myostatin by using a peptide of the present invention.

The present invention also includes use of a peptide of the present invention for detection of myostatin.

The present invention also relates to a diagnostic or detection kit including a peptide of the present invention. The diagnostic or detection kit includes a reagent and a tool (including, but is not limited to, any or all of the peptide of the present invention, an antibody, a solid-phase carrier, buffer solution, enzyme reaction stopping solution, and/or a microplate reader) necessary for the above detection.

The present invention also relates to a method of testing a peptide or a pharmaceutically acceptable salt thereof, including testing at least one of
a) solubility in a solvent,
b) myostatin inhibitory activity,
c) cell and/or tissue toxicity, or
d) toxicity to an experimental animal,
wherein the peptide or pharmaceutically acceptable salt thereof is the peptide or pharmaceutically acceptable salt thereof according to any one of claims 1 to 7.

The "solubility in a solvent" can be measured using known methods. In measuring solubility, the solvent is not limited and may be freely selected depending on the intended purpose. As for the method of solubility measurement, any of the known methods can be appropriately selected depending on the type of solvent. Without limitation, the solubility may be the solubility obtained when the peptide is dissolved in a known solvent such as water, glycerol, PBS, or DMSO.

The "myostatin inhibitory activity" may be measured, for example, as described in "3. Peptide (B)".

The "cell and/or tissue toxicity" can be measured using known methods. For example, testing for the cell and/or tissue toxicity may be a known toxicity assessment test using cells and/or tissues, and may be a method performed *in vitro.* The cells and/or tissues may be, but are not limited to, cells and/or tissues that are normally evaluated and tested for toxicity for pharmaceutical products.

The "toxicity to an experimental animal" can be measured using known methods. For example, experimental animals are not particularly limited as long as they are those commonly used, and examples thereof include mice, rats, guinea pigs, gerbils, hamsters, ferrets, rabbits, dogs, cats, pigs, goats, horses, cattle, birds (e.g., chickens, quails), monkeys, and non-human primates (e.g., cynomolgus monkeys, marmosets, rhesus monkeys).

In addition, the above toxicity evaluation tests may be, but are not limited to, safety studies that are usually conducted in non-clinical studies of pharmaceutical products. Examples include general toxicity studies (single-dose and repeated-dose toxicity studies), genotoxicity studies (Ames test, chromosomal aberration test, *in vitro* micronucleus test), carcinogenicity studies, reproductive and developmental toxicity studies (ICH-I, II, III), local irritation studies (ocular and skin irritation studies or the like), other toxicity studies (skin sensitization test, phototoxicity test, antigenicity test), and chemical and bioanalytical evaluations (TK/PK).

### 6. Combinatory Use, Combination

The present invention may be used in combination with a medicament for preventing or treating another myostatin-related disease or disease or symptom associated with reduced muscle function. In one embodiment, the present invention relates to a combination of the peptide and a medicament for preventing or treating another myostatin-related disease or disease or symptom associated with reduced muscle function.

The peptide and a medicament for preventing or treating another myostatin-related disease or disease or symptom associated with reduced muscle function may be administered simultaneously or sequentially. Preferably, the peptide and a medicament for preventing or treating another myostatin-related disease or disease or symptom associated with reduced muscle function are administered so as to achieve an additive effect of both, preferably a synergetic effect. When administered sequentially, the order of administration does not matter. When administered sequentially, it is preferable, without limitation, that both are administered within 2 hours, within 1 hour, within 30 min, within 20 min, within 15 min, within 10 min, or within 5 min.

The matters described in other sections also apply to this section, unless otherwise noted.

### EXAMPLES

Hereinafter, the present invention is further described in detail with reference to Examples. The present invention, however, is not limited to them. A person skilled in the art may easily make modifications and changes to the present invention based on the description herein, and they are included in the technical scope of the present invention. Note that the compound names given in the following Reference Examples and Examples do not necessarily follow the IUPAC nomenclature. Also note that abbreviations may be used for the sake of simplicity of description, but these abbreviations are as stated above.

Raw materials, building blocks, reagents, acids, bases, solid-phase resins, and solvents used in the chemical synthesis of the compounds were either commercially available products as they were or, when otherwise specified, were synthesized using organic chemistry. Note that a protecting group-containing amino acids were commercially available products as they were.

As for the numbering of peptide residues, the amino acid residue that is ClAc-modified is counted as residue 1, and the subsequent residues are counted as residue 2, residue 3, and so on, toward the resin. The common amino acids used are listed below, and each side chain protecting group is indicated in parentheses.
Fmoc-Phe-OH;
Fmoc-Val-OH;
Fmoc-Trp(Boc)-OH;
Fmoc-Arg(Pbf)-OH;
Fmoc-Ala-OH H₂O;
Fmoc-Gly-OH;
Fmoc-N-Me-Tyr(tBu)-OH;
Fmoc-Ile-OH;
Fmoc-Leu-OH;
Fmoc-Asn(Trt)-OH;
Fmoc-Asp(OMpe)-OH;
Fmoc-(Dmb)Gly-OH (CAS No. 166881-42-1);
Fmoc-D-Pro-OH;
Fmoc-Cys(Trt)-OH;
Fmoc-Lys(Boc)-OH

In addition, for non-natural amino acids, the following and those listed in the abbreviations were used.
Fmoc-dp-OH;
Fmoc-de(tBu)-OH;
Fmoc-KCOpipzaa(tBu)-OH;
Fmoc-W4N-OH;
Fmoc-W7N-OH;
Fmoc-Me4Py-OH;
Fmoc-MeE(tBu)-OH;
Fmoc-MeF-OH;
Fmoc-MeY(tBu)-OH;
Fmoc-MeA-OH;
Fmoc-MeG-OH;
Fmoc-W4F-OH;
Fmoc-W7F-OH;
Fmoc-MeF2F-OH;
Fmoc-3Py6NH2(Boc)-OH;
Fmoc-Kac-OH;
Fmoc-W73Pyz1Me(Boc)-OH;
Fmoc-W7Ph3C-OH;
Fmoc-MeF4C-OH;
Fmoc-PEG8c-OH;
Fmoc-W6H(Boc)1Ph4COO(tBu)-OH;
Fmoc-W6H(Boc,Boc)-OH;
Fmoc-MeDap(alloc)-OH (CAS: 2973753-13-6);
Fmoc-MeF4am(alloc)-OH (CAS: 2973754-55-9);
Fmoc-MeHgl(allyl)-OH (2973754-77-5);
Fmoc-Hgl(allyl)-OH (CAS: 133464-45-6);
Fmoc-CeG(allyl)-OH (CAS: 174800-18-1);
Fmoc-Ano-OH (CAS: 1262886-65-6);
Fmoc-Ado-OH (CAS: 1338002-17-7);
Fmoc-F4COO(tBu)-OH (CAS: 183070-44-2);
Fmoc-F3aao(tBu)-OH (CAS: 2973753-95-4);
Fmoc-CrpG(tBu)-OH (CAS: 174799-90-7);
Fmoc-de(allyl)-OH (CAS: 204251-33-2);
Fmoc-da-OH (CAS: 79990-15-1);
Fmoc-W1Ph4COO(tBu)-OH;
Fmoc-CrpG(allyl)-OH (CAS: 1403683-46-4);
Fmoc-4Py-OH (CAS: 169555-95-7);
Fmoc-ApG(Boc)-OH (CAS: 143192-31-8);
Fmoc-Dap(Boc)-OH (CAS: 162558-25-0);
Fmoc-F4CON-OH (CAS: 204716-17-6);
Fmoc-Cha-OH (CAS: 135673-97-1);
Fmoc-Pip4mAc-OH (CAS: 154938-68-8);
Fmoc-EtG-OH (CAS: 162545-29-1);
Fmoc-Bph-OH (CAS: 199110-64-0);
Fmoc-F44Py-OH (CAS: 1282042-56-1);
Fmoc-Ahp-OH (CAS: 1197020-22-6);
Fmoc-Me3Py-OH (CAS: 1979173-93-7);
Fmoc-Ade-OH (CAS: 193885-59-5);
Fmoc-F4F-OH (CAS: 169243-86-1);
Fmoc-PipzAc-OH (CAS: 180576-05-0);
Fmoc-F3CON-OH (CAS: 959573-22-9);
Fmoc-dkCOpipzaa(tBu)-OH (CAS: 2973752-83-7);
Fmoc-MeKCOpipzaa(tBu)-OH (CAS: 2973752-84-8);
Fmoc-MeC(Trt)-OH (CAS: 944797-51-7);
Fmoc-Medc(Trt)-OH (CAS: 1349807-46-0);
Fmoc-Hcy(Trt)-OH (CAS: 167015-23-8);
Fmoc-dhcy(Trt)-OH (CAS: 2991226-74-3);
Fmoc-Aeoac-OH (CAS: 260367-12-2);
Fmoc-Ape-OH (CAS: 123622-48-0);
Fmoc-Pic4-OH (CAS: 148928-15-8);
Fmoc-ddab(alloc)-OH (CAS: 387824-78-4);
Fmoc-dorn(alloc)-OH (CAS: 214750-74-0);
Fmoc-KMe(alloc)-OH (CAS: 2246708-86-9);
Fmoc-mBph-OH (CAS: 1260616-69-0);
Fmoc-MeE(allyl)-OH (CAS: 2444356-85-6);
Fmoc-de-OtBu (CAS: 109745-15-5);
Fmoc-dcaa-OtBu

The structure of each chemically synthesized peptide was determined by ESI-MS(+) in mass spectrometry to confirm the molecular weight calculated by considering the amino acids used according to the target sequence and the building blocks used as necessary. Note that the term "ESI-MS(+)" indicates electrospray ionization mass spectrometry performed in positive ion mode. The detected masses are reported in "m/z" units. Note that when the molecular weight was approximately larger than 1000, the compound was frequently detected as a divalent or trivalent ion.

### Analytical conditions A

Column: Kinetex EVO C18 2.6 µm, 2.1 ID × 150 mm, 100 Å (Phenomenex, Inc.)
Column temperature: 60°C
Mobile phase A: 0.025% TFA (in water)
Mobile phase B: 0.025% TFA (in CH₃CN)
Gradient: 20-60% over 20 min
Flow rate: 0.25 mL/min
Detection: PDA (225 nm)

### Analytical conditions B

Column: Kinetex EVO C18 2.6 µm, 2.1 ID × 150 mm, 100 Å (Phenomenex, Inc.)
Column temperature: 60°C
Mobile phase A: 0.025% TFA (in water)
Mobile phase B: 0.025% TFA (in CH₃CN)
Gradient: 20-60% over 7.2 min
Flow rate: 0.5 mL/min
Detection: PDA (225 nm)

### Analytical conditions C

Column: Kinetex EVO C18 2.6 µm, 2.1 ID × 150 mm, 100 Å (Phenomenex, Inc.)
Column temperature: 60°C
Mobile phase A: 0.025% TFA (in water)
Mobile phase B: 0.025% TFA (in CH₃CN)
Gradient: 40-80% over 7.2 min
Flow rate: 0.5 mL/min
Detection: PDA (225 nm)

### Analytical conditions D

Column: Kinetex EVO C18 2.6 µm, 2.1 ID × 150 mm, 100 Å (Phenomenex, Inc.)
Column temperature: 60°C
Mobile phase A: 0.025% TFA (in water)
Mobile phase B: 0.025% TFA (in CH₃CN)
Gradient: 5-45% over 7.2 min
Flow rate: 0.5 mL/min
Detection: PDA (225 nm)

### Example 1:

In this Example, the following peptides or linker-attached peptides were synthesized.

### Example 1-1: Synthesis of Myostatin 99 v145 (SEQ ID NO: 30)

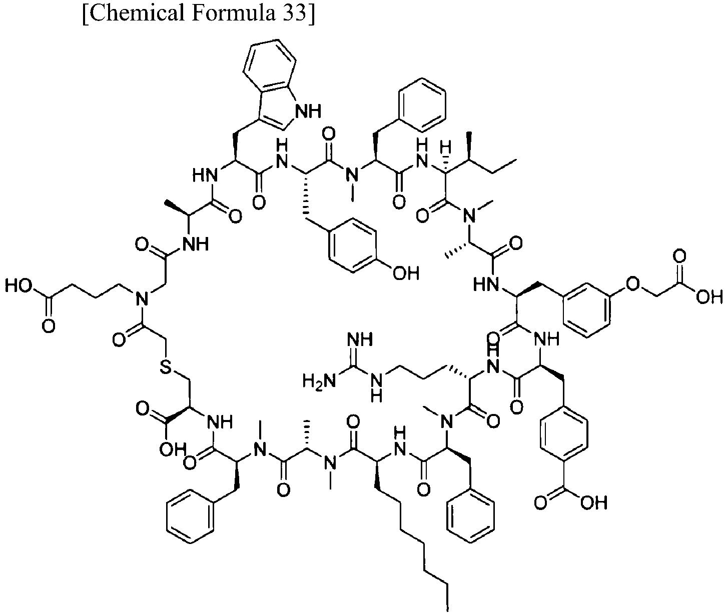

H-dc(Trt)-Trt(2-Cl) resin (Watanabe Chemical Industries, LTD.; 0.83 mmol/g, 1.20 g) was used to synthesize the target peptide. At that time, CEM's Liberty Blue was used as a solid-phase synthesizer for synthesis according to the manufacturer's instruction.

For the introduction of each residue, 0.21 M Fmoc-AA (in DMF)/0.5 M HATU (in DMF)/1 M DIEA (in DMF) (4.2 equivalents/4 equivalents/8 equivalents) were used per equivalent of resin, and the reaction was carried out once for 30 min at 25°C. Meanwhile, the reaction was carried out twice for residues 1, 4, 10, 12, and 13 at 25°C for 30 min. The reaction was carried out twice for residue 6 at 25°C for 60 min. In addition, the Fmoc was removed by a reaction twice with a 20% piperidine-containing DMF solution at 25°C for 5 min.

A chloroacetyl group was introduced as follows: the Fmoc group of the α-amino group was removed from the solid-phase resin, on which the Fmoc-protected peptide obtained in the previous step was immobilized, by the procedure described above; and 0.13 M ClAcOH (in DMF)/0.13 M HATU (in DMF)/0.25 M DIEA (in DMF) (5.2 equivalents/5 equivalents/10 equivalents) were added to the solid-phase resin and the mixture was shaken for 60 min at 25°C.

For the deprotection of side chains and the cleavage from the solid-phase resin, the resin obtained after the chloroacetyl group introduction step was first washed five times with DMF and three times with methylene chloride, then washed with diethyl ether, and then dried under reduced pressure. The reaction agent cocktail (30 mL; a mixture of TFA/H₂O/TIS/DODT at a volume ratio of 90:2.5:2.5:5.0) was then added to a reaction vessel containing the solid-phase resin, and the vessel was shaken at room temperature for 30 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diethyl ether/hexane (1/1) mixed solvent cooled to 0°C, a cloudy precipitate occurred.

The mixture was centrifuged (at 9000 rpm at 0°C for 2 min), and the solution was then decanted. The resulting solid was washed again with a small amount of diethyl ether cooled to 0°C and then dried under reduced pressure. The solid (peptide) obtained was used in the next cyclization reaction. The cyclization reaction of the peptide was performed by dissolving the peptide in DMSO/water/acetonitrile (2/1/1) to a final concentration of 5 mM based on the number of moles of the solid-phase resin; adding 10 equivalents of triethylamine; stirring the mixture at room temperature for 60 min; and adding DODT at a concentration of 1%. The resulting reaction solution was concentrated under reduced pressure by using Genevac HT-12.

The crude product obtained was purified using the following conditions (column: Waters XBridge (registered trademark) C18 5 µm 50 × 250 mm; mobile phase: A = 0.1% TFA (in water), B = 0.1% TFA (in MeCN); temperature: 50°C; gradient (%B): 15.4-12.2% over 0.1 min, 12.2-12.2% over 4.9 min, 12.2-15.4% over 2 min, 15.4-40.8% over 3 min, 40.8-45.9% over 15 min, 45.9-60% over 3 min; flow rate: 118-18 mL/min over 0.1 min, 18-18 mL/min over 4.9 min, 18-118 mL/min over 2 min, then 118 mL/min). After lyophilization, purification was performed using the following conditions (column: Waters XBridge (registered trademark) C18 5 µm 50 × 150 mm; mobile phase: A = 0.1% TFA (in water), B = 0.1% TFA (in MeCN); temperature: 50°C; gradient (%B): 15-15% over 2 min, 15-40% over 1 min, 40-45% over 8 min, then 45-60% over 1 min; flow rate: 20-20 mL/min over 1 min, 20-120 mL/min over 1 min, then 120 ml/min).

The purity of the target product was calculated from the area ratio of LC/MS (UV wavelength: 225 nm) chromatogram under the analytical conditions. The purity of the target product was 99.1%.

Analytical conditions: retention time = 5.96 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 1109.04 (M+2H)²⁺

### Example 1-2: Synthesis of Myostatin 99 Variant 07 (SEQ ID NO: 3)

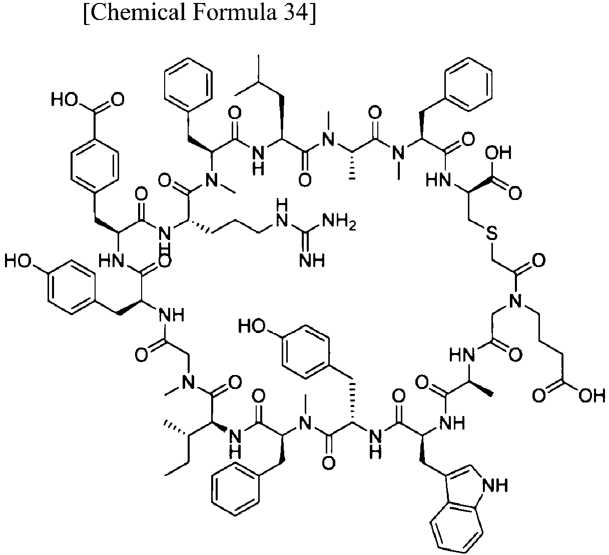

H-dc(Trt)-Trt(2-Cl) resin (Watanabe Chemical Industries, LTD.; 0.83 mmol/g, 1.20 g) was used to synthesize the target peptide. At that time, CEM's Liberty Blue was used as a solid-phase synthesizer for synthesis according to the manufacturer's instruction.

For the introduction of each residue, 0.21 M Fmoc-AA (in DMF)/0.5 M HATU (in DMF)/1 M DIEA (in DMF) (4.2 equivalents/4 equivalents/8 equivalents) were used per equivalent of resin, and the reaction was carried out once for 30 min at 25°C. Meanwhile, the reaction was carried out twice for residues 4, 6, 10, 12, and 13 at 25°C for 60 min. In addition, the Fmoc was removed by a reaction twice with a 20% piperidine-containing DMF solution at 25°C for 5 min. A chloroacetyl group was introduced as follows: the Fmoc group of the α-amino group was removed from the solid-phase resin, on which the Fmoc-protected peptide obtained in the previous step was immobilized, by the procedure described above; and 0.1 M ClAcOH (in DMF)/0.1 M HATU (in DMF)/0.2 M DIEA (in DMF) (5 equivalents/5 equivalents/10 equivalents) were added to the solid-phase resin and the mixture was shaken for 30 min at 25°C.

For the deprotection of side chains and the cleavage from the solid-phase resin, the resin obtained after the chloroacetyl group introduction step was first washed five times with DMF and three times with methylene chloride, then washed with diethyl ether, and then dried under reduced pressure. The reaction agent cocktail (20 mL; a mixture of TFA/H₂O/TIS/DODT at a volume ratio of 92.5:2.5:2.5:2.5) was then added to each of two reaction vessels containing the solid-phase resin, and each vessel was shaken at room temperature for 40 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diethyl ether/hexane (1/1) mixed solvent cooled to 0°C, a cloudy precipitate occurred.

The mixture was centrifuged (at 9000 rpm at 0°C for 2 min), and the solution was then decanted. The resulting solid was washed again with a small amount of diethyl ether cooled to 0°C and then dried under reduced pressure. The solid (peptide) obtained was used in the next cyclization reaction. The cyclization reaction of the peptide was performed by dissolving the peptide in water/acetonitrile (1/1) to a final concentration of 5 mM based on the number of moles of the solid-phase resin; adding 10 equivalents of triethylamine; stirring the mixture at room temperature for 120 min; and then adding acetic acid for quenching the reaction. The resulting reaction solution was concentrated under reduced pressure by using Genevac EZ-II Elite.

The resulting crude product was purified using the following conditions (column: Waters XBridge (registered trademark) C18 5 µm 50 × 150 mm; mobile phase: A = 0.1% TFA (in water), B = 0.1% TFA (in MeCN); temperature: 40°C; gradient (%B): 33-33% over 3 min, 33-38% over 8 min, 38-60% over 1 min; flow rate: 20-20 mL/min over 1 min, 20-120 mL/min over 2 min, then 120 ml/min).

The purity of the target product was calculated from the area ratio of LC/MS (UV wavelength: 225 nm) chromatogram under the analytical conditions. The purity of the target product was 97.8%.

Analytical conditions: retention time = 5.04 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 1051.58 (M+2H)²⁺

### Example 1-3: Synthesis of Myostatin 99 v196 (SEQ ID NO: 67)

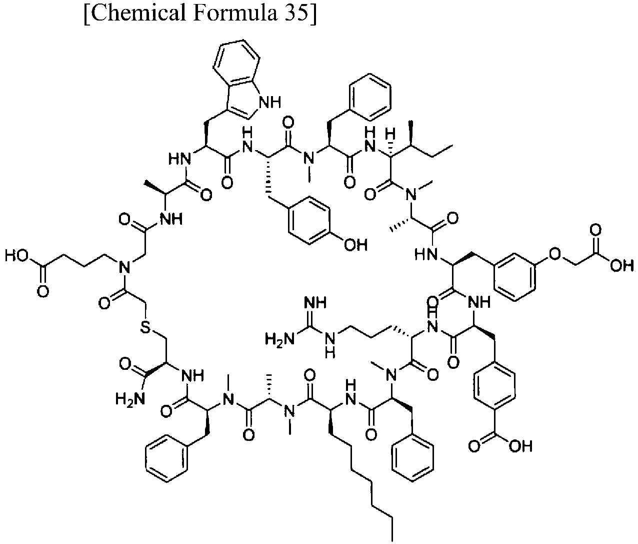

Sieber amide resin (Watanabe Chemical Industries, LTD.; 0.57 mmol/g, 0.22 g) was used to synthesize the target peptide. At that time, CEM's Liberty Blue was used as a solid-phase synthesizer for synthesis according to the manufacturer's instruction.

For the introduction of each residue, 0.21 M Fmoc-AA (in DMF)/1 M DIC (in DMF)/0.5 M Oxyma pure (in DMF) (4.2 equivalents/8 equivalents/4 equivalents) were used per equivalent of resin, and the reaction was carried out once for 3 min at 90°C. Meanwhile, the reaction was carried out twice for residues 4 and 12 at 90°C for 10 min. The reaction was carried out once for residue 5 at 90°C for 10 min. The reaction was carried out twice for residues 6 and 13 at 75°C for 30 min. The reaction was carried out twice for residue 10 at 50°C for 15 min. The reaction was carried out once for residue 15 at 50°C for 15 min. In addition, the Fmoc was removed by a reaction once with a 20% piperidine-containing DMF solution at 75°C for 3 min. Meanwhile, the reaction was carried out twice for residues 4, 6, 10, 12, and 13 at 25°C for 5 min. A chloroacetyl group was introduced as follows: the Fmoc group of the α-amino group was removed from the solid-phase resin, on which the Fmoc-protected peptide obtained in the previous step was immobilized, by the procedure described above; and 0.21 M ClAcOH (in DMF)/0.21 M HATU (in DMF)/0.42 M DIEA (in DMF) (5 equivalents/5 equivalents/10 equivalents) were added to the solid-phase resin and the mixture was shaken for 30 min at 25°C.

For the deprotection of side chains and the cleavage from the solid-phase resin, the resin obtained after the chloroacetyl group introduction step was first washed five times with DMF and three times with methylene chloride, then washed with diethyl ether, and then dried under reduced pressure. The reaction agent cocktail (7 mL; a mixture of TFA/H₂O/TIS/DODT at a volume ratio of 92.5:2.5:2.5:2.5) was then added to a reaction vessel containing the solid-phase resin, and the vessel was shaken at room temperature for 35 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diethyl ether/hexane (1/1) mixed solvent cooled to 0°C, a cloudy precipitate occurred.

The mixture was centrifuged (at 9000 rpm at 0°C for 2 min), and the solution was then decanted. The resulting solid was washed again with a small amount of diethyl ether cooled to 0°C and then dried under reduced pressure. The solid (peptide) obtained was used in the next cyclization reaction. The cyclization reaction of the peptide was performed by dissolving the peptide in water/acetonitrile (1/1) to a final concentration of 5 mM based on the number of moles of the solid-phase resin; adding 10 equivalents of triethylamine; stirring the mixture at room temperature for 21 hours; and then adding acetic acid for quenching the reaction. The resulting reaction solution was concentrated under reduced pressure by using Genevac EZ-II Elite.

The resulting crude product was purified using the following conditions (column: Waters XBridge (registered trademark) C18 5 µm 50 × 150 mm; mobile phase: A = 0.1% TFA (in water), B = 0.1% TFA (in MeCN); temperature: 40°C; gradient (%B): 17-17% over 2 min, 17-42% over 1 min, 42-47% over 8 min, 47-60% over 1 min; flow rate: 20-20 mL/min over 1 min, 20-120 mL/min over 1 min, then 120 ml/min).

The purity of the target product was calculated from the area ratio of LC/MS (UV wavelength: 225 nm) chromatogram under the analytical conditions. The purity of the target product was 95.0%.

Analytical conditions: retention time = 6.12 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 1108.16 (M+2H)²⁺

### Example 1-4: Synthesis of Myostatin 99 v309 (SEQ ID NO: 94)

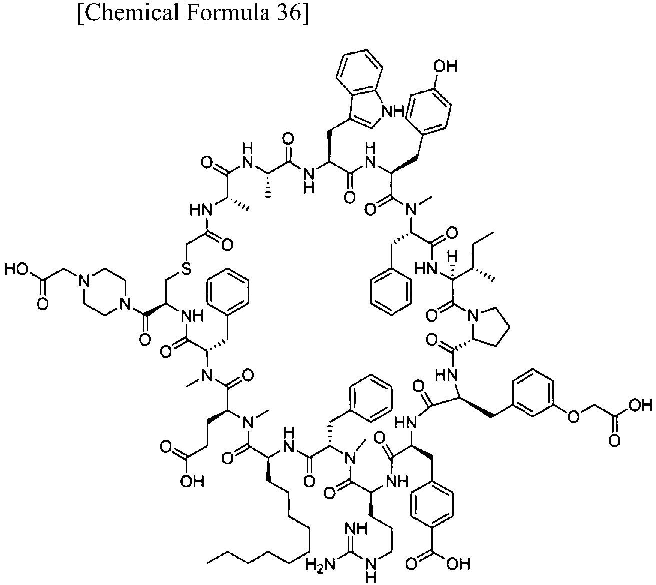

Cl-Trt(2-Cl) resin (Watanabe Chemical Industries, LTD.; 0.79 mmol/g, 95 mg) swollen with DCM was suspended in a DCM/DMF (9/1) solution containing 2 equivalents of Fmoc-pipzAc-OH, and 4 equivalents of DIEA was added and the mixture was shaken at room temperature for 90 min. Methanol in an excess amount was added and the mixture was stirred for 30 min. The solid-phase resin was washed with DMF, DCM, and diethyl ether, and then dried under reduced pressure. A portion (0.79 mmol/g, 0.095 g) of the obtained solid-phase resin was used to synthesize the target peptide. At that time, Biotage's Syro I was used as a solid-phase synthesizer for synthesis according to the manufacturer's instruction.

For the introduction of each residue, 0.21 M Fmoc-AA (in DMF)/0.5 M HATU (in DMF)/1.05 M DIEA (in DMF) (4.2 equivalents/4 equivalents/8.4 equivalents) were used per equivalent of resin, and the reaction was carried out twice for 60 min at 25°C. In addition, the Fmoc was removed by a reaction with a 20% piperidine-containing DMF solution at 25°C for 5 min, followed by the reaction for 10 min. A chloroacetyl group was introduced as follows: the Fmoc group of the α-amino group was removed from the solid-phase resin, on which the Fmoc-protected peptide obtained in the previous step was immobilized, by the procedure described above; and 10 equivalents of ClAcOSu was added to the solid-phase resin and the mixture was shaken for 60 min at 25°C.

For the deprotection of side chains and the cleavage from the solid-phase resin, the resin obtained after the chloroacetyl group introduction step was first washed five times with DMF and three times with methylene chloride, then washed with diethyl ether, and then dried under reduced pressure. The reaction agent cocktail (3 mL; a mixture of TFA/H₂O/TIS/DODT at a volume ratio of 92.5:2.5:2.5:2.5) was then added to a reaction vessel containing the solid-phase resin, and the vessel was shaken at room temperature for 45 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diisopropylether/hexane (1/1) mixed solvent cooled to 0°C, a cloudy precipitate occurred.

The mixture was centrifuged (at 9000 rpm at 0°C for 2 min), and the solution was then decanted. The resulting solid was washed again with a small amount of diethyl ether cooled to 0°C and then dried under reduced pressure. The solid (peptide) obtained was used in the next cyclization reaction. The cyclization reaction of the peptide was performed by dissolving the peptide in DMSO/water/acetonitrile (1/1/1) to a final concentration of 2 mM based on the number of moles of the solid-phase resin; adding 10 equivalents of triethylamine; stirring the mixture at room temperature for 30 min; and then adding acetic acid for quenching the reaction. The resulting reaction solution was concentrated under reduced pressure by using Genevac HT-12.

The resulting crude product was purified using the following conditions (column: Waters XBridge (registered trademark) C18 5 µm 50 × 150 mm; mobile phase: A = 0.1% TFA (in water), B = 0.1% TFA (in MeCN); temperature: 40°C; gradient (%B): 13-13% over 2 min, 13-38% over 1 min, 38-43% over 8 min, 43-60% over 1 min; flow rate: 20-20 mL/min over 1 min, 20-120 mL/min over 1 min, then 120 ml/min).

The purity of the target product was calculated from the area ratio of LC/MS (UV wavelength: 225 nm) chromatogram under the analytical conditions. The purity of the target product was 98.9%.

Analytical conditions: retention time = 5.22 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 1191.89 (M+2H)²⁺

### Example 1-5: Synthesis of Myostatin_99_v423 (SEQ ID NO: 290)

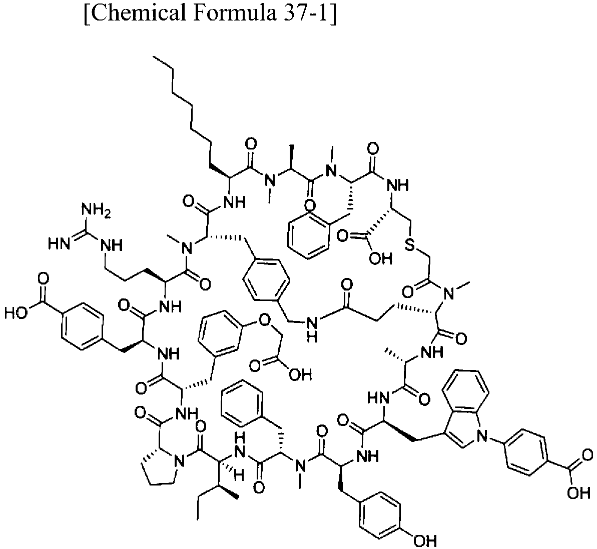

Cl-TCP(Cl) ProTide Resin (CEM; 0.36 mmol/g, 0.69 g) swollen with DCM was permeated with a DCM/DMF (10/1) containing 4 equivalents of Fmoc-dc(Trt)-OH and 8 equivalents of DIEA for 120 min. An excess amount of methanol was added and the mixture was shaken for 10 min. After washing with DMF, DCM, and diethyl ether, the mixture was dried under reduced pressure. The obtained solid-phase resin was used to synthesize the target peptide. At that time, CEM's Liberty Blue HT was used as a solid-phase synthesizer for synthesis according to the manufacturer's instruction.

For the introduction of each residue, 0.21 M Fmoc-AA (in DMF)/1 M DIC (in DMF)/0.5 M Oxyma pure (in DMF) (4.2 equivalents/8 equivalents/4 equivalents) were used per equivalent of resin, and the reaction was carried out once for 30 min at 40°C. Meanwhile, the reaction was carried out twice for residues 4, 6, 7, 10, 12, and 13 at 40°C for 30 min. Fmoc-MeE(allyl)-OH was used for the reaction of residue 1 and Fmoc-MeF4am(alloc)-OH for the reaction of residue 11. In addition, the Fmoc was removed by a reaction twice with a 10% pyrrolidine-containing DMF solution at 25°C for 5 min. Meanwhile, the reaction was carried out twice for residue 2 at 25°C for 1 min. Here, 1 equivalent of the resulting solid-phase resin was reacted with 5 equivalents of Fmoc-OSu for 60 min, followed by washing with DMF. One equivalent of the solid-phase resin obtained was suspended in acetic acid/DCM (8 mL; 2/98), 0.3 equivalents of Pd(PPh₃)₄ and 20 equivalents of phenylsilane were added, permeated for 60 min, washed with DCM, and washed with DMF. The resulting solid-phase resin was suspended in DMF and shaken with 1.2 equivalents of PyAOP and 5 equivalents of DIEA at 25°C for 60 min. After washing with DMF, the solid-phase resin obtained was reacted with a DMF solution containing 10% pyrrolidine for 25 min at 25°C and washed with DMF.

The solid-phase resin having the peptide, from which the Fmoc group had been removed, was admixed with 0.16 M ClAcOH (in DMF)/0.16 M HATU (in DMF)/0.32 M DIEA (in DMF) (5 equivalents/5 equivalents/10 equivalents), and the mixture was shaken for 60 min at 25°C to introduce a chloroacetyl group.

For the deprotection of side chains and the cleavage from the solid-phase resin, the resin obtained after the chloroacetyl group introduction step was first washed five times with DMF and three times with methylene chloride, then washed with diethyl ether, and then dried under reduced pressure. The reaction agent cocktail (14 mL; a mixture of TFA/H₂O/TIS/DODT at a volume ratio of 92.5:2.5:2.5:2.5) was then added to a reaction vessel containing the solid-phase resin, and the vessel was shaken at room temperature for 40 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diisopropylether/hexane (1/1) mixed solvent cooled to 0°C, a cloudy precipitate occurred.

The mixture was centrifuged (at 9000 rpm at 0°C for 2 min), and the solution was then decanted. The resulting solid was washed again with a small amount of diethyl ether cooled to 0°C and then dried under reduced pressure. The solid (peptide) obtained was used in the next cyclization reaction. The cyclization reaction of the peptide was performed by dissolving the peptide in water/acetonitrile (1/1) to a final concentration of 1.4 mM based on the number of moles of the solid-phase resin; adding 10 equivalents of triethylamine; stirring the mixture at room temperature for 60 min; and then adding acetic acid for quenching the reaction. The resulting reaction solution was concentrated under reduced pressure by using Genevac HT-12.

The resulting crude product was purified using the following conditions (column: Waters XSelect (registered trademark) C18 5 µm 50 × 150 mm; mobile phase: A = 0.1% TFA (in water), B = 0.1% TFA (in MeCN); temperature: 40°C; gradient (%B): 38-38% over 3 min, 38-43% over 8 min, 43-60% over 1 min; flow rate: 20-20 mL/min over 1 min, 20-120 mL/min over 2 min, then 120 ml/min).

The purity of the target product was calculated from the area ratio of LC/MS (UV wavelength: 225 nm) chromatogram under the analytical conditions. The purity of the target product was 95.0%.

Analytical conditions: retention time = 5.87 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 1180.10 (M+2H)²⁺

### Example 1-6: Synthesis of Myostatin_99_v450 (SEQ ID NO: 302)

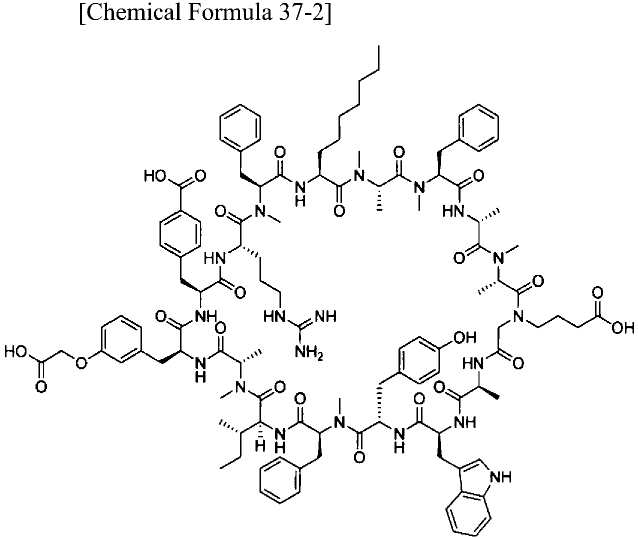

H-A-Trt(2-Cl) resin (Watanabe Chemical Industries, LTD.; 0.83 mmol/g, 0.15 g) was used to synthesize the target peptide. At that time, CEM's Liberty Blue was used as a solid-phase synthesizer for synthesis according to the manufacturer's instruction. For the introduction of each residue, 0.21 M Fmoc-AA (in DMF)/1 M DIC (in DMF)/0.5 M Oxyma pure (in DMF) (4.2 equivalents/8 equivalents/4 equivalents) were used per equivalent of resin.

The Fmoc group was deprotected using a DMF solution containing 10% pyrrolidine. Further, the peptide was elongated according to the conditions listed in Table 1 below.

**[Table 1]**

| Solid-phase synthesis introduction order | Residue number | Fmoc amino acid | Coupling reaction conditions | Fmoc deprotection conditions |
|---|---|---|---|---|
| 1 | 1 | Fmoc-CrpG(tBu)-OH | 50°C, 15 min, 1 time | 25°C, 1 min, 2 times |
| 2 | 16 | Fmoc-MeA-OH | 50°C, 15 min, 2 times | 25°C, 1 min, 2 times |
| 3 | 15 | Fmoc-da-OH | 50°C, 15 min, 2 times | 25°C, 1 min, 2 times |
| 4 | 14 | Fmoc-MeF-OH | 50°C, 15 min, 1 time | 25°C, 1 min, 2 times |
| 5 | 13 | Fmoc-MeA-OH | 50°C, 15 min, 2 times | 25°C, 1 min, 2 times |
| 6 | 12 | Fmoc-Ano-OH | 50°C, 15 min, 2 times | 25°C, 1 min, 2 times |
| 7 | 11 | Fmoc-MeF-OH | 50°C, 15 min, 1 time | 25°C, 1 min, 2 times |
| 8 | 10 | Fmoc-R(Pbf)-OH | 50°C, 15 min, 2 times | 25°C, 1 min, 2 times |
| 9 | 9 | Fmoc-F4COO(tBu)-OH | 50°C, 15 min, 1 time | 25°C, 1 min, 2 times |
| 10 | 8 | Fmoc-F3aao(tBu)-OH | 50°C, 15 min, 1 time | 25°C, 1 min, 2 times |
| 11 | 7 | Fmoc-MeA-OH | 50°C, 15 min, 1 time | 25°C, 1 min, 2 times |
| 12 | 6 | Fmoc-I-OH | 50°C, 15 min, 2 times | 25°C, 1 min, 2 times |
| 13 | 5 | Fmoc-MeF-OH | 50°C, 15 min, 1 time | 25°C, 1 min, 2 times |
| 14 | 4 | Fmoc-Y(tBu)-OH | 50°C, 15 min, 2 times | 25°C, 1 min, 2 times |
| 15 | 3 | Fmoc-W(Boc)-OH | 50°C, 15 min, 1 time | 25°C, 1 min, 2 times |

For the cleavage from the solid-phase resin, the resin obtained was first washed five times with DMF and three times with methylene chloride, then washed with diethyl ether, and then dried under reduced pressure. The reaction agent cocktail (4 mL; a mixture of HFIP/DCM at a volume ratio of 20/80) was then added to a reaction vessel containing the solid-phase resin, and the vessel was shaken at room temperature for 60 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diisopropylether/hexane (1/1) mixed solvent cooled to 0°C, a cloudy precipitate occurred.

The mixture was centrifuged (at 9000 rpm at 0°C for 2 min), and the solution was then decanted. The resulting solid was washed again with a small amount of diethyl ether cooled to 0°C and then dried under reduced pressure. The solid (peptide) obtained was used in the next cyclization reaction. The cyclization reaction of the peptide was performed by dissolving the peptide in DMF to a final concentration of 5 mM based on the number of moles of the solid-phase resin; adding 1.2 equivalents of HATU and 3 equivalents of triethylamine; stirring the mixture at room temperature for 30 min; and then adding acetic acid for quenching the reaction. The resulting reaction solution was concentrated under reduced pressure by using Genevac EZ-II Elite.

The resulting residue was washed with diisopropyl ether, then with diethyl ether and dried under reduced pressure. The resulting residue was dissolved in a reaction agent cocktail (4 mL; a mixture of TFA/TIS/H₂O at a volume ratio of 95/2.5/2.5) and shaken for 15 min. The resulting reaction solution was added to diethyl ether, and a precipitate occurred. The mixture was centrifuged (at 9000 rpm at 0°C for 2 min), and the solution was then decanted. The resulting solid was washed again with a small amount of diethyl ether cooled to 0°C and then dried under reduced pressure.

The resulting crude product was purified using the following conditions (column: Waters XBridge (registered trademark) C18 5 µm 50 × 150 mm; mobile phase: A = 0.1% TFA (in water), B = 0.1% TFA (in MeCN); temperature: 40°C; gradient (%B): 18-18% over 2 min, 18-43% over 1 min, 43-48% over 8 min, 48-60% over 1 min; flow rate: 20-20 mL/min over 1 min, 20-120 mL/min over 1 min, then 120 ml/min).

The purity of the target product was calculated from the area ratio of LC/MS (UV wavelength: 225 nm) chromatogram under the analytical conditions. The purity of the target product was 92.0%.

Analytical conditions: retention time = 5.88 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 1106.21 (M+2H)²⁺

### Example 1-7: Synthesis of Myostatin_99_v456 (SEQ ID NO: 308)

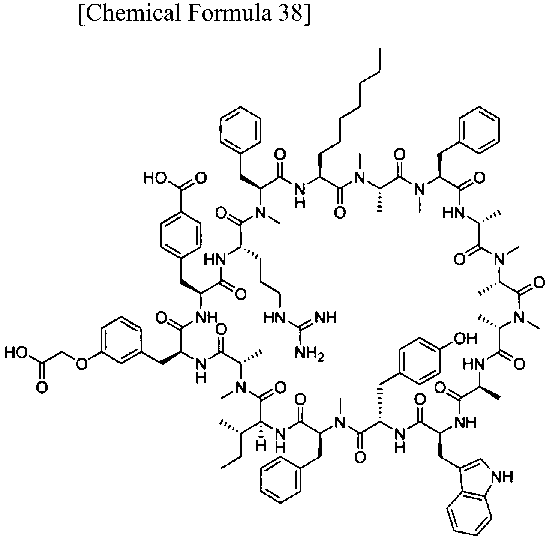

Cl-TCP(Cl) ProTide Resin (CEM; 1.08 mmol/g, 0.56 g) swollen with DCM was permeated with a DCM/DMF (5/1) solution containing 0.5 equivalents of Fmoc-da-OH and 1.5 equivalents of DIEA for 60 min. An excess amount of methanol was added and the mixture was shaken for 10 min. After washing sequentially with DMF, DCM, and diethyl ether, the mixture was dried under reduced pressure. A part of the obtained solid-phase resin was used to synthesize the target peptide. At that time, CEM's Liberty Blue was used as a solid-phase synthesizer for synthesis according to the manufacturer's instruction. For the introduction of each residue, 0.21 M Fmoc-AA (in DMF)/1 M DIC (in DMF)/0.5 M Oxyma pure (in DMF) (4.2 equivalents/8 equivalents/4 equivalents) were used per equivalent of resin.

The Fmoc group was deprotected using a DMF solution containing 10% pyrrolidine. The peptide was elongated according to the conditions listed in Table 2 below.

**[Table 2]**

| Solid-phase synthesis introduction order | Residue number | Fmoc amino acid | Coupling reaction conditions | Fmoc deprotection conditions |
|---|---|---|---|---|
| 1 | 15 | Fmoc-MeF-OH | 50°C, 15 min, 1 time | 25°C, 1 min, 2 times |
| 2 | 14 | Fmoc-MeA-OH | 50°C, 15 min, 2 times | 25°C, 1 min, 2 times |
| 3 | 13 | Fmoc-Ano-OH | 50°C, 15 min, 2 times | 25°C, 1 min, 2 times |
| 4 | 12 | Fmoc-MeF-OH | 50°C, 15 min, 1 time | 25°C, 1 min, 2 times |
| 5 | 11 | Fmoc-R(Pbf)-OH | 50°C, 15 min, 2 times | 25°C, 1 min, 2 times |
| 6 | 10 | Fmoc-F4COO(tBu)-OH | 50°C, 15 min, 1 time | 25°C, 1 min, 2 times |
| 7 | 9 | Fmoc-F3aao(tBu)-OH | 50°C, 15 min, 1 time | 25°C, 1 min, 2 times |
| 8 | 8 | Fmoc-MeA-OH | 50°C, 15 min, 1 time | 25°C, 1 min, 2 times |
| 9 | 7 | Fmoc-I-OH | 50°C, 15 min, 2 times | 25°C, 1 min, 2 times |
| 10 | 6 | Fmoc-MeF-OH | 50°C, 15 min, 1 time | 25°C, 1 min, 2 times |
| 11 | 5 | Fmoc-Y(tBu)-OH | 50°C, 15 min, 2 times | 25°C, 1 min, 2 times |
| 12 | 4 | Fmoc-W(Boc)-OH | 50°C, 15 min, 1 time | 25°C, 1 min, 2 times |
| 13 | 3 | Fmoc-A-OH | 50°C, 15 min, 1 time | 25°C, 1 min, 2 times |
| 14 | 2 | Fmoc-MeA-OH | 50°C, 15 min, 1 time | 25°C, 1 min, 2 times |
| 15 | 1 | Fmoc-MeA-OH | 50°C, 15 min, 2 times | 25°C, 1 min, 2 times |
| 16 | 16 | Fmoc-MeA-OH | 50°C, 15 min, 2 times | 25°C, 1 min, 2 times |

For the cleavage from the solid-phase resin, the resin obtained was first washed five times with DMF and three times with methylene chloride, then washed with diethyl ether, and then dried under reduced pressure. The reaction agent cocktail (6 mL; a mixture of HFIP/DCM at a volume ratio of 20/80) was then added to a reaction vessel containing the solid-phase resin, and the vessel was shaken at room temperature for 60 min. The reaction solution was collected by filtration through a frit. The solid-phase resin remaining in the reaction vessel was shaken again with the cocktail for cleavage, and the solution component was collected from the frit and mixed with the above filtrate. When this filtrate was added to an excess amount of diisopropyl ether mixed solvent cooled to 0°C, a cloudy precipitate occurred. The mixture was centrifuged (at 9000 rpm at 0°C for 2 min), and the solution was then decanted. The resulting solid was washed again with a small amount of diethyl ether cooled to 0°C and then dried under reduced pressure. The solid (peptide) obtained was used in the next cyclization reaction. The cyclization reaction of the peptide was performed by dissolving the peptide in DMF to a final concentration of 2.5 mM based on the number of moles of the solid-phase resin; adding 1 equivalent of HATU and 5 equivalents of triethylamine; stirring the mixture at room temperature for 30 min; and then adding acetic acid for quenching the reaction. The resulting reaction solution was concentrated under reduced pressure by using Genevac EZ-II Elite.

The resulting residue was washed with diethyl ether and dried under reduced pressure. The resulting residue was dissolved in a reaction agent cocktail (5 mL; a mixture of TFA/TIS/H₂O at a volume ratio of 95/2.5/2.5) and shaken for 20 min. The resulting reaction solution was added to diisopropyl ether/hexane (1/1), and a precipitate occurred. The mixture was centrifuged (at 9000 rpm at 0°C for 2 min), and the solution was then decanted. The resulting solid was washed again with a small amount of diethyl ether cooled to 0°C and then dried under reduced pressure.

The crude product obtained was purified using the following conditions (column: Waters XBridge (registered trademark) C18 5 µm 50 × 250 mm; mobile phase: A = 0.1% TFA (in water), B = 0.1% TFA (in MeCN); temperature: 50°C; gradient (%B): 22.5-20% over 0.1 min, 20-20% over 4.9 min, 20-22.5% over 2 min, 22.5-48% over 3 min, 48-53% over 15 min, 53-60% over 3 min; flow rate: 118-18 mL/min over 0.1 min, 18-118 mL/min over 4.9 min, then 118 mL/min).

The purity of the target product was calculated from the area ratio of LC/MS (UV wavelength: 225 nm) chromatogram under the analytical conditions. The purity of the target product was 94.4%.

Analytical conditions: retention time = 7.08 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 1077.06 (M+2H)²⁺

### Example 1-8: Synthesis of Myostatin 99 v455 (SEQ ID NO: 307)

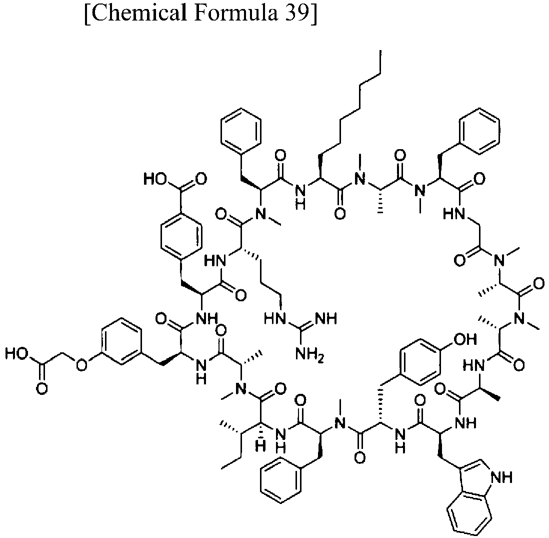

H-G-Trt(2-Cl) resin (Watanabe Chemical Industries, LTD.; 0.86 mmol/g, 0.15 g) was used to synthesize the target peptide. At that time, CEM's Liberty Blue was used as a solid-phase synthesizer for synthesis according to the manufacturer's instruction. For the introduction of each residue, 0.21 M Fmoc-AA (in DMF)/0.5 M HATU (in DMF)/1 M DIEA (in DMF) (4.2 equivalents/4 equivalents/8 equivalents) were used per equivalent of resin. The Fmoc group was deprotected using a DMF solution containing 10% pyrrolidine. The peptide was elongated according to the conditions listed in Table 3 below.

**[Table 3]**

| Solid-phase synthesis introduction order | Residue number | Fmoc amino acid | Coupling reaction conditions | Fmoc deprotection conditions |
|---|---|---|---|---|
| 1 | 14 | Fmoc-MeF-OH | 25°C, 30 min, 1 time | 25°C, 1 min, 2 times |
| 2 | 13 | Fmoc-MeA-OH | 25°C, 30 min, 2 times | 25°C, 1 min, 2 times |
| 3 | 12 | Fmoc-Ano-OH | 25°C, 30 min, 2 times | 25°C, 1 min, 2 times |
| 4 | 11 | Fmoc-MeF-OH | 25°C, 30 min, 1 time | 25°C, 1 min, 2 times |
| 5 | 10 | Fmoc-R(Pbf)-OH | 25°C, 30 min, 2 times | 25°C, 1 min, 2 times |
| 6 | 9 | Fmoc-F4COO(tBu)-OH | 25°C, 30 min, 1 time | 25°C, 1 min, 2 times |
| 7 | 8 | Fmoc-F3aao(tBu)-OH | 25°C, 30 min, 1 time | 25°C, 1 min, 2 times |
| 8 | 7 | Fmoc-MeA-OH | 25°C, 30 min, 1 time | 25°C, 1 min, 2 times |
| 9 | 6 | Fmoc-I-OH | 25°C, 30 min, 2 times | 25°C, 1 min, 2 times |
| 10 | 5 | Fmoc-MeF-OH | 25°C, 30 min, 1 time | 25°C, 1 min, 2 times |
| 11 | 4 | Fmoc-Y(tBu)-OH | 25°C, 30 min, 2 times | 25°C, 1 min, 2 times |
| 12 | 3 | Fmoc-W(Boc)-OH | 25°C, 30 min, 1 time | 25°C, 1 min, 2 times |
| 13 | 2 | Fmoc-A-OH | 25°C, 30 min, 1 time | 25°C, 1 min, 2 times |
| 14 | 1 | Fmoc-MeA-OH | 25°C, 30 min, 1 time | 25°C, 1 min, 2 times |
| 15 | 16 | Fmoc-MeA-OH | 25°C, 30 min, 2 times | 25°C, 1 min, 2 times |

For the cleavage from the solid-phase resin, the resin obtained was first washed five times with DMF and three times with methylene chloride, then washed with diethyl ether, and then dried under reduced pressure. The reaction agent cocktail (6 mL; a mixture of HFIP/DCM at a volume ratio of 20/80) was then added to a reaction vessel containing the solid-phase resin, and the vessel was shaken at room temperature for 60 min. The reaction solution was collected by filtration through a frit. The solid-phase resin remaining in the reaction vessel was shaken again with the cocktail for cleavage, and the solution component was collected from the frit and mixed with the above filtrate. The filtrate was concentrated under reduced pressure. When diisopropyl ether was added, a precipitate occurred. The mixture was centrifuged (at 9000 rpm at 0°C for 2 min), and the solution was then decanted. The resulting solid was washed again with a small amount of diethyl ether cooled to 0°C and then dried under reduced pressure. The solid (peptide) obtained was used in the next cyclization reaction. The cyclization reaction of the peptide was performed by dissolving the peptide in DMF to a final concentration of 2.5 mM based on the number of moles of the solid-phase resin; adding 1 equivalent of HATU and 5 equivalents of triethylamine; stirring the mixture at room temperature for 30 min; and then adding acetic acid for quenching the reaction. The resulting reaction solution was concentrated under reduced pressure by using Genevac EZ-II Elite.

The resulting residue was washed with diethyl ether and dried under reduced pressure. The resulting residue was dissolved in a reaction agent cocktail (5 mL; a mixture of TFA/TIS/H₂O at a volume ratio of 95/2.5/2.5) and shaken for 20 min. The resulting reaction solution was added to diisopropyl ether/hexane (1/1), and a precipitate occurred. The mixture was centrifuged (at 9000 rpm at 0°C for 2 min), and the solution was then decanted. The resulting solid was washed again with a small amount of diethyl ether cooled to 0°C and then dried under reduced pressure.

The resulting crude product was purified using the following conditions (column: Waters XBridge (registered trademark) C18 5 µm 50 × 150 mm; mobile phase: A = 0.1% TFA (in water), B = 0.1% TFA (in MeCN); temperature: 40°C; gradient (%B): 22-22% over 2 min, 22-47% over 1 min, 47-52% over 8 min, 52-60% over 1 min; flow rate: 20-20 mL/min over 1 min, 20-120 mL/min over 1 min, then 120 ml/min).

The purity of the target product was calculated from the area ratio of LC/MS (UV wavelength: 225 nm) chromatogram under the analytical conditions. The purity of the target product was 97.5%.

Analytical conditions: retention time = 3.44 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 40-80% over 7.15 min, then 80-95% over 0.3 min, then 95-95% over 1.55 min; flow rate: 0.5 mL/min.

ESI-MS(+): observed m/z = 1070.05 (M+2H)²⁺

### Example 1-9: Synthesis of Myostatin_99_v434 (SEQ ID NO: 291)

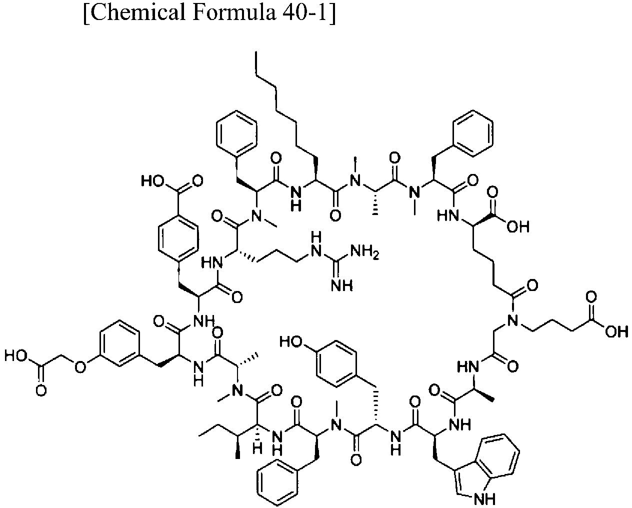

Cl-Trt(2-Cl) resin (Watanabe Chemical Industries, LTD.; 1.35 mmol/g, 0.37 g) swollen with DCM was suspended in a DCM/DMF (30 mL, 4/1) solution containing 4 equivalents of Fmoc-dhgl-OtBu, and 8 equivalents of DIEA was added and the mixture was shaken at room temperature for 180 min. Methanol in an excess amount was added and the mixture was stirred for 30 min. The solid-phase resin was washed with DMF, DCM, and diethyl ether, and then dried under reduced pressure. A portion (0.90 mmol/g, 0.27 g) of the obtained solid-phase resin was used to synthesize the target peptide. At that time, CEM's Liberty Blue was used as a solid-phase synthesizer for synthesis according to the manufacturer's instruction.

For the introduction of each residue, 0.21 M Fmoc-AA (in DMF)/0.5 M HATU (in DMF)/1 M DIEA (in DMF) (4.2 equivalents/4 equivalents/8 equivalents) were used per equivalent of resin, and the reaction was carried out once for 30 min at 25°C. Meanwhile, the reaction was carried out twice for residues 4, 6, 10, 12, and 13 at 25°C for 30 min. The Fmoc was removed by a reaction twice with a 10% pyrrolidine-containing DMF solution at 25°C for 1 min.

For the cleavage from the solid-phase resin, the resin obtained was first washed five times with DMF and three times with methylene chloride, then washed with diethyl ether, and then dried under reduced pressure. The reaction agent cocktail (10 mL; a mixture of HFIP/DCM at a volume ratio of 20/80) was then added to a reaction vessel containing the solid-phase resin, and the vessel was shaken at room temperature for 60 min. The reaction solution was collected by filtration through a frit. The solid-phase resin remaining in the reaction vessel was shaken again with the cocktail for cleavage, and the solution component was collected from the frit and mixed with the above filtrate. The filtrate was concentrated under reduced pressure. When diisopropyl ether was added, a precipitate occurred. The mixture was centrifuged (at 9000 rpm at 0°C for 2 min), and the solution was then decanted. The resulting solid was washed again with a small amount of diethyl ether cooled to 0°C and then dried under reduced pressure. The solid (peptide) obtained was used in the next cyclization reaction. The cyclization reaction of the peptide was performed by dissolving the peptide in DMF to a final concentration of 5 mM based on the number of moles of the solid-phase resin; adding 1.2 equivalents of HATU and 6 equivalents of triethylamine; stirring the mixture at room temperature for 20 min; and then adding acetic acid for quenching the reaction. The resulting reaction solution was concentrated under reduced pressure by using Genevac EZ-II Elite. The resulting residue was washed with diethyl ether and dried under reduced pressure. The resulting residue was dissolved in a reaction agent cocktail (5 mL; a mixture of TFA/H₂O at a volume ratio of 97.5/2.5) and shaken for 30 min. The resulting reaction solution was concentrated under reduced pressure and dried.

The crude product obtained was purified using the following conditions (column: Waters XBridge (registered trademark) C18 5 µm 50 × 250 mm; mobile phase: A = 0.1% TFA (in water), B = 0.1% TFA (in MeCN); temperature: 50°C; gradient (%B): 16.4-13.3% over 0.1 min, 13.3-13.3% over 4.9 min, 13.3-16.4% over 2 min, 16.4-41.9% over 3 min, 41.9-46.9% over 15 min, 46.9-60% over 3 min; flow rate: 118-18 mL/min over 0.1 min, 18-18 mL/min over 4.9 min, 18-118 mL/min over 2 min, then 118 mL/min).

The purity of the target product was calculated from the area ratio of LC/MS (UV wavelength: 225 nm) chromatogram under the analytical conditions. The purity of the target product was 97.7%.

Analytical conditions: retention time = 5.82 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 1099.58 (M+2H)²⁺

### Example 1-10: Synthesis of Myostatin 99 v302 (SEQ ID NO: 195)

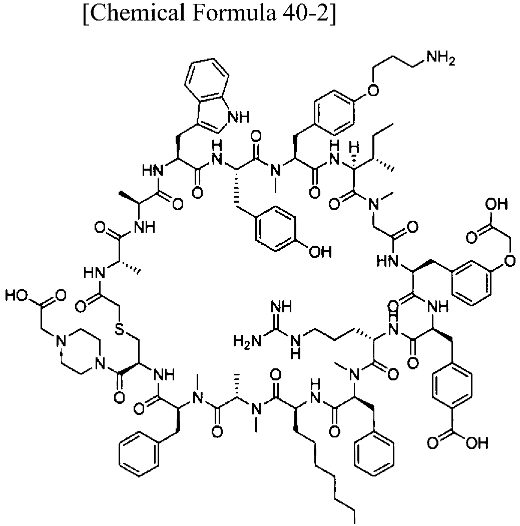

Cl-Trt(2-Cl) resin (Watanabe Chemical Industries, LTD.; 0.79 mmol/g, 4.1 g) swollen with DCM was suspended in a DCM/DMF (9/1) solution containing 2 equivalents of Fmoc-pipzAc-OH, and 4 equivalents of DIEA was added and the mixture was shaken at room temperature for 90 min. Methanol in an excess amount was added and the mixture was stirred for 30 min. The solid-phase resin was washed with DMF, DCM, and diethyl ether, and then dried under reduced pressure. A portion (0.79 mmol/g, 0.095 g) of the obtained solid-phase resin was used to synthesize the target peptide. At that time, Biotage's Syro I was used as a solid-phase synthesizer for synthesis according to the manufacturer's instruction.

For the introduction of each residue, 0.21 M Fmoc-AA (in DMF)/0.5 M HATU (in DMF)/1.1 M DIEA (in DMF) (4.2 equivalents/4 equivalents/8.4 equivalents) were used per equivalent of resin, and the reaction was carried out twice for 60 min at 25°C. In addition, the Fmoc was removed by a reaction with a 20% piperidine-containing DMF solution at 25°C for 5 min, followed by the reaction for 10 min. A chloroacetyl group was introduced as follows: the Fmoc group of the α-amino group was removed from the solid-phase resin, on which the Fmoc-protected peptide obtained in the previous step was immobilized, by the procedure described above; and 10 equivalents of ClAcOSu was added to the solid-phase resin and the mixture was shaken for 60 min at 25°C.

For the deprotection of side chains and the cleavage from the solid-phase resin, the resin obtained after the chloroacetyl group introduction step was first washed five times with DMF and three times with methylene chloride, then washed with diethyl ether, and then dried under reduced pressure. The reaction agent cocktail (3 mL; a mixture of TFA/H₂O/TIS/DODT at a volume ratio of 92.5:2.5:2.5:2.5) was then added to a reaction vessel containing the solid-phase resin, and the vessel was shaken at room temperature for 45 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diisopropylether/hexane (1/1) mixed solvent cooled to 0°C, a cloudy precipitate occurred.

The mixture was centrifuged (at 9000 rpm at 0°C for 2 min), and the solution was then decanted. The resulting solid was washed again with a small amount of diethyl ether cooled to 0°C and then dried under reduced pressure. The solid (peptide) obtained was used in the next cyclization reaction. The cyclization reaction of the peptide was performed by dissolving the peptide in DMSO/water/acetonitrile (1/1/1) to a final concentration of 2 mM based on the number of moles of the solid-phase resin; adding 10 equivalents of triethylamine; stirring the mixture at room temperature for 30 min; and then adding acetic acid for quenching the reaction. The resulting reaction solution was concentrated under reduced pressure by using Genevac HT-12. The resulting residue was dissolved in DMF (5 mL), 0.05 equivalents of Pd(PPh₃)₄, 0.1 mL of piperidine, and 20 equivalents of dimethylamine borane (CAS 74-94-2) were added, and the mixture was shaken at room temperature for 150 min.

The resulting crude product was purified using the following conditions (column: Waters XSelect (registered trademark) C18 5 µm 19 × 150 mm; mobile phase: A = 0.1% TFA (in water), B = 0.1% TFA (in MeCN); temperature: 60°C; gradient (%B): 5-30% over 3 min, 30-35% over 8 min, 35-60% over 1 min; flow rate: 17 ml/min).

The purity of the target product was calculated from the area ratio of LC/MS (UV wavelength: 225 nm) chromatogram under the analytical conditions. The purity of the target product was 99.6%.

Analytical conditions: retention time = 3.90 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 777.2 (M+3H)³⁺

### Example 1-11: Synthesis of Myostatin_99_v292 (SEQ ID NO: 189)

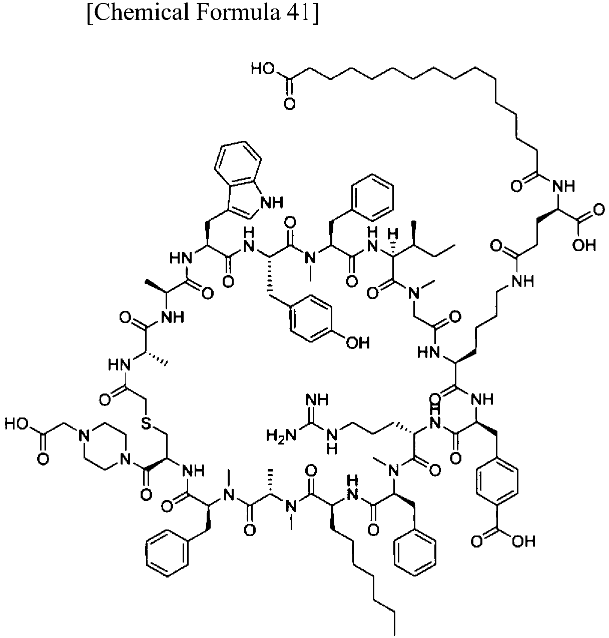

HMPB-MBHA resin (Novabiochem; 0.92 mmol/g, 0.14 g) swollen with DCM was suspended in 4 equivalents of Fmoc-pipzAc-OH and 0.4 equivalents of DCM/DMF (9/1) solution, and 4 equivalents of DIC was added and the mixture was shaken at room temperature for 60 min. The solid-phase resin was washed with 1% DIEA-containing DMF, DMF, DCM, and diethyl ether, and then dried under reduced pressure. The obtained solid-phase resin was used to synthesize the target peptide. At that time, CEM's Liberty Blue was used as a solid-phase synthesizer for synthesis according to the manufacturer's instruction.

For the introduction of each of residue 15 to residue 8, 0.21 M Fmoc-AA (in DMF)/1 M DIC (in DMF)/0.5 M Oxyma pure (in DMF) (4.2 equivalents/8 equivalents/4 equivalents) were used per equivalent of resin, and the reaction was carried out once for 3 min at 90°C. Meanwhile, the reaction was carried out twice for residues 10 and 15 at 50°C for 15 min. The reaction was carried out twice for residue 12 at 75°C for 30 min. The reaction was carried out twice for residues 13 and 14 at 90°C for 10 min. Alloc-K(Fmoc)-OH was used for the reaction of residue 8. For a reaction of elongation from the side chain of residue 8, the reaction was carried out once at 90°C for 10 min. Fmoc-de-OtBu was introduced, the Fmoc group was removed, and HO-cC14COO-tBu was then introduced. In addition, the Fmoc was removed by a reaction twice with a 10% pyrrolidine-containing DMF solution at 25°C for 1 min. The solid-phase resin obtained was suspended in 1% HFIP-containing dichloromethane, and 0.2 equivalents of Pd(PPh₃)₄ and 10 equivalents of phenylsilane were added. The mixture was shaken at room temperature for 60 min, and washed with dichloromethane and then DMF.

For the introduction of each of residue 7 to residue 1 to the resulting solid-phase resin, 0.21 M Fmoc-AA (in DMF)/1 M DIC (in DMF)/0.5 M Oxyma pure (in DMF) (4.2 equivalents/8 equivalents/4 equivalents) were used per equivalent of resin, and the reaction was carried out once for 3 min at 90°C. Meanwhile, the reaction was carried out twice for residue 4 at 90°C for 10 min. The reaction was carried out twice for residues 6 and 7 at 75°C for 30 min. In addition, the Fmoc was removed by a reaction twice with a 10% pyrrolidine-containing DMF solution at 25°C for 1 min. A chloroacetyl group was introduced as follows: the Fmoc group of the α-amino group was removed from the solid-phase resin, on which the Fmoc-protected peptide obtained in the previous step was immobilized, by the procedure described above; and a DMF solution (6 mL) containing 10 equivalents of ClAcOSu was added to the solid-phase resin and the mixture was shaken for 60 min at 25°C.

For the deprotection of side chains and the cleavage from the solid-phase resin, the resin obtained after the chloroacetyl group introduction step was first washed five times with DMF and three times with methylene chloride, then washed with diethyl ether, and then dried under reduced pressure. The reaction agent cocktail (7 mL; a mixture of TFA/H₂O/TIS/DODT at a volume ratio of 92.5:2.5:2.5:2.5) was then added to a reaction vessel containing the solid-phase resin, and the vessel was shaken at room temperature for 40 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diisopropylether/hexane (1/1) mixed solvent cooled to 0°C, a cloudy precipitate occurred.

The mixture was centrifuged (at 9000 rpm at 0°C for 2 min), and the solution was then decanted. The resulting solid was washed again with a small amount of diethyl ether cooled to 0°C and then dried under reduced pressure. The solid (peptide) obtained was used in the next cyclization reaction. The cyclization reaction of the peptide was performed by dissolving the peptide in DMSO/water/acetonitrile (1/2/2) to a final concentration of 5 mM based on the number of moles of the solid-phase resin; adding 10 equivalents of triethylamine; stirring the mixture at room temperature for 300 min; and then adding acetic acid for quenching the reaction. The resulting reaction solution was concentrated under reduced pressure by using Genevac EZ-II Elite.

The resulting crude product was purified using the following conditions (column: Waters XBridge (registered trademark) C18 5 µm 50 × 150 mm; mobile phase: A = 0.1% TFA (in water), B = 0.1% TFA (in MeCN); temperature: 40°C; gradient (%B): 18-18% over 2 min, 18-43% over 1 min, 43-48% over 8 min, 48-60% over 1 min; flow rate: 20-20 mL/min over 1 min, 20-120 mL/min over 1 min, then 120 ml/min).

The purity of the target product was calculated from the area ratio of LC/MS (UV wavelength: 225 nm) chromatogram under the analytical conditions. The purity of the target product was 97.9%.

Analytical conditions: retention time = 5.90 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 1281.0 (M+2H)²⁺

### Example 2: Analysis of Peptide

The target peptides were synthesized according to the common methods described above.

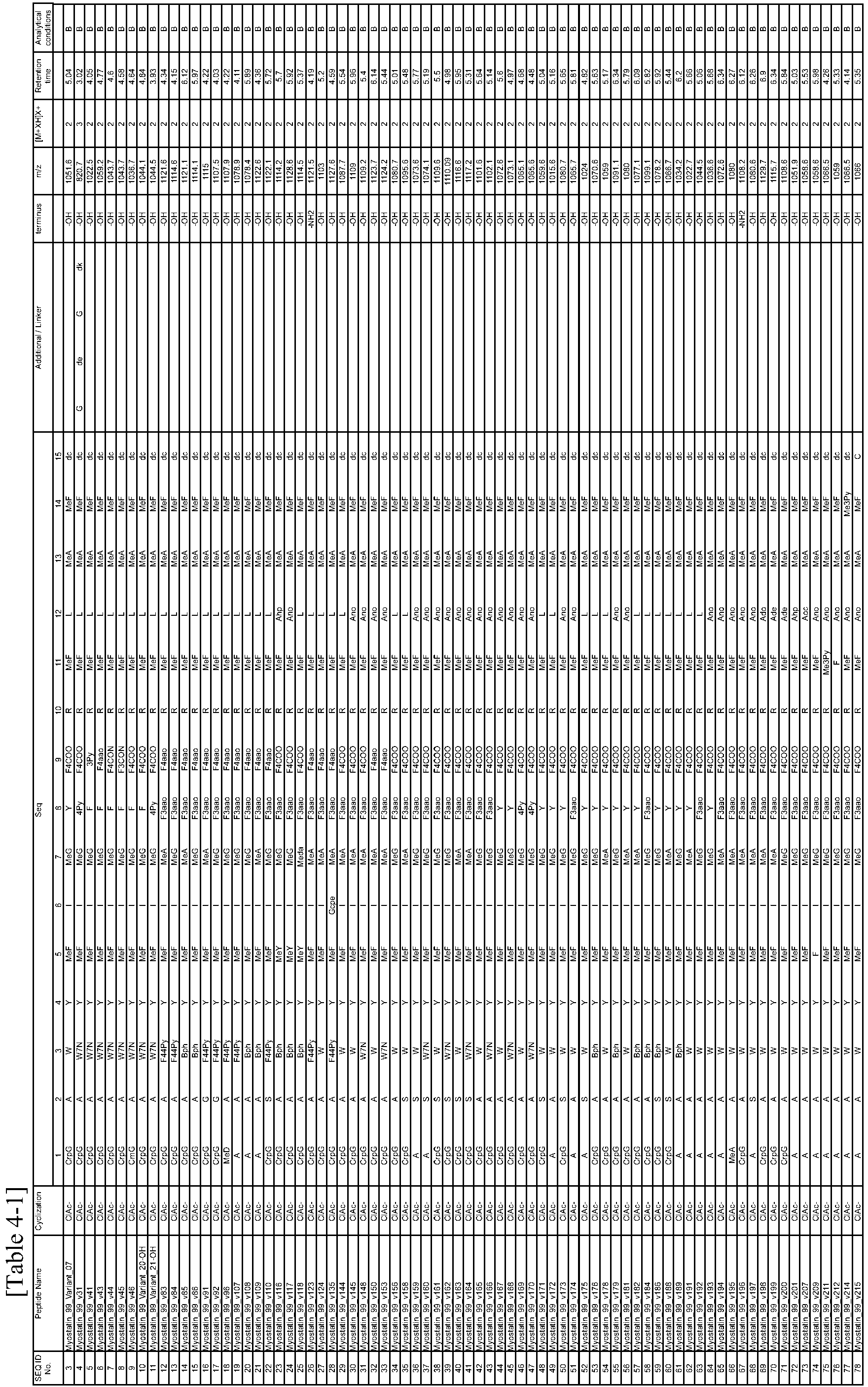

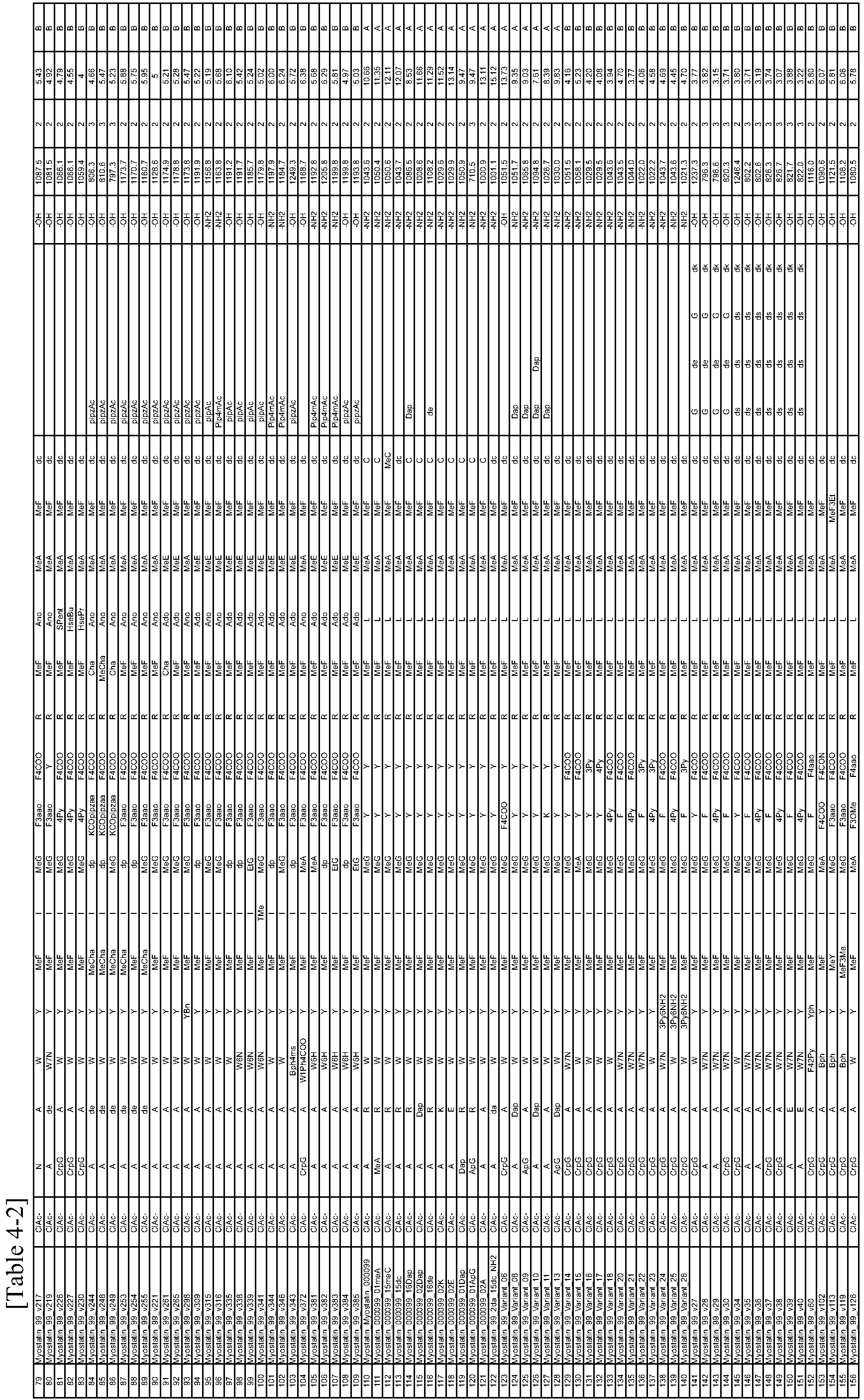

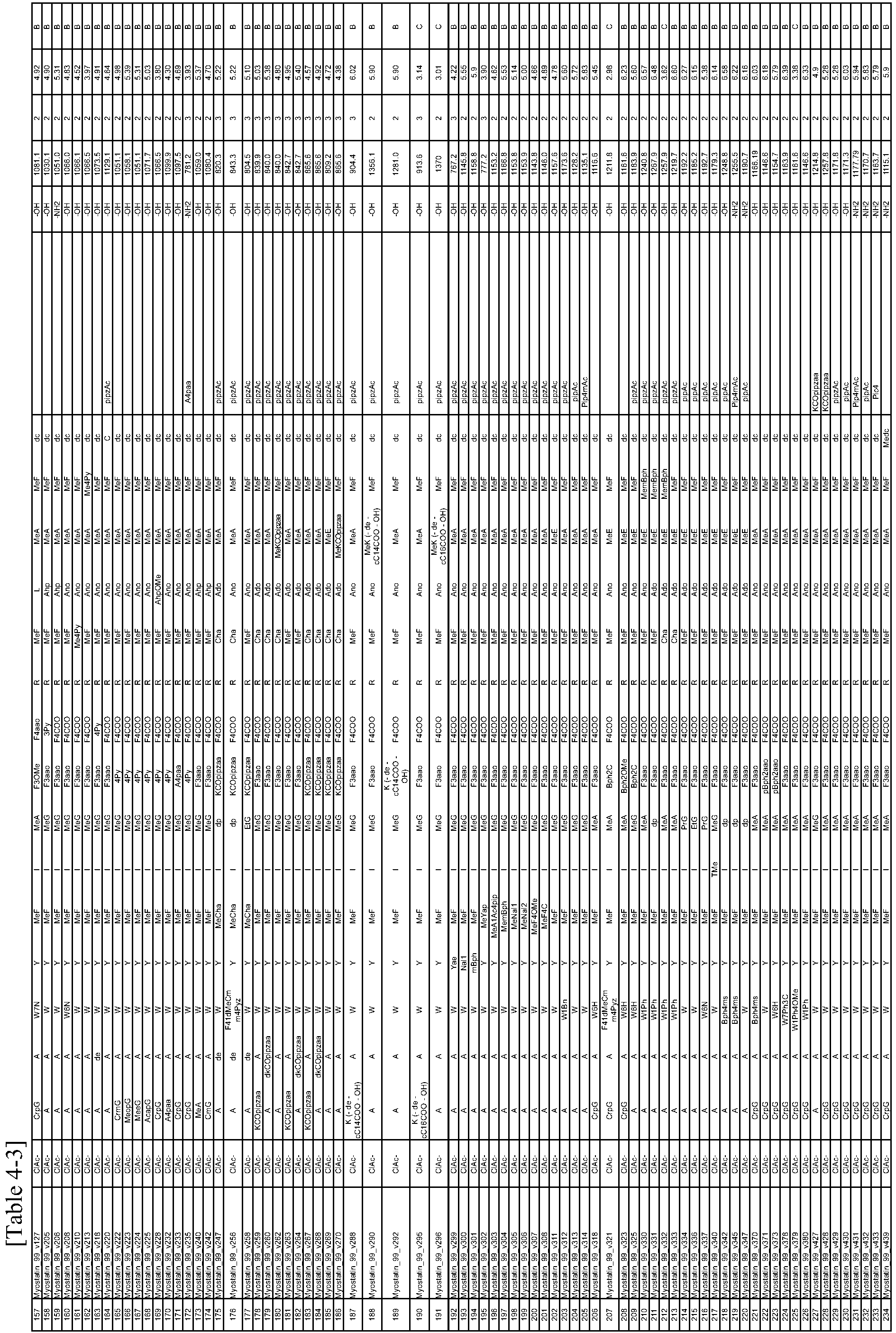

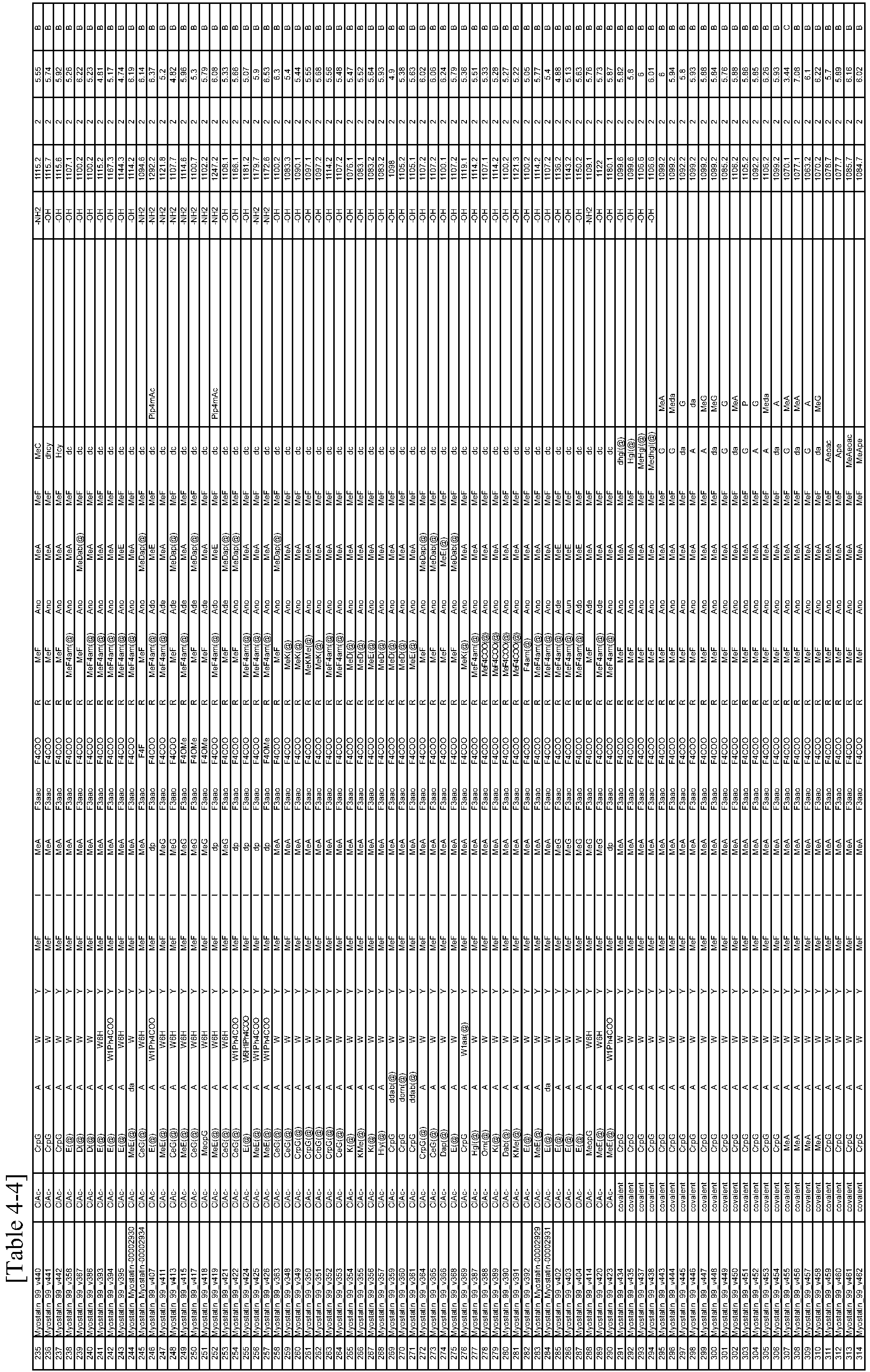

Table 4 lists the target peptides, analytical conditions, and retention times, and ESI-MS(+) observed values. The "terminus" indicates the C-terminal functional group (in the tables, "-OH" indicates COOH and "-NH2" indicates CO(NH₂)), and the blank case indicates that no functional group is present at the C-terminus. With respect to "Cyclization" in the tables, entries with the description "ClAc" represent that cyclization was achieved by linking the amino acid at position 1 into which a chloroacetyl group was introduced with Cys present at the C-terminus, whereas the description "covalent" indicates that the N-terminal amino group of the amino acid at position 1 is linked to the C-terminal carboxyl group of the amino acid at position 15 or 16. Note that in this case, (@) indicates that the side chain terminal functional group is linked to the amino group of the amino acid at position 1. In addition, the "Additional/Linker" in the tables indicates, except for SEQ ID NOs: 295-310, a linker or additional amino acid(s) that is not included in the cyclic structure. For example, in Myostatin_99_v31 (SEQ ID NO: 4), the amino acid sequence X1-X15 forms a cyclic structure, and an amino acid sequence G-de-G-dk is further added as a linker from the dc of the C-terminal X15.

For the peptides of SEQ ID NOs: 238-290, the side chains of the amino acid residues marked with (@) are linked to each other to form a crosslinking structure.

Further, for SEQ ID NOs: 187-191, the following is shown; in Myostatin_99_v288 (SEQ ID NO: 187), the terminal NH₂ of the side chain of K at position 1 is linked to the terminal carboxyl group of the side chain of de, and cC14COO is further attached from the amino group of the de, the terminus of which is OH. In Myostatin_99_v290 (SEQ ID NO: 188), the terminal NH₂ of the side chain of MeK at position 13 is linked to the terminal carboxyl group of the side chain of de, and cC14COO is further attached from the amino group of the de, the terminus of which is OH. In Myostatin_99_v292 (SEQ ID NO: 189), the terminal NH₂ of the side chain of K at position 8 is linked to the terminal carboxyl group of the side chain of de, and cC14COO is further attached from the amino group of the de, the terminus of which is OH. In Myostatin_99_v295 (SEQ ID NO: 190), the terminal NH₂ of the side chain of K at position 1 is linked to the terminal carboxyl group of the side chain of de, and cC16COO is further attached from the amino group of the de, the terminus of which is OH. In Myostatin _99_v296 (SEQ ID NO: 191), the terminal NH₂ of the side chain of MeK at position 13 is linked to the terminal carboxyl group of the side chain of de, and cC16COO is further attached from the amino group of the de, the terminus of which is OH.

In addition, the following describes SEQ ID NOs: 291-294. In Myostatin_99_v434 (SEQ ID NO: 291), a cyclic structure is formed by linking the N-terminal amino group of CrpG at position 1 to the side-chain terminal carboxyl group of dhgl at position 15, and the C-terminal carboxyl group of the dhgl at position 15 remains unsubstituted (-COOH). Similarly, in the peptides of SEQ ID NOs: 292-294, a cyclic structure is formed by linking the N-terminal amino group of CrpG at position 1 to the side-chain terminal carboxyl group of one of dhgl, Hgl, MeHgl, or Medhgl at position 15, and the C-terminal carboxyl group of the amino acid at position 15 remains unsubstituted (-COOH).

In addition, the following describes SEQ ID NOs: 295-310. In Myostatin_99_v443 (SEQ ID NO: 295), a cyclic structure is formed by linking the N-terminal amino group of CrpG at position 1 to the C-terminal carboxyl group of MeA at position 16, and no additional amino acids are included. Similarly, in the peptides of SEQ ID NOs: 296-310, a cyclic structure is formed by linking the N-terminal amino group of CrpG or MeA at position 1 to the C-terminal carboxy group of Meda, G, da, MeG, MeA, P, or A at position 16.

In addition, the following describes SEQ ID NOs: 310-314. In Myostatin_99_v459 (SEQ ID NO: 311), a cyclic structure is formed by linking the N-terminal amino group of CrpG at position 1 to the C-terminal carboxyl group of Aeoac at position 15, and no additional amino acids are included. Similarly, in the peptides of SEQ ID NOs: 311-314, a cyclic structure is formed by linking the N-terminal amino group of CrpG at position 1 to the C-terminal carboxy group of Aeoac, Ape, MeAeoac, or MeApe at position 15.

### Example 3: Evaluation of In Vitro Activity by Using Reporter Gene Assay for Myostatin Signaling in HepG2 Cell Line

In this Example, the *in vitro* myostatin inhibitory effects of the peptides synthesized in Examples 1 and 2 were evaluated using a reporter gene assay for myostatin signaling in HepG2 cell line.

### Evaluation Procedure (1)

HepG2 cell line (HB-8065, ATCC) cultured in EMEM (055-08975, WAKO Industries) containing 10% FBS (10270106, Gibco) and 50 µg/mL gentamicin (11980-14, Nacalai Tesque) was seeded in 96-well plates (167008, Thermo) at 2.0 × 10⁴ cells/well and incubated in a CO₂ incubator (37°C, 5% CO₂) for 24 hours. The pGL3(CAGA)₁₂-luc plasmid, namely a pGL3 vector (E1751, Promega) having a luciferase gene inserted downstream of a promoter sequence with 12 CAGA repeats attached, was added to the wells along with a transfection reagent (X-treamGENE9 DNA Transfection Reagent, 6365809001, Roche) at a concentration of 0.04 µg/well. The cell line was cultured in a CO₂ incubator (37°C, 5% CO₂) for 24 hours for transfection. The medium was discarded and replaced with EMEM containing 50 µL/well of 0.1% bovine serum albumin (A7030, Sigma) and 50 µg/mL gentamicin, and incubated in a CO₂ incubator (37°C, 5% CO₂) for 2 hours.

Myostatin (788-G8/C, R&D systems) or GDF-11 (1958-GD-G8/CF, R&D systems) was added so that the final concentrations were those listed in Table 5.

**[Table 5]**

| | Myostatin | GDF-11 |
|---|---|---|
| Method A | 10 ng/mL | 10 ng/mL |
| Method B | 1 ng/mL | 1 ng/mL |
| Method C | 1.5 ng/mL | 1 ng/mL |
| Method D | 1.5 ng/mL | 1.5 ng/mL |

A 4-fold dilution series of various peptides synthesized in Examples 1 and 2 was added to achieve 0.006-100 nmol/L (myostatin inhibition) and 0.12-2000 nmol/L (GDF-11 inhibition). At this time, blank wells, in which neither myostatin, GDF-11, nor each peptide was added, were also prepared and incubated in a CO₂ incubator (37°C, 5% CO₂) for 20 to 24 hours.

The medium containing the cells was removed, and a mixture containing PBS (14190144, Thermo) and a chromogenic substrate One-Glo Luciferase Assay System (E6120, Promega) at a ratio of 1:1 was added to each well at 100 µL/well. This was stirred with a plate shaker for 5 min and transferred to a 384-well plate (3570, Corning) at a volume of 20 µL/well.

The plate was centrifuged at 1,000 × g for 1 min and the luminescence signal (700 nm luminescence) was measured using a plate reader (Envision 2104, PerkinElmer). Based on the measured values, IC50 values of the peptides were calculated using software (Prism 9, GraphPad). Table 6 shows the results.

**[Table 6-1]**

| SEQ ID No. | Peptide Name | IC50 (Luciferase) Myostatin HepG2 (nM) | IC50 (Luciferase) GDF-11 HepG2 (nM) | Selectivity | Method |
|---|---|---|---|---|---|
| 3 | Myostatin_99_Variant_07 | 26.2 | 1047 | 40 | A |
| 4 | Myostatin_99_v31 | 54.7 | 1342 | 25 | A |
| 5 | Myostatin_99_v41 | 63.7 | 1631 | 26 | A |
| 6 | Myostatin_99_v43 | 33.2 | 1993 | 60 | A |
| 7 | Myostatin_99_v44 | 8.4 | 810 | 96 | A |
| 8 | Myostatin_99_v45 | 12.8 | 332 | 26 | A |
| 9 | Myostatin_99_v46 | 43.2 | 1536 | 36 | A |
| 10 | Myostatin_99_Variant_20-OH | 44.8 | 2014 | 45 | A |
| 11 | Myostatin_99_Variant_21-OH | 106.1 | 4316 | 41 | A |
| 12 | Myostatin_99_v83 | 20.9 | 3011 | 144 | A |
| 13 | Myostatin_99_v84 | 27.3 | 3046 | 112 | A |
| 14 | Myostatin_99_v85 | 13.5 | 3374 | 250 | A |
| 15 | Myostatin_99_v86 | 10.4 | 1651 | 159 | A |
| 16 | Myostatin_99_v91 | 72.3 | 7378 | 102 | A |
| 17 | Myostatin_99_v92 | 78.1 | 3883 | 50 | A |
| 18 | Myostatin_99_v96 | 88.8 | 26259 | 296 | A |
| 19 | Myostatin_99_v107 | 2.8 | 466 | 166 | A |
| 20 | Myostatin_99_v108 | 3.6 | 374 | 104 | A |
| 21 | Myostatin_99_v109 | 25.6 | 3842 | 150 | A |
| 22 | Myostatin_99_v110 | 9.5 | 1695 | 178 | A |
| 23 | Myostatin_99_v116 | 5.4 | 1963 | 364 | A |
| 24 | Myostatin_99_v117 | 1.7 | 713 | 419 | A |
| 25 | Myostatin_99_v118 | 23.1 | 4034 | 175 | A |
| 26 | Myostatin_99_v123 | 38.1 | 7394 | 194 | A |
| 27 | Myostatin_99_v124 | 17.1 | 2268 | 133 | A |
| 28 | Myostatin_99_v135 | 22.6 | 3286 | 145 | A |
| 29 | Myostatin_99_v144 | 16.3 | 2759 | 169 | A |
| 30 | Myostatin_99_v145 | 1.6 | 414 | 259 | A |
| 31 | Myostatin_99_v148 | 1.5 | 439 | 293 | A |
| 32 | Myostatin_99_v150 | 1.7 | 395 | 232 | A |
| 33 | Myostatin_99_v153 | 1.2 | 267 | 222 | A |
| 34 | Myostatin_99_v156 | 69.0 | 4811 | 70 | A |
| 35 | Myostatin_99_v158 | 62.0 | 6456 | 104 | A |
| 36 | Myostatin_99_v159 | 3.9 | 404 | 104 | A |
| 37 | Myostatin_99_v160 | 3.2 | 350 | 109 | A |
| 38 | Myostatin_99_v161 | 8.2 | 1241 | 151 | A |
| 39 | Myostatin_99_v162 | 6.1 | 90 | 15 | A |
| 40 | Myostatin_99_v163 | 3.6 | 1004 | 279 | A |
| 41 | Myostatin_99_v164 | 4.2 | 1343 | 320 | A |
| 42 | Myostatin_99_v165 | 14.0 | 1240 | 89 | A |
| 43 | Myostatin_99_v166 | 3.5 | 951 | 272 | A |
| 44 | Myostatin_99_v167 | 4.5 | 214 | 48 | A |

**[Table 6-2]**

| | | | | | |
|---|---|---|---|---|---|
| 45 | Myostatin_99_v168 | 2.1 | 119 | 57 | A |
| 46 | Myostatin_99_v169 | 1.2 | 372 | 310 | A |
| 47 | Myostatin_99_v170 | 3.3 | 215 | 65 | A |
| 48 | Myostatin_99_v171 | 44.7 | 909 | 20 | A |
| 49 | Myostatin_99_v172 | 5.1 | 173 | 34 | A |
| 50 | Myostatin_99_v173 | 5.5 | 232 | 42 | A |
| 51 | Myostatin_99_v174 | 1.7 | 226 | 133 | A |
| 52 | Myostatin_99_v175 | 16.4 | 399 | 24 | A |
| 53 | Myostatin_99_v176 | 17.3 | 377 | 22 | A |
| 54 | Myostatin_99_v178 | 12.1 | 250 | 21 | A |
| 55 | Myostatin_99_v179 | 1.5 | 163 | 108 | A |
| 56 | Myostatin_99_v181 | 4.5 | 194 | 43 | A |
| 57 | Myostatin_99_v182 | 53.0 | 1356 | 26 | A |
| 58 | Myostatin_99_v184 | 14.2 | 1834 | 129 | A |
| 59 | Myostatin_99_v186 | 43.2 | 935 | 22 | A |
| 60 | Myostatin_99_v188 | 55.4 | 911 | 16 | A |
| 61 | Myostatin_99_v189 | 6.9 | 234 | 34 | A |
| 62 | Myostatin_99_v191 | 8.1 | 283 | 35 | A |
| 63 | Myostatin_99_v192 | 12.1 | 2060 | 170 | A |
| 64 | Myostatin_99_v193 | 1.0 | 57 | 57 | A |
| 65 | Myostatin_99_v194 | 1.2 | 139 | 116 | A |
| 66 | Myostatin_99_v195 | 1.3 | 255 | 196 | A |
| 67 | Myostatin_99_v196 | 3.4 | 603 | 177 | A |
| 68 | Myostatin_99_v197 | 3.4 | 438 | 129 | A |
| 69 | Myostatin_99_v198 | 0.7 | 90 | 123 | A |
| 70 | Myostatin_99_v199 | 1.2 | 159 | 133 | A |
| 71 | Myostatin_99_v200 | 1.0 | 233 | 233 | A |
| 72 | Myostatin_99_v201 | 8.6 | 2257 | 262 | A |
| 73 | Myostatin_99_v207 | 3.5 | 270 | 77 | A |
| 74 | Myostatin_99_v209 | 5.4 | 794 | 147 | A |
| 75 | Myostatin_99_v211 | 1.1 | 232 | 211 | A |
| 76 | Myostatin_99_v212 | 3.3 | 278 | 84 | A |
| 77 | Myostatin_99_v214 | 6.6 | 1324 | 201 | A |
| 78 | Myostatin_99_v215 | 1.9 | 202 | 106 | A |
| 79 | Myostatin_99_v217 | 3.0 | 696 | 232 | A |
| 80 | Myostatin_99_v219 | 2.7 | 313 | 116 | A |
| 81 | Myostatin_99_v226 | 10.2 | 3992 | 391 | A |
| 82 | Myostatin_99_v227 | 20.8 | 683 | 33 | A |
| 83 | Myostatin_99_v230 | 57.1 | 8375 | 147 | A |
| 84 | Myostatin_99_v244 | 17.7 | 13647 | 771 | A |
| 85 | Myostatin_99_v248 | 21.5 | 3525 | 164 | A |
| 86 | Myostatin_99_v249 | 0.5 | 119 | 237 | A |
| 87 | Myostatin_99_v253 | 5.7 | 2439 | 428 | A |

**[Table 6-3]**

| | | | | | |
|---|---|---|---|---|---|
| 88 | Myostatin_99_v254 | 3.6 | 3314 | 921 | A |
| 89 | Myostatin_99_v255 | 1.6 | 191 | 119 | A |
| 3 | Myostatin_99_Variant_07 | 26.2 | 1047 | 40 | A |
| 30 | Myostatin_99_v145 | 1.6 | 414 | 259 | A |
| 90 | Myostatin_99_v221 | 0.9 | 72 | 80 | A |
| 91 | Myostatin_99_v261 | 0.16 | 29.00 | 181 | B |
| 92 | Myostatin_99_v265 | 0.10 | 5.30 | 55 | B |
| 93 | Myostatin_99_v298 | 0.11 | 6.80 | 62 | B |
| 94 | Myostatin_99_v309 | 0.09 | 25.00 | 287 | B |
| 95 | Myostatin_99_v315 | 0.10 | 56.81 | 541.6 | B |
| 96 | Myostatin_99_v316 | 0.37 | 89.70 | 239.8 | B |
| 97 | Myostatin_99_v335 | 0.32 | 46.65 | 145.0 | B |
| 98 | Myostatin_99_v338 | 0.38 | 114.30 | 299.8 | B |
| 99 | Myostatin_99_v339 | 0.89 | 128.20 | 144.7 | B |
| 100 | Myostatin_99_v341 | 0.76 | 131.90 | 173.9 | B |
| 101 | Myostatin_99_v344 | 0.08 | 62.30 | 812.6 | C |
| 102 | Myostatin_99_v346 | 0.09 | 10.49 | 116.0 | C |
| 103 | Myostatin_99_v343 | 0.10 | 14.29 | 137.1 | C |
| 104 | Myostatin_99_v372 | 0.09 | 7.08 | 76.611 | D |
| 105 | Myostatin_99_v381 | 0.05 | 0.74 | 16.294 | D |
| 106 | Myostatin_99_v382 | 0.04 | 29.56 | 711.775 | D |
| 107 | Myostatin_99_v383 | 0.18 | 40.45 | 220.797 | D |
| 108 | Myostatin_99_v384 | 0.04 | 32.75 | 805.658 | D |
| 109 | Myostatin_99_v385 | 0.17 | 42.52 | 251.449 | D |
| 238 | Myostatin_99_v358 | 0.17 | 52.83 | 310.4 | D |
| 239 | Myostatin_99_v367 | 0.15 | 116.60 | 757.1 | D |
| 240 | Myostatin_99_v386 | 0.19 | 55.05 | 294.1 | D |
| 241 | Myostatin_99_v393 | 0.12 | 20.84 | 181.1 | D |
| 242 | Myostatin_99_v394 | 0.06 | 2.51 | 43.0 | D |
| 243 | Myostatin_99_v395 | 0.34 | 49.20 | 144.7 | D |
| 244 | Myostatin_Myostatin-00002930 | 2.19 | 194.00 | 88.7 | D |
| 245 | Myostatin_Myostatin-00002934 | 0.15 | 23.99 | 164.2 | D |
| 246 | Myostatin_99_v407 | 0.05 | 2.84 | 51.8 | D |
| 247 | Myostatin_99_v411 | 0.05 | 5.30 | 100.0 | D |
| 248 | Myostatin_99_v413 | 0.09 | 9.42 | 105.1 | D |
| 249 | Myostatin_99_v415 | 0.05 | 2.03 | 37.3 | D |
| 250 | Myostatin_99_v417 | 0.08 | 6.42 | 82.2 | D |
| 251 | Myostatin_99_v418 | 0.22 | 29.62 | 136.2 | D |
| 252 | Myostatin_99_v419 | 0.06 | 22.24 | 384.0 | D |
| 253 | Myostatin_99_v421 | 0.07 | 5.08 | 72.9 | D |
| 254 | Myostatin_99_v422 | 0.06 | 30.87 | 482.9 | D |
| 255 | Myostatin_99_v424 | 0.04 | 15.23 | 422.5 | D |
| 256 | Myostatin_99_v425 | 0.02 | 4.10 | 166.3 | D |
| 257 | Myostatin_99_v426 | 0.05 | 8.28 | 163.5 | D |
| 258 | Myostatin_99_v363 | 0.15 | 57.79 | 395.0 | D |

### Evaluation Procedure (2): Evaluation by 2-concentrations assay

The pGL3(CAGA)₁₂-luc plasmid, namely a pGL3 vector (E1751, Promega) having a luciferase gene inserted downstream of a promoter sequence with 12 CAGA repeats attached, was transfected into HepG2 cell line (HB-8065, ATCC). The culture of HepG2 cell line (HB-8065, ATCC) was performed as in the Evaluation Procedure (1).

Myostatin (788-G8/CF, R&D systems) or GDF-11 (1958-GD-G8/CF, R&D systems) was added at a final concentration of 10 ng/mL. Various peptides synthesized in Examples 1 and 2 were added at 30 nmol/L or 100 nmol/L for myostatin inhibition assay or GDF-11 inhibition assay, respectively. At this time, blank wells, in which neither myostatin, GDF-11, nor each peptide was added, were also prepared and incubated in a CO₂ incubator (37°C, 5% CO₂) for 20 to 24 hours.

The medium containing the cells was removed, and a mixture containing PBS (14190144, Thermo) and a chromogenic substrate One-Glo Luciferase Assay System (E6120, Promega) at a ratio of 1:1 was added to each well at 100 µL/well. This was stirred with a plate shaker for 5 min and transferred to a 384-well plate (3570, Corning) at a volume of 20 µL/well.

The plate was centrifuged at 1,000 × g for 1 min and the luminescence signal (700 nm luminescence) was measured using a plate reader (Envision 2104, PerkinElmer). Based on the measurement results, the inhibition rate at each peptide concentration was calculated. Table 7 shows the results.

**[Table 7-1]**

| SEQ ID No. | Peptide Name | Myostatin Inhibition | | GDF11 Inhibition | |
|---|---|---|---|---|---|
| | | 30nM | 100nM | 30nM | 100nM |
| 110 | Myostatin_Myostatin_000099 | 56% | 87% | 0% | 19% |
| 111 | Myostatin_000099_01meA | 55% | 82% | 0% | 0% |
| 112 | Myostatin_000099_15meC | 50% | 75% | 0% | 4% |
| 113 | Myostatin_000099_15dc | 69% | 89% | 0% | 12% |
| 114 | Myostatin_000099_16Dap | 56% | 83% | 0% | 11% |
| 115 | Myostatin_000099_02Dap | 50% | 82% | 0% | 2% |
| 116 | Myostatin_000099_16de | 50% | 82% | 0% | 3% |
| 117 | Myostatin_000099_02K | 35% | 73% | 0% | 0% |
| 118 | Myostatin_000099_02E | 58% | 83% | 0% | 9% |
| 119 | Myostatin_000099_01Dap | 18% | 62% | 0% | 0% |
| 120 | Myostatin_000099_01ApG | 19% | 65% | 0% | 0% |
| 121 | Myostatin_000099_02A | 64% | 89% | 0% | 5% |
| 122 | Myostatin_99_2da_15dc_NH2 | 94% | 98% | 15% | 48% |
| 123 | Myostatin_99_Variant_06 | 40% | 65% | 0% | 0% |
| 124 | Myostatin_99_Variant_08 | 72% | 92% | 7% | 22% |
| 125 | Myostatin_99_Variant_09 | 65% | 90% | 0% | 11% |
| 126 | Myostatin_99_Variant_10 | 60% | 89% | 11% | 29% |
| 127 | Myostatin_99_Variant_11 | 75% | 94% | 22% | 26% |
| 128 | Myostatin_99_Variant_13 | 51% | 76% | 8% | 12% |
| 129 | Myostatin_99_Variant_14 | 41% | 73% | 0% | 1% |
| 130 | Myostatin_99_Variant_15 | 54% | 84% | 0% | 0% |
| 131 | Myostatin_99_Variant_16 | 34% | 75% | 0% | 4% |
| 132 | Myostatin_99_Variant_17 | 37% | 72% | 0% | 14% |
| 133 | Myostatin_99_Variant_18 | 56% | 80% | 0% | 0% |
| 134 | Myostatin_99_Variant_20 | 62% | 89% | 0% | 0% |
| 135 | Myostatin_99_Variant_21 | 40% | 76% | 0% | 0% |
| 136 | Myostatin_99_Variant_22 | 45% | 72% | 0% | 0% |
| 137 | Myostatin_99_Variant_23 | 32% | 71% | 0% | 0% |
| 138 | Myostatin_99_Variant_24 | 1% | 54% | 0% | 0% |
| 139 | Myostatin_99_Variant_25 | 20% | 50% | 0% | 0% |
| 140 | Myostatin_99_Variant_26 | 26% | 50% | 0% | 0% |
| 141 | Myostatin_99_v27 | 24% | 54% | 0% | 0% |
| 142 | Myostatin_99_v28 | 69% | 92% | 0% | 20% |
| 143 | Myostatin_99_v29 | 57% | 81% | 0% | 0% |
| 144 | Myostatin_99_v30 | 31% | 60% | 0% | 0% |
| 145 | Myostatin_99_v34 | 65% | 89% | 0% | 32% |
| 146 | Myostatin_99_v35 | 95% | 100% | 13% | 36% |
| 147 | Myostatin_99_v36 | 92% | 98% | 2% | 18% |
| 148 | Myostatin_99_v37 | 69% | 88% | 0% | 0% |
| 149 | Myostatin_99_v38 | 40% | 80% | 0% | 11% |
| 150 | Myostatin_99_v39 | 39% | 79% | 0% | 11% |
| 151 | Myostatin_99_v40 | 39% | 67% | 0% | 0% |
| 152 | Myostatin_99_v60 | 25% | 51% | 7% | 5% |
| 153 | Myostatin_99_v102 | 60% | 81% | 0% | 0% |
| 154 | Myostatin_99_v113 | 35% | 70% | 0% | 0% |

**[Table 7-2]**

| | | | | | |
|---|---|---|---|---|---|
| 155 | Myostatin_99_v119 | 46% | 70% | 0% | 0% |
| 156 | Myostatin_99_v126 | 28% | 68% | 0% | 0% |
| 157 | Myostatin_99_v127 | 31% | 57% | 0% | 0% |
| 158 | Myostatin_99_v205 | 37% | 69% | 6% | 1% |
| 159 | Myostatin_99_v206 | 88% | 97% | 0% | 0% |
| 160 | Myostatin_99_v208 | 98% | 100% | 0% | 20% |
| 161 | Myostatin_99_v210 | 95% | 99% | 0% | 7% |
| 162 | Myostatin_99_v213 | 90% | 98% | 0% | 0% |
| 163 | Myostatin_99_v218 | 44% | 74% | 0% | 0% |
| 164 | Myostatin_99_v220 | 95% | 99% | 0% | 5% |
| 165 | Myostatin_99_v222 | 93% | 99% | 0% | 8% |
| 166 | Myostatin_99_v223 | 94% | 99% | 0% | 28% |
| 167 | Myostatin_99_v224 | 96% | 99% | 0% | 0% |
| 168 | Myostatin_99_v225 | 94% | 99% | 0% | 3% |
| 169 | Myostatin_99_v228 | 19% | 60% | 4% | 0% |
| 170 | Myostatin_99_v232 | 58% | 84% | 0% | 0% |
| 171 | Myostatin_99_v233 | 50% | 85% | 8% | 0% |
| 172 | Myostatin_99_v235 | 91% | 99% | 0% | 0% |
| 173 | Myostatin_99_v240 | 69% | 91% | 0% | 0% |
| 174 | Myostatin_99_v242 | 18% | 58% | 0% | 0% |
| 175 | Myostatin_99_v247 | 68% | 94% | 0% | 6% |
| 176 | Myostatin_99_v256 | 57% | 88% | 0% | 4% |
| 177 | Myostatin_99_v258 | 21% | 61% | 0% | 0% |
| 178 | Myostatin_99_v259 | 100% | 100% | 0% | 28% |
| 179 | Myostatin_99_v260 | 100% | 100% | 37% | 75% |
| 91 | Myostatin_99_v261 | 26% | 72% | | |
| 180 | Myostatin_99_v262 | 99% | 100% | 0% | 24% |
| 181 | Myostatin_99_v263 | 100% | 100% | 8% | 56% |
| 182 | Myostatin_99_v264 | 100% | 100% | 24% | 72% |
| 92 | Myostatin_99_v265 | 91% | 98% | | |
| 183 | Myostatin_99_v267 | 100% | 100% | 24% | 64% |
| 184 | Myostatin_99_v268 | 100% | 100% | 65% | 92% |
| 185 | Myostatin_99_v269 | 100% | 100% | 3% | 59% |
| 186 | Myostatin_99_v270 | 100% | 100% | 5% | 68% |
| 187 | Myostatin_99_v288 | 100% | 100% | 56% | 93% |
| 188 | Myostatin_99_v290 | 100% | 100% | 66% | 94% |
| 189 | Myostatin_99_v292 | 95% | 100% | 3% | 2% |
| 190 | Myostatin_99_v295 | 50% | 84% | | |
| 191 | Myostatin_99_v296 | 41% | 83% | | |
| 93 | Myostatin_99_v298 | 100% | 100% | 12% | 55% |
| 192 | Myostatin_99_v299 | 93% | 99% | 0% | 0% |
| 193 | Myostatin_99_v300 | 81% | 97% | 0% | 0% |
| 194 | Myostatin_99_v301 | 93% | 99% | 0% | 0% |
| 195 | Myostatin_99_v302 | 99% | 100% | 0% | 0% |
| 196 | Myostatin_99_v303 | 100% | 100% | 39% | 67% |
| 197 | Myostatin_99_v304 | 74% | 88% | 0% | 0% |
| 198 | Myostatin_99_v305 | 100% | 100% | 0% | 24% |

**[Table 7-3]**

| | | | | | |
|---|---|---|---|---|---|
| 199 | Myostatin_99_v306 | 80% | 94% | 0% | 0% |
| 200 | Myostatin_99_v307 | 89% | 98% | 0% | 12% |
| 201 | Myostatin_99_v308 | 87% | 97% | 0% | 3% |
| 202 | Myostatin_99_v311 | 99% | 100% | 0% | 5% |
| 203 | Myostatin_99_v312 | 98% | 100% | 0% | 6% |
| 204 | Myostatin_99_v313 | 100% | 100% | 12% | 40% |
| 205 | Myostatin_99_v314 | 100% | 100% | 2% | 51% |
| 206 | Myostatin_99_v318 | 100% | 100% | 0% | 19% |
| 207 | Myostatin_99_v321 | 58% | 91% | 0% | 0% |
| 208 | Myostatin_99_v323 | 97% | 99% | 0% | 0% |
| 209 | Myostatin_99_v325 | 100% | 100% | 49% | 86% |
| 210 | Myostatin_99_v330 | 100% | 100% | 0% | 0% |
| 211 | Myostatin_99_v331 | 100% | 100% | 0% | 0% |
| 212 | Myostatin_99_v332 | 100% | 100% | 0% | 0% |
| 213 | Myostatin_99_v333 | 100% | 100% | 0% | 34% |
| 214 | Myostatin_99_v334 | 90% | 99% | 0% | 0% |
| 215 | Myostatin_99_v336 | 99% | 100% | 0% | 0% |
| 216 | Myostatin_99_v337 | 82% | 96% | 0% | 0% |
| 217 | Myostatin_99_v340 | 95% | 100% | 0% | 0% |
| 218 | Myostatin_99_v342 | 100% | 100% | 0% | 47% |
| 219 | Myostatin_99_v345 | 100% | 100% | 7% | 70% |
| 220 | Myostatin_99_v347 | 100% | 100% | 0% | 8% |
| 259 | Myostatin_99_v348 | 61% | 88% | 0% | 0% |
| 260 | Myostatin_99_v349 | 67% | 92% | 0% | 0% |
| 261 | Myostatin_99_v350 | 45% | 85% | 2% | 0% |
| 262 | Myostatin_99_v351 | 90% | 98% | 0% | 0% |
| 263 | Myostatin_99_v352 | 99% | 100% | 0% | 0% |
| 264 | Myostatin_99_v353 | 98% | 100% | 0% | 0% |
| 265 | Myostatin_99_v354 | 98% | 100% | 0% | 0% |
| 266 | Myostatin_99_v355 | 59% | 88% | 0% | 0% |
| 267 | Myostatin_99_v356 | 99% | 100% | 0% | 0% |
| 268 | Myostatin_99_v357 | 98% | 100% | 0% | 0% |
| 269 | Myostatin_99_v359 | 99% | 100% | 0% | 0% |
| 270 | Myostatin_99_v360 | 94% | 99% | 0% | 0% |
| 271 | Myostatin_99_v361 | 96% | 99% | 0% | 0% |
| 272 | Myostatin_99_v364 | 93% | 97% | 0% | 0% |
| 273 | Myostatin_99_v365 | 94% | 99% | 0% | 0% |
| 274 | Myostatin_99_v366 | 99% | 100% | 0% | 0% |
| 275 | Myostatin_99_v368 | 95% | 99% | 0% | 0% |
| 276 | Myostatin_99_v369 | 75% | 95% | 0% | 0% |
| 221 | Myostatin_99_v370 | 100% | 100% | 0% | 0% |
| 222 | Myostatin_99_v371 | 100% | 100% | 0% | 0% |
| 223 | Myostatin_99_v373 | 100% | 100% | 0% | 47% |
| 224 | Myostatin_99_v378 | 99% | 100% | 0% | 0% |
| 225 | Myostatin_99_v379 | 100% | 100% | 0% | 0% |
| 226 | Myostatin_99_v380 | 100% | 100% | 0% | 0% |
| 277 | Myostatin_99_v387 | 100% | 100% | 0% | 0% |

**[Table 7-4]**

| | | | | | |
|---|---|---|---|---|---|
| 278 | Myostatin_99_v388 | 100% | 100% | 0% | 0% |
| 279 | Myostatin_99_v389 | 100% | 100% | 0% | 0% |
| 280 | Myostatin_99_v390 | 99% | 100% | 0% | 0% |
| 281 | Myostatin_99_v391 | 100% | 100% | 0% | 0% |
| 282 | Myostatin_99_v392 | 99% | 100% | 0% | 0% |
| 283 | Myostatin_Myostatin-00002929 | 100% | 100% | 0% | 0% |
| 284 | Myostatin_Myostatin-00002931 | 99% | 100% | 0% | 0% |
| 285 | Myostatin_99_v402 | 99% | 100% | 0% | 0% |
| 286 | Myostatin_99_v403 | 100% | 100% | 0% | 0% |
| 287 | Myostatin_99_v404 | 100% | 100% | 0% | 0% |
| 288 | Myostatin_99_v414 | 100% | 100% | 0% | 44% |
| 289 | Myostatin_99_v420 | 100% | 100% | 0% | 25% |
| 290 | Myostatin_99_v423 | 100% | 100% | 0% | 0% |
| 227 | Myostatin_99_v427 | 98% | 100% | 0% | 0% |
| 228 | Myostatin_99_v428 | 96% | 99% | 0% | 0% |
| 229 | Myostatin_99_v429 | 100% | 100% | 0% | 0% |
| 230 | Myostatin_99_v430 | 100% | 100% | 0% | 0% |
| 231 | Myostatin_99_v431 | 100% | 100% | 0% | 0% |
| 232 | Myostatin_99_v432 | 100% | 100% | 0% | 0% |
| 233 | Myostatin_99_v433 | 100% | 100% | 0% | 0% |
| 291 | Myostatin_99_v434 | 98% | 100% | 0% | 0% |
| 292 | Myostatin_99_v435 | 99% | 100% | 0% | 0% |
| 293 | Myostatin_99_v437 | 100% | 100% | 0% | 9% |
| 294 | Myostatin_99_v438 | 100% | 100% | 0% | 10% |
| 234 | Myostatin_99_v439 | 100% | 100% | 2% | 36% |
| 235 | Myostatin_99_v440 | 99% | 100% | 0% | 9% |
| 236 | Myostatin_99_v441 | 100% | 100% | 0% | 0% |
| 237 | Myostatin_99_v442 | 100% | 100% | 0% | 0% |
| 295 | Myostatin_99_v443 | 98% | 100% | 0% | 0% |
| 296 | Myostatin_99_v444 | 96% | 99% | 0% | 0% |
| 297 | Myostatin_99_v445 | 99% | 100% | 0% | 0% |
| 298 | Myostatin_99_v446 | 76% | 95% | 6% | 0% |
| 299 | Myostatin_99_v447 | 89% | 98% | 0% | 0% |
| 300 | Myostatin_99_v448 | 100% | 100% | 0% | 0% |
| 301 | Myostatin_99_v449 | 95% | 99% | 0% | 0% |
| 302 | Myostatin_99_v450 | 100% | 100% | 0% | 0% |
| 303 | Myostatin_99_v451 | 99% | 100% | 0% | 0% |
| 304 | Myostatin_99_v452 | 94% | 99% | 0% | 0% |
| 305 | Myostatin_99_v453 | 18% | 57% | 1% | 0% |
| 306 | Myostatin_99_v454 | 100% | 100% | 0% | 0% |
| 307 | Myostatin_99_v455 | 99% | 100% | 1% | 0% |
| 308 | Myostatin_99_v456 | 100% | 100% | 1% | 13% |
| 309 | Myostatin_99_v457 | 100% | 100% | 0% | 5% |
| 310 | Myostatin_99_v458 | 100% | 100% | 0% | 3% |
| 311 | Myostatin_99_v459 | 97% | 100% | 0% | 0% |
| 312 | Myostatin_99_v460 | 94% | 100% | 0% | 0% |
| 313 | Myostatin_99_v461 | 100% | 100% | 0% | 0% |
| 314 | Myostatin_99_v462 | 100% | 100% | 0% | 0% |

Tables 6 and 7 have demonstrated that all peptides of the present invention exhibited inhibitory activity against myostatin. In addition, the GDF-11 inhibitory activity of each peptide of the present invention was lower than the myostatin inhibitory activity.

### Example 4: Evaluation of In Vitro Activity by Using Reporter Gene Assay for Myostatin Signaling in HEK-Blue (Trademark) TGF-β Cells

In this Example, the *in vitro* activity of the peptide Myostatin_99_v145 (SEQ ID NO: 30) was evaluated using a reporter gene assay for myostatin signaling.

HEK-Blue (trademark) TGF-β Cells (hkb-tgfbv2, InvivoGen) were cultured in DMEM High glucose GlutaMAX (10569010, Thermo) containing 10% FBS (Heat-inactivated FBS QUALIFIED, 10270-106, Gibco), 50 mg/mL Normocin (ant-nr-05, InvivoGen), 100 mg/mL Zeocin (ant-zn-05, InvivoGen), and 50 µg/mL Gentamicin (11980-14, Nacalai Tesque). The cultured HEK-Blue (trademark) TGF-β Cells were suspended in an assay medium containing DMEM High glucose GlutaMAX, 50 µg/mL Gentamicin, and 1% bovine serum albumin (A7030, Sigma). The cells were seeded at 4 × 10⁴ cells/well onto a 96-well plate (356461, Corning), namely BioCoat poly-D-lysine multi-well plate, and allowed to stand for 30 min.

A 4-fold dilution series of the peptide, namely Myostatin_99_v145 (SEQ ID NO: 30), synthesized according to Example 1, the final concentration of which was from 0.006 nmol/L up to a maximum concentration of 0.1 µmol/L for the myostatin inhibitory activity test, was added to a 96-well U-bottom Non-Binding MicroPlate (650901, Greiner). For the GDF-11 or activin A inhibitory activity test, a 4-fold dilution series, the final concentration of which was from 0.12 nmol/L to a maximum concentration of 2 µmol/L, was added to a 96-well U-bottom Non-Binding MicroPlate (650901, Greiner).

Subsequently, myostatin (788-G8/CF, R&D systems), GDF-11 (1958-GD-G8/CF, R&D systems), or activin A (338-AC-010/CF, R&D systems) was each added at a final concentration of 3 ng/mL. The plate was sealed with Parafilm, and incubated in a CO₂ incubator (37°C, 5% CO₂) for 30 min. The samples were added at 50 µL/well to a plate containing HEK-Blue (trademark) TGF-β Cells, and incubated in a CO₂ incubator (37°C, 5% CO₂) for 24 hours.

To each well of a UV-Star µClear 96-well (655801, Greiner), 180 µL of Detection buffer (prepared by diluting, with 17.64 mL of distilled water, 180 µL of QUANTI-Blue buffer and 180 µL of QUANTI-Blue solution) was dispensed. Here, 20 µL of the supernatant of the culture medium was transferred to the above Detection buffer and stirred with a shaker for 1 min. The plate was covered and incubated in a CO₂ incubator for 3 to 5 hours, and absorbance was measured (Envision 2104, PerkinElmer, 620 nm/bp 40 nm). Based on the absorbance, IC50 values of the peptide were calculated using software (Prism 9, GraphPad). Table 8 and Fig. 1 show the results.

**[Table 8]**

| IC₅₀ and IC₉₀ of peptide v145 against Myostatin, GDF11, and Activin A | | |
|---|---|---|
| | IC50 (nmol/L) | IC90 (nmol/L) |
| Myostatin | 0.23 | 0.64 |
| GDF11 | 61.18 | 701.73 |
| Activin A | >20,000 | >20,000 |
| Selectivity (GDF11/myostatin) | 270 | 1,088 |

The results in Table 8 and Fig. 1 have demonstrated that the peptide of the present invention exhibited myostatin inhibitory activity, and that the GDF-11 inhibitory activity was lower than the myostatin inhibitory activity.

### Example 5: Distribution of Peptide in Skeletal Muscle

In this Example, the distribution of the peptide Myostatin_99_Variant_07 (SEQ ID NO: 3) in skeletal muscle was examined.

The peptide Myostatin_99_Variant_07 (SEQ ID NO: 3), synthesized according to Example 1, was administered subcutaneously at 10 mg/kg to male 6-8-week-old CD1 mice. Plasma and quadriceps concentrations were measured at 24, 48, and 72 hours after administration. Table 9 shows the results.

**[Table 9]**

| Myostatin_99_Variant_07 peptide administered in plasma and muscle tissue | | |
|---|---|---|
| Time after administration (hr) | Plasma concentration (ng/mL) | Intramuscular concentration (ng/mL) |
| 24 | 232 | 283 |
| 48 | 55 | 190 |
| 72 | 40 | 74 |

The peptide was found to be distributed in muscle tissue at a higher concentration than in plasma at all time points. Note that plasma was separated from blood by centrifugation; 25% homogenate of tissue was prepared and deproteinized with 5% formic acid-containing acetonitrile; and the drug concentrations were determined using an LC/MS/MS system (LCMS-8060, Shimadzu Corporation).

### Example 6: In Vivo Myostatin Inhibitory Effect in Wild-Type Mice

In this Example, the *in vivo* myostatin signaling inhibitory effects of various peptides synthesized according to Examples 1 or 2 were evaluated.

Four-week-old DBA/2NCrl male mice (Jackson Laboratory Japan Inc.) were used. The mice were reared in groups of 5 mice/cage and fed *ad libitum* with CRF-1 (Oriental Yeast Co., Ltd.) and water (tap water). Each peptide as shown in Table 10 was administered subcutaneously daily for 14 days, and the body weights of the mice in each group were measured. The body weights were weighed daily using an electronic balance, starting the day before administration.

**[Table 10]**

| Group configuration | | | | | | | |
|---|---|---|---|---|---|---|---|
| Group | Test substance | Dose [mg/kg] | Volume [mL/kg] | Route | Dosing frequency | Number of doses | Number of mice |
| 1 | PBS | - | | | | | 5 |
| 2 | Myostatin_99_Variant_07 | 1 | | | | | 5 |
| 3 | Myostatin_99_Variant_07 | 3 | | | | | 5 |
| 4 | Myostatin_99_v194 | 0.1 | | | | | 5 |
| 5 | Myostatin_99_v194 | 0.3 | | | | | 5 |
| 6 | Myostatin_99_v194 | 1 | | | | | 5 |
| 7 | Myostatin_99_v145 | 0.3 | 10 | Subcutaneous | Once a day | 14 | 5 |
| 8 | Myostatin_99_v145 | 1 | | | | | 5 |
| 9 | Myostatin_99_v145 | 3 | | | | | 5 |
| 10 | Myostatin_99_v145 | 10 | | | | | 5 |
| 11 | Myostatin_99_v148 | 0.1 | | | | | 5 |
| 12 | Myostatin_99_v148 | 0.3 | | | | | 5 |
| 13 | Myostatin_99_v200 | 10 | | | | | 5 |

Fig. 2 shows the time-course change in body weight. Note that for mean ± standard error, * indicates: P < 0.05, ** indicates : P < 0.01 vs. PBS, Student t-test.

In Fig. 2, v07 represents the evaluation results of Myostatin_99_Variant_07 (SEQ ID NO: 3), v194 represents those of Myostatin_99_v194 (SEQ ID NO: 65), v145 represents those of Myostatin_99_v145 (SEQ ID NO: 30), v148 represents those of Myostatin_99_v148 (SEQ ID NO: 31), and v200 represents those of Myostatin_99_v200 (SEQ ID NO: 71).

As shown in Fig. 2, all peptides were found to exhibit dose-dependent weight gain except for v200, which was examined using only one dose. The *in vivo* myostatin inhibitory effect of each peptide of the present invention was verified.

### Example 7: Effect of Peptide on Improvement of Muscle Strength and Inhibition of Muscle Necrosis in B10 mdx Mice

In this Example, the effect of the peptide Myostatin_99_Variant_07 (SEQ ID NO: 3), synthesized according to Example 1, on improvement of muscle strength in DMD model mice was evaluated using B10 mdx model mice (NPL 17).

To five-week-old male C57BL/10-mdx/Jcl (CLEA Japan, Inc.) mice, the peptide or solvent control (Vehicle) PBS was subcutaneously administered as shown in Table 11 three times a week or every day for 5 weeks.

**[Table 11]**

| Group configuration | | | | | | | |
|---|---|---|---|---|---|---|---|
| Group | Test substance | Dose [mg/kg] | Volume [mL/kg] | Route | Dosing frequency | Number of doses | Number of mice |
| 1 | PBS | | 5 | Subcutaneous | Once a day | 21 | 10 |
| 2 | Myostatin_99_Variant_07 | 10 | | | 3 times a week | 9 | 10 |

Using a smart-type grip strength meter for rats and mice (MK-380V; Muromachi Kikai Co., Ltd.), forelimb and hindlimb grip strength measurements and body weight measurements were performed on the day of completion of the acclimation period (grouping day), and on Days 7, 14, 21, 27, and 33. The measurements were conducted with individual identification numbers blinded so that the examiner was unaware of the test conditions of the mice. The grip strength was measured seven times consecutively, and the average of five points, excluding the two points: the highest and lowest values, was used as individual data. The body weight was weighed using an electronic balance.

Fig. 3 shows the time-course change in grip strength. Note that v07 in the figure indicates the evaluation result of Myostatin_99_Variant_07; and for mean ± standard error, * indicates: P < 0.05, ** indicates : P < 0.01 vs. PBS, Student t-test. In the Myostatin_99_Variant_07 peptide administration group, continuous increase in grip strength was observed compared to the PBS administration Vehicle group.

Fig. 4 shows the time-course change in body weight. Note that for mean ± standard error, * indicates: P < 0.05 vs. PBS, Student t-test. The administration of the peptide Myostatin_99_Variant_07 was found to cause the body weight to continuously increase compared to the Vehicle group.

Serum creatine kinase (CK) concentrations (mean ± SEM) on Day 35, when the animals were euthanized, were also examined. The control Vehicle group was 3,836 ± 754 IU/L and the v07 group was 3,247 ± 558 IU/L. Thus, a trend toward lower CK concentrations when v07 was administrated was observed, suggesting an inhibitory effect on skeletal muscle necrosis.

### Example 8: Effect of Subcutaneously Administered Peptide on Improvement of Muscle Strength in DBA/2 mdx Mice

In this Example, the effect of the peptide Myostatin_99_v145 (SEQ ID NO: 30) was evaluated on improvement of muscle strength in DMD model mice. In this Example, DBA/2 mdx model mice (NPLs 18 and 19), which exhibit more severely reduced muscle function than the B10 mdx mice documented in Example 7, were used.

To twenty-week-old female DBA/2N-mdx mice (Central Institute for Experimental Medicine and Life Science), Myostatin_99_v145 or solvent control (PBS) was subcutaneously administered once a week for 4 weeks as shown in Table 12. As a Comparative Example, a single intravenous dose of anti-latent myostatin antibody (GYM329-BS) was administered.

**[Table 12]**

| Group configuration | | | | | | | |
|---|---|---|---|---|---|---|---|
| Group | Test substance | Dose [mg/kg] | Volume [mL/kg] | Route | Dosing frequency | Number of doses | Number of mice |
| 1 | PBS | - | 5 | Subcutaneous | Once a week | 4 | 9 |
| 2 | Myostatin_99_v145 | 1 | | | | | 8 |
| 3 | | 3 | | | | | 8 |
| 4 | GYM329-BS | 10 | 1.3 | Intravenous | Once a month | 1 | 8 |

Using a smart-type grip strength meter for rats and mice (MK-380V; Muromachi Kikai Co., Ltd.), forelimb and hindlimb grip strength measurements were performed on the day of completion of the acclimation period (grouping day), and on Days 7, 14, 21, and 26. The measurements were conducted with individual identification numbers blinded so that the examiner was unaware of the test conditions of the mice. The grip strength was measured seven times consecutively, and the average of five points, excluding the two points: the highest and lowest values, was used as individual data.

Fig. 5 shows the time-course change in grip strength. Note that for mean ± standard error, * indicates: P < 0.05, ** indicates : P < 0.01 vs. PBS, Student t-test. In the Myostatin_99_v145 administration group, continuous increase in grip strength was observed in a dose-dependent manner compared to the PBS administration Vehicle group. The maximum effect of Myostatin_99_v145 was higher than that of GYM329-BS.

### Example 9: Effect of Orally Administered Peptide on Improvement of Muscle Strength in B10-mdx Mice

In this Example, the effect of the peptide Myostatin_99_v145 (SEQ ID NO: 30) was evaluated on improvement of muscle strength when the peptide was orally administered to DMD model mice.

B10-mdx mice were used as the DMD model mice. To five-week-old female C57BL/10-mdx/Jcl mice (CLEA Japan, Inc.; NPL 17), the peptide or solvent control (Vehicle) PBS was subcutaneously administered once a week for 4 weeks. In addition, the peptide or the solvent control, namely 30% Labrasol/PBS, was administered orally to the DMD model mice once a week after fasting in a total of 4 times.

**[Table 13]**

| Group configuration | | | | | | | |
|---|---|---|---|---|---|---|---|
| Group | Test substance | Dose [mg/kg] | Volume [mL/kg] | Route | Dosing frequency | Number of doses | Number of mice |
| 1 | Vehicle | - | | | | | 13 |
| 2 | Myostatin_99_v145 | 3 | 5 (SC) | Oral and Subcutaneous | Once a week | 4 | 12 |
| 3 | | 65 | 10 (PO) | | | | 12 |
| 4 | | 200 | | | | | 13 |

Using a smart-type grip strength meter for rats and mice (MK-380V; Muromachi Kikai Co., Ltd.), forelimb and hindlimb grip strength measurements were performed on the day of completion of the acclimation period (grouping day; 5-week-old), and on Day 7 (6-week-old), Day 14 (7-week-old), Day 20 (8-week-old), and Day 27 (9-week-old). The measurements were conducted with individual identification numbers blinded so that the examiner was unaware of the test conditions of the mice. The grip strength was measured seven times consecutively, and the average of five points, excluding the two points: the highest and lowest values, was used as individual data.

Fig. 6 shows the time-course change in grip strength. Note that for mean ± standard error, * indicates: P < 0.05, ** indicates : P < 0.01 vs. PBS, Student t-test. Even in the case of oral administration, in the Myostatin_99_v145 administration group, continuous increase in grip strength was observed in a dose-dependent manner compared to the PBS administration Vehicle group.

Table 14 shows only the amino acid sequences included in the linkers or additional amino acids used in the Examples.

**[Table 14]**

| SEQ ID No. | SEQ | | | |
|---|---|---|---|---|
| 315 | G | de | G | dk |
| 316 | pipzAc | | | |
| 317 | pipAc | | | |
| 318 | Pip4mAc | | | |
| 319 | Dap | | | |
| 320 | de | | | |
| 321 | Dap | Dap | | |
| 322 | ds | ds | ds | dk |
| 323 | A4paa | | | |
| 324 | KCOpipzaa | | | |
| 325 | Pic4 | | | |

### Example 10:

In this Example, the following peptides or linker-attached peptides were synthesized.

### Example 10-1: Synthesis of Myostatin_99_v424 (SEQ ID NO: 255)

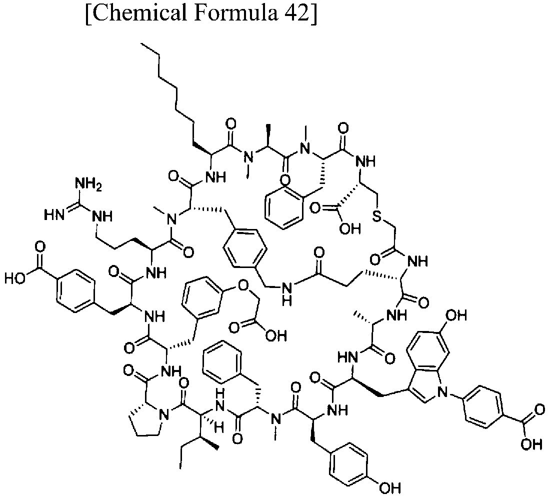

To 1 equivalent of Cl-Trt(2-Cl) resin (Watanabe Chemical Industries, LTD.; 0.79 mmol/g, 5.7 g) swollen with DCM were added a DCM solution containing 0.66 equivalents of Fmoc-dcaa-OtBu and 1.7 equivalents of DIEA, and the mixture was shaken at room temperature for 60 min. Methanol in an excess amount was added and the mixture was stirred for 10 min. The solid-phase resin was washed with DMF, DCM, and diethyl ether, and then dried under reduced pressure. A portion (0.79 mmol/g, 1.04 g) of the obtained solid-phase resin was used to synthesize the target peptide. At that time, CEM's Liberty Blue was used as a solid-phase synthesizer for synthesis according to the manufacturer's instruction. Here, 0.21 M Fmoc-AA (in DMF)/1 M DIC (in DMF)/0.5 M Oxyma pure (in DMF) (5.1 equivalents/9.8 equivalents/4.9 equivalents) were used, and the reaction was carried out once for 30 min at 40°C. Meanwhile, the reaction was carried out twice for residues 4, 6, 7, 10, 12, and 13 at 40°C for 30 min. Fmoc-E(allyl)-OH was used for the reaction of residue 1 and Fmoc-MeF4am(alloc)-OH for the reaction of residue 11. For residue 3, 0.15 M Fmoc-W6H(Boc)1Ph4COO(tBu)-OH (in NMP)/1 M DIC (in DMF)/0.5 M Oxyma pure (in DMF) (3.6 equivalents/9.8 equivalents/4.9 equivalents) were used, and the reaction was carried out once for 30 min at 40°C. The resin was then washed with DMF. In addition, the Fmoc was removed by a reaction twice with a 10% pyrrolidine-containing DMF solution at 25°C for 5 min.

For the cleavage from the solid-phase resin, the resin obtained was first washed five times with DMF and three times with methylene chloride, then washed with diethyl ether, and then dried under reduced pressure. The reaction agent cocktail (40 mL; a mixture of HFIP/DCM at a volume ratio of 20/80) was then added to a reaction vessel containing the solid-phase resin, and the vessel was shaken at room temperature for 60 min. The reaction solution was collected by filtration through a frit. The solid-phase resin remaining in the reaction vessel was shaken again with the cocktail for cleavage, and the solution component was collected from the frit and mixed with the above filtrate. The filtrate obtained was concentrated under reduced pressure by using Genevac EZ-II Elite. When diisopropyl ether was added, a white precipitate occurred.

The mixture was centrifuged (at 9000 rpm at 0°C for 2 min), and the solution was then decanted. The resulting solid was washed again with a small amount of diethyl ether cooled to 0°C and then dried under reduced pressure. The solid (peptide) obtained was used in the next cyclization reaction. The peptide was dissolved in DMF to a final concentration of 1.5 mM based on the number of moles of the solid-phase resin; 1.1 equivalents of HATU and 5 equivalents of DIEA were added; the mixture was stirred at room temperature for 30 min; and acetic acid was then added for quenching the reaction. The resulting reaction solution was concentrated under reduced pressure by using Genevac HT-12.

The resulting mixture was dissolved in 3% HFIP-containing DMF, 0.02 equivalents of Pd(PPh₃)₄ and 20 equivalents of phenylsilane were added. The mixture was stirred for 60 min, and diisopropyl ether was then added. The resulting precipitate was centrifuged (at 9000 rpm at 0°C for 2 min), and the solution was then decanted. The resulting solid was washed again with a small amount of diethyl ether cooled to 0°C and then dried under reduced pressure.

The solid (peptide) obtained was used in the next cyclization reaction. The peptide was dissolved in DMF to a final concentration of 1.5 mM based on the number of moles of the solid-phase resin; 1.1 equivalents of HATU and 5 equivalents of DIEA were added; the mixture was stirred at room temperature for 30 min; and acetic acid was then added for quenching the reaction. The reaction solution was concentrated under reduced pressure by using Genevac EZ-II Elite. When diisopropyl ether was added, a white precipitate occurred. The mixture was centrifuged (at 9000 rpm at 0°C for 2 min), and the solution was then decanted. The resulting solid was washed again with a small amount of diethyl ether cooled to 0°C and then dried under reduced pressure.

The resulting solid was dissolved in a reaction agent cocktail (20 mL; a mixture of TFA/H₂O/TIS/DODT at a volume ratio of 92.5/2.5/2.5/2.5) and shaken at 0°C for 20 min. The white precipitate caused by the addition of diethyl ether was centrifuged (at 9000 rpm at 0°C for 2 min), and the solution was then decanted. The resulting solid was washed again with a small amount of diethyl ether cooled to 0°C and then dried under reduced pressure.

The resulting crude product was purified using the following conditions (column: Agela Claricep Spherical AQ C18 20-35 µm 100 Å 120 g; mobile phase: A = 0.1% TFA (in water), B = 0.1% TFA (in MeCN); temperature: 25°C; gradient (%B): 32-32% over 1 min, 32-47% over 24 min, 47-95% over 10 min, 95-95% over 5 min; flow rate: 10-50 mL/min over 1 min, then 50 ml/min). After lyophilization, the product was purified using the following conditions (column: Waters XSelect (registered trademark) CSH C18 5 µm OBD 50 × 250 mm; mobile phase: A = 1.0% AcOH (in water), B = 1.0% AcOH (in MeCN), C = 0.2 M TEAA (in water), D = acetonitrile; temperature: 50°C; gradient (%A): 0.1-0.1% over 5 min, 0.1-66.7% over 0.1 min, then 100-%B; gradient (%B): 0-0% over 5 min, 0-33.3% over 0.1 min, then 33.3-34.7% over 1.9 min, then 34.7%-34.7% over 3 min, then 34.7%-39.8% over 15.5 min, then 39.8-60% over 1.5 min, then 60-90% over 4 min; gradient (%C): 66.6-66.6% over 5 min, 66.6-0% over 0.1 min, then 0%; gradient (%D): 33.3-33.3% over 5 min, 33.3-0% over 0.1 min, then 0%; flow rate: 20-20 ml/min over 5.1 min, 20-120 ml/min over 1.9 min, then 120 mL/min).

The purity of the target product was calculated from the area ratio of LC/MS (UV wavelength: 225 nm) chromatogram under the analytical conditions. The purity of the target product was 95.3%.

Analytical conditions: retention time = 5.07 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 1181.18 (M+2H)²⁺

### Example 10-2: Synthesis of Myostatin_99_v490 (SEQ ID NO: 349)

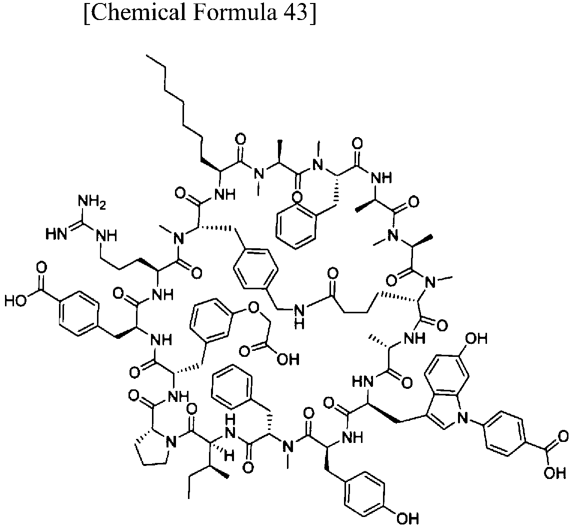

To 1 equivalent of Cl-Trt(2-Cl) resin (Watanabe Chemical Industries, LTD.; 1.15 mmol/g, 2.2 g) swollen with DCM were added a DCM solution containing 0.4 equivalents of Fmoc-da-OH and 1.4 equivalents of DIEA, and the mixture was shaken at room temperature for 60 min. Methanol in an excess amount was added and the mixture was stirred for 10 min. The solid-phase resin was washed with DMF, DCM, and diethyl ether, and then dried under reduced pressure. A portion (0.46 mmol/g, 0.22 g) of the obtained solid-phase resin was used to synthesize the target peptide. At that time, CEM's Liberty Blue was used as a solid-phase synthesizer for synthesis according to the manufacturer's instruction.

For the introduction of each residue, 0.21 M Fmoc-AA (in DMF)/1 M DIC (in DMF)/0.5 M Oxyma pure (in DMF) (4.2 equivalents/8 equivalents/4 equivalents) were used per equivalent of resin, and the reaction was carried out once for 15 min at 50°C. Meanwhile, the reaction was carried out twice for residues 4, 6, 10, 12, and 13 at 50°C for 15 min. Fmoc-MeHgl(allyl)-OH was used for the reaction of residue 1 and Fmoc-MeF4am(alloc)-OH for the reaction of residue 11. The Fmoc was removed by a reaction twice with a 10% pyrrolidine-containing DMF solution at 25°C for 1 min. For residue 16, 0.21 M Fmoc-MeA-OH (in DMF)/0.5 M HATU (in DMF)/1 M DIEA (in DMF) (4.2 equivalents/4 equivalents/8.4 equivalents) were used, and the reaction was carried out once for 30 min at 25°C. The resin was then washed with DMF.

The resin obtained was suspended in 1% HFIP-containing DCM, and 0.2 equivalents of Pd(PPh₃)₄ and 10 equivalents of phenylsilane were added. The mixture was shaken at 25°C for 60 min, and washed with dichloromethane and then DMF in this order. The resulting solid-phase resin was suspended in DMF and shaken with 1.2 equivalents of PyAOP and 3 equivalents of DIEA at room temperature for 30 min. After washing with DMF, the Fmoc group was removed by shaking with a 10% pyrrolidine-containing DMF solution for 3 min at room temperature.

For the cleavage from the solid-phase resin, the resin obtained was first washed five times with DMF and three times with methylene chloride, then washed with diethyl ether, and then dried under reduced pressure. The reaction agent cocktail (14 mL; a mixture of HFIP/DCM at a volume ratio of 20/80) was then added to a reaction vessel containing the solid-phase resin, and the vessel was shaken at room temperature for 60 min. The reaction solution was collected by filtration through a frit. The solid-phase resin remaining in the reaction vessel was shaken again with the cocktail for cleavage, and the solution component was collected from the frit and mixed with the above filtrate. The filtrate obtained was concentrated under reduced pressure. When diethyl ether/hexane (1/1) was then added, a white precipitate occurred. The resulting precipitate was centrifuged (at 9000 rpm at 0°C for 2 min), and the solution was then decanted. The resulting solid was washed again with a small amount of diethyl ether cooled to 0°C and then dried under reduced pressure.

The solid (peptide) obtained was used in the next cyclization reaction. The peptide was dissolved in DMF to a final concentration of 1.5 mM based on the number of moles of the solid-phase resin; 1 equivalent of HATU and 5 equivalents of DIEA were added; the mixture was stirred at room temperature for 30 min; and acetic acid was then added for quenching the reaction. The resulting reaction solution was concentrated under reduced pressure by using Genevac EZ-II Elite. The resulting mixture was dissolved in a reaction agent cocktail (15 mL; a mixture of TFA/H₂O/TIS/DODT/thioanisole at a volume ratio of 72/4/2/2/20) and shaken at 25°C for 20 min. The white precipitate caused by the addition of diethyl ether/hexane (1/1) was centrifuged (at 9000 rpm at 0°C for 2 min), and the solution was then decanted. The resulting solid was washed again with a small amount of diethyl ether cooled to 0°C and then dried under reduced pressure.

The crude product obtained was purified using the following conditions (column: Waters XSelect (registered trademark) CSH Prep C18 5 µm 30 × 150 mm; mobile phase: A = 50 mM TEAA (in water), B = acetonitrile; temperature: 50°C; gradient (%B): 12.1-8.8% over 0.1 min, 8.8-8.8% over 4.9 min, 8.8-12.1% over 1 min, 12.1-37.7% over 3 min, 37.7-42.8% over 9 min, 42.8-60% over 1 min; flow rate: 44-9 mL/min over 0.1 min, 9-9 mL/min over 4.9 min, 9-44 mL/min over 1 min, then 44 mL/min). After lyophilization, the product was dissolved in 0.1 TFA-containing water/acetonitrile (a mixture at a volume ratio of 50/50) and lyophilized again.

The purity of the target product was calculated from the area ratio of LC/MS (UV wavelength: 225 nm) chromatogram under the analytical conditions. The purity of the target product was 92.9%.

Analytical conditions: retention time = 6.04 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 1192.71 (M+2H)²⁺

### Example 10-3: Synthesis of Myostatin_99_v482 (SEQ ID NO: 341)

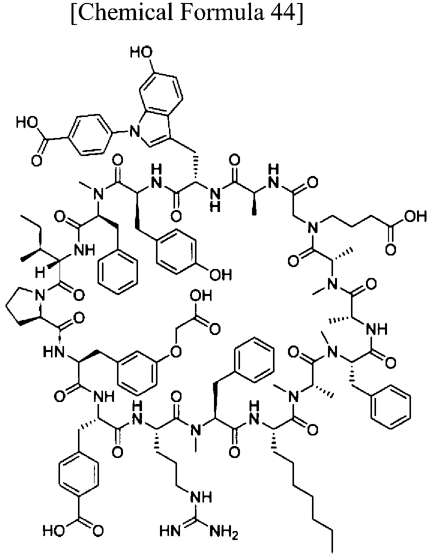

H-A-Trt(2-Cl) resin (Watanabe Chemical Industries, LTD.; 1.08 mmol/g, 0.23 g) was used to synthesize the target peptide. At that time, CEM's Liberty Blue was used as a solid-phase synthesizer for synthesis according to the manufacturer's instruction. For the introduction of each residue, 0.21 M Fmoc-AA (in DMF)/1 M DIC (in DMF)/0.5 M Oxyma pure (in DMF) (4.2 equivalents/8 equivalents/4 equivalents) were used per equivalent of resin.

The Fmoc group was deprotected using a DMF solution containing 10% pyrrolidine. Further, the peptide was elongated according to the conditions listed in Table 15 below.

**[Table 15]**

| Solid-phase synthesis introduction order | Residue number | Fmoc amino acid | Coupling reaction conditions | Fmoc deprotection conditions |
|---|---|---|---|---|
| 1 | 1 | Fmoc-CrpG(tBu)-OH | 50°C, 15 min, 1 time | 25°C, 1 min, 2 times |
| 2 | 16 | Fmoc-MeA-OH | 50°C, 15 min, 2 times | 25°C, 1 min, 2 times |
| 3 | 15 | Fmoc-da-OH | 50°C, 15 min, 2 times | 25°C, 1 min, 2 times |
| 4 | 14 | Fmoc-MeF-OH | 50°C, 15 min, 1 time | 25°C, 1 min, 2 times |
| 5 | 13 | Fmoc-MeA-OH | 50°C, 15 min, 2 times | 25°C, 1 min, 2 times |
| 6 | 12 | Fmoc-Ano-OH | 50°C, 15 min, 2 times | 25°C, 1 min, 2 times |
| 7 | 11 | Fmoc-MeF-OH | 50°C, 15 min, 1 time | 25°C, 1 min, 2 times |
| 8 | 10 | Fmoc-R(Pbf)-OH | 50°C, 15 min, 2 times | 25°C, 1 min, 2 times |
| 9 | 9 | Fmoc-F4COO(tBu)-OH | 50°C, 15 min, 1 time | 25°C, 1 min, 2 times |
| 10 | 8 | Fmoc-F3aao(tBu)-OH | 50°C, 15 min, 1 time | 25°C, 1 min, 2 times |
| 11 | 7 | Fmoc-dp-OH | 50°C, 15 min, 2 times | 25°C, 1 min, 2 times |
| 12 | 6 | Fmoc-I-OH | 50°C, 15 min, 2 times | 25°C, 1 min, 2 times |
| 13 | 5 | Fmoc-MeF-OH | 50°C, 15 min, 1 time | 25°C, 1 min, 2 times |
| 14 | 4 | Fmoc-Y(tBu)-OH | 50°C, 15 min, 2 times | 25°C, 1 min, 2 times |
| 15 | 3 | Fmoc-W6H(Boc)1 Ph4COO(tBu)-OH | 50°C, 15 min, 1 time | 25°C, 5 min, 2 times |

For the cleavage from the solid-phase resin, the resin obtained was first washed five times with DMF and three times with methylene chloride, then washed with diethyl ether, and then dried under reduced pressure. The reaction agent cocktail (10 mL; a mixture of HFIP/DCM at a volume ratio of 20/80) was then added to a reaction vessel containing the solid-phase resin, and the vessel was shaken at room temperature for 60 min. The reaction solution was collected by filtration through a frit. The solid-phase resin remaining in the reaction vessel was shaken again with the cocktail for cleavage, and the solution component was collected from the frit and mixed with the above filtrate. The filtrate was concentrated under reduced pressure. When diethyl ether was added to the resulting mixture, a cloudy precipitate occurred.

The mixture was centrifuged (at 9000 rpm at 0°C for 2 min), and the solution was then decanted. The resulting solid was washed again with a small amount of diethyl ether cooled to 0°C and then dried under reduced pressure. The solid (peptide) obtained was used in the next cyclization reaction. The cyclization reaction of the peptide was performed by dissolving the peptide in DMF to a final concentration of 7 mM based on the number of moles of the solid-phase resin; adding 1.1 equivalents of HATU and 5 equivalents of DIEA; stirring the mixture at room temperature for 30 min; and then adding acetic acid for quenching the reaction. The resulting reaction solution was concentrated under reduced pressure by using Genevac HT-12.

The resulting residue was dissolved in a reaction agent cocktail-D (4 mL; a mixture of TFA/TIS/H₂O/thioanisole at a volume ratio of 70/2.5/2.5/2.5) and shaken at room temperature for 55 min. The resulting reaction solution was added to diethyl ether, and a precipitate occurred. The mixture was centrifuged (at 9000 rpm at 0°C for 2 min), and the solution was then decanted. The resulting solid was washed again with a small amount of diethyl ether cooled to 0°C and then dried under reduced pressure.

The resulting crude product was purified using the following conditions (column: Waters XBridge (registered trademark) C18 5 µm 50 × 150 mm; mobile phase: A = 0.1% TFA (in water), B = 0.1% TFA (in MeCN); temperature: 40°C; gradient (%B): 16-16% over 2 min, 16-41% over 1 min, 41-46% over 8 min, 46-60% over 1 min; flow rate: 20-20 mL/min over 1 min, 20-120 mL/min over 1 min, then 120 ml/min).

The purity of the target product was calculated from the area ratio of LC/MS (UV wavelength: 225 nm) chromatogram under the analytical conditions. The purity of the target product was 95.0%.

Analytical conditions: retention time = 5.90 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 1180.16 (M+2H)²⁺

### Example 10-4: Synthesis of Myostatin_99_v483 (SEQ ID NO: 342)

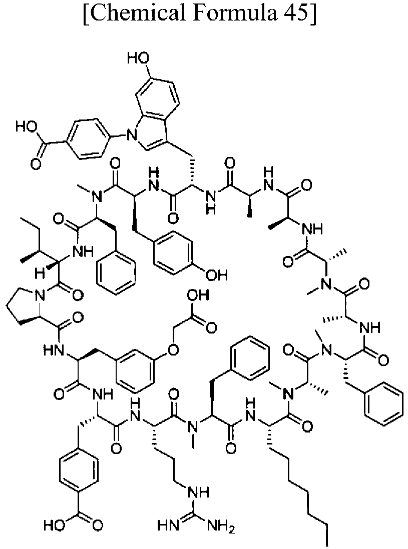

H-A-Trt(2-Cl) resin (Watanabe Chemical Industries, LTD.; 1.08 mmol/g, 0.23 g) was used to synthesize the target peptide. Peptide elongation, cleavage from the resin, cyclization reaction, and deprotection were performed in substantially the same manner as for Myostatin_99_v482.

The resulting crude product was purified using the following conditions (column: Waters XBridge (registered trademark) C18 5 µm 50 × 150 mm; mobile phase: A = 0.1% TFA (in water), B = 0.1% TFA (in MeCN); temperature: 40°C; gradient (%B): 19-19% over 2 min, 19-44% over 1 min, 44-49% over 8 min, 49-60% over 1 min; flow rate: 20-20 mL/min over 1 min, 20-120 mL/min over 1 min, then 120 ml/min).

The purity of the target product was calculated from the area ratio of LC/MS (UV wavelength: 225 nm) chromatogram under the analytical conditions. The purity of the target product was 96.0%.

Analytical conditions: retention time = 6.13 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 1144.14 (M+2H)²⁺

### Example 10-5: Synthesis of Myostatin_99_v482-biotin (SEQ ID NO: 364)

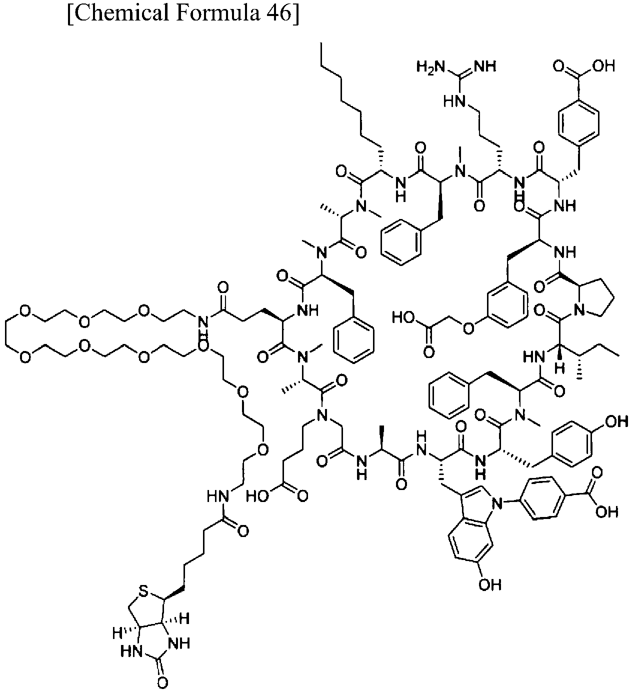

H-A-Trt(2-Cl) resin (Watanabe Chemical Industries, LTD.; 1.08 mmol/g, 0.12 g) was used to synthesize the target peptide. At that time, CEM's Liberty Blue was used as a solid-phase synthesizer for synthesis according to the manufacturer's instruction. For the introduction of each residue, 0.21 M Fmoc-AA (in DMF)/1 M DIC (in DMF)/0.5 M Oxyma pure (in DMF) (4.2 equivalents/8 equivalents/4 equivalents) were used per equivalent of resin.

The Fmoc group was deprotected using a DMF solution containing 10% pyrrolidine. Further, the peptide was elongated according to the conditions listed in Table 16 below.

**[Table 16]**

| Solid-phase synthesis introduction order | Residue number | Fmoc amino acid | Coupling reaction conditions | Fmoc deprotection conditions |
|---|---|---|---|---|
| 1 | 1 | Fmoc-CrpG(tBu)-OH | 50°C, 15 min, 1 time | 25°C, 1 min, 2 times |
| 2 | 16 | Fmoc-MeA-OH | 50°C, 15 min, 2 times | 25°C, 1 min, 2 times |
| 3 | 15 | Fmoc-de(allyl)-OH | 50°C, 15 min, 2 times | 25°C, 1 min, 2 times |
| 4 | 14 | Fmoc-MeF-OH | 50°C, 15 min, 1 time | 25°C, 1 min, 2 times |
| 5 | 13 | Fmoc-MeA-OH | 50°C, 30 min, 2 times | 25°C, 1 min, 2 times |
| 6 | 12 | Fmoc-Ano-OH | 50°C, 30 min, 2 times | 25°C, 1 min, 2 times |
| 7 | 11 | Fmoc-MeF-OH | 50°C, 15 min, 1 time | 25°C, 1 min, 2 times |
| 8 | 10 | Fmoc-R(Pbf)-OH | 50°C, 15 min, 2 times | 25°C, 1 min, 2 times |
| 9 | 9 | Fmoc-F4COO(tBu)-OH | 50°C, 15 min, 1 time | 25°C, 1 min, 2 times |
| 10 | 8 | Fmoc-F3aao(tBu)-OH | 50°C, 15 min, 1 time | 25°C, 1 min, 2 times |
| 11 | 7 | Fmoc-dp-OH | 50°C, 15 min, 1 time | 25°C, 1 min, 2 times |
| 12 | 6 | Fmoc-I-OH | 50°C, 30 min, 2 times | 25°C, 1 min, 2 times |
| 13 | 5 | Fmoc-MeF-OH | 50°C, 15 min, 1 time | 25°C, 1 min, 2 times |
| 14 | 4 | Fmoc-Y(tBu)-OH | 50°C, 15 min, 2 times | 25°C, 1 min, 2 times |
| 15 | 3 | Fmoc-W6H(Boc)1Ph4COO(tBu)-OH | 50°C, 15 min, 1 time | - |

The resulting solid-phase resin was suspended in DCM, 5 equivalents of Fmoc-OSu was added, shaken at 25°C for 1 hour, and then washed with DMF. The mixture obtained was suspended in DCM, and 0.2 equivalents of Pd(PPh₃)₄ and 10 equivalents of phenylsilane were added. The resulting mixture was shaken at 25°C for 60 min, and washed with DMF and then DCM in this order. The resulting solid-phase resin was suspended in DMF, and 3 equivalents of Boc-NH-PEG9-NH2, 6 equivalents of DIC, and 3 equivalents of Oxyma pure were added. The mixture was then shaken for 60 min at room temperature. The resin obtained for deprotection of the Fmoc group was washed with DMF, and was shaken with a 10% pyrrolidine-containing DMF solution for 10 min at 25°C.

For the cleavage from the solid-phase resin, the resin obtained was first washed five times with DMF and three times with methylene chloride, then washed with diethyl ether, and then dried under reduced pressure. The reaction agent cocktail (5 mL; a mixture of HFIP/DCM at a volume ratio of 20/80) was then added to a reaction vessel containing the solid-phase resin, and the vessel was shaken at 25°C for 60 min. The reaction solution was collected by filtration through a frit. The solid-phase resin remaining in the reaction vessel was shaken again with the cocktail for cleavage, and the solution component was collected from the frit and mixed with the above filtrate. The filtrate was concentrated under reduced pressure. When diisopropyl ether was added to the resulting mixture, a cloudy precipitate occurred.

The mixture was centrifuged (at 9000 rpm at 0°C for 2 min), and the solution was then decanted. The resulting solid was washed again with a small amount of diethyl ether cooled to 0°C and then dried under reduced pressure. The solid (peptide) obtained was used in the next cyclization reaction. The cyclization reaction of the peptide was performed by dissolving the peptide in DMF to a final concentration of 5 mM based on the number of moles of the solid-phase resin; adding 1.2 equivalents of HATU and 3 equivalents of DIEA; stirring the mixture at 25°C for 60 min; and then adding acetic acid for quenching the reaction. The resulting reaction solution was concentrated under reduced pressure by using Genevac EZ-II Elite.

The resulting residue was dissolved in a reaction agent cocktail (4 mL; a mixture of TFA/TIS/H₂O at a volume ratio of 95/2.5/2.5) and shaken for 60 min at 25°C. The resulting reaction solution was added to diisopropyl ether, and a precipitate occurred. The mixture was centrifuged (at 9000 rpm at 0°C for 2 min), and the solution was then decanted. The resulting solid was washed again with a small amount of diethyl ether cooled to 0°C and then dried under reduced pressure. The resulting mixture was dissolved in DMSO to a final concentration of 25 mM based on the number of moles of the solid-phase resin; 1.1 equivalents of Biotin-Osu and 5 equivalents of DIEA were added; the mixture was stirred at 25°C for 60 min; and acetic acid was then added for quenching.

The resulting mixture was purified using the following conditions (column: Waters XBridge (registered trademark) C18 5 µm 19 × 150 mm; mobile phase: A = 0.1% TFA (in water), B = 0.1% TFA (in MeCN); temperature: 50°C; gradient (%B): 13-38% over 3 min, 38-43% over 8 min, 43-60% over 1 min; flow rate: 17 ml/min).

The purity of the target product was calculated from the area ratio of LC/MS (UV wavelength: 225 nm) chromatogram under the analytical conditions. The purity of the target product was 92.9%.

Analytical conditions: retention time = 5.59 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 1028.11 (M+3H)³⁺

### Example 10-6: Synthesis of Myostatin_99_v488 (SEQ ID NO: 347)

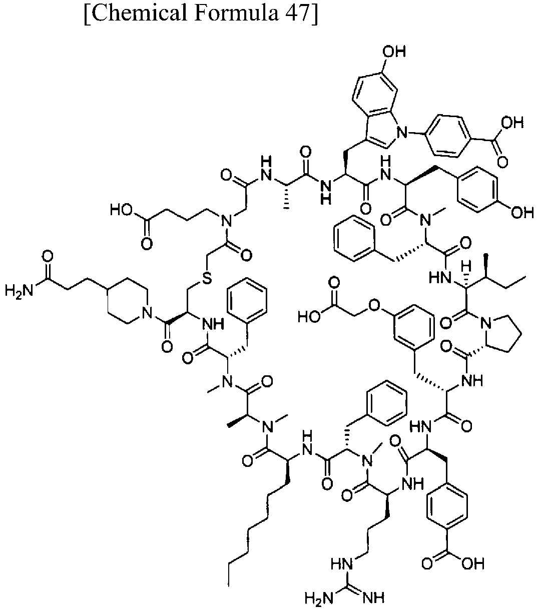

To 1 equivalent of Sieber amide resin (Watanabe Chemical Industries, LTD.; 1 mmol/g, 0.70 g) swollen with DCM were added a DMF solution containing 4.2 equivalents of Fmoc-Pip4mAc-OH, 4 equivalents of HATU, and 8 equivalents of DIEA, and the mixture was shaken at room temperature for 60 min. The solid-phase resin was washed with DMF, DCM, and diethyl ether, and then dried under reduced pressure. A portion (0.60 mmol/g, 0.42 g) of the solid-phase resin obtained was used. The Fmoc group was first removed by the above-described common method, and the target peptide was then synthesized. At that time, CEM's Liberty Blue was used as a solid-phase synthesizer for synthesis according to the manufacturer's instruction. For the introduction of each residue, 0.21 M Fmoc-AA (in DMF)/0.5 M HATU (in DMF)/1 M DIEA (in DMF) (4.2 equivalents/4 equivalents/8 equivalents) were used per equivalent of resin. The reaction was carried out once at 90°C for 3 min. Meanwhile, the reaction was carried out twice for residues 4 and 13 at 90°C for 10 min. The reaction was carried out twice for residues 10 and 15 at 50°C for 15 min. The reaction was carried out twice for residue 12 at 75°C for 30 min. In addition, the Fmoc was removed by a reaction once with a 10% pyrrolidine-containing DMF solution at 90°C for 1 min. Meanwhile, the reaction was carried out twice for residues 4, 6, 10, 12, 13, and 15 at 25°C for 1 min.

A chloroacetyl group was introduced as follows: the Fmoc group of the α-amino group was removed from the solid-phase resin, on which the Fmoc-protected peptide obtained in the previous step was immobilized, by the procedure described above; the resin was suspended in DMF; 5 equivalents of chloroacetic acid, 5 equivalents of HATU, and 8 equivalents of DIEA were added; and the mixture was shaken at 25°C for 30 min.

For the deprotection of side chains and the cleavage from the solid-phase resin, the resin obtained after the chloroacetyl group introduction step was first washed five times with DMF and three times with methylene chloride, then washed with diethyl ether, and then dried under reduced pressure. The reaction agent cocktail-D (15 mL; a mixture of TFA/H₂O/TIS/thioanisole at a volume ratio of 70:2.5:2.5:2.5) was then added to a reaction vessel containing the solid-phase resin, and the vessel was shaken at room temperature for 60 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diisopropylether/hexane (1/1) mixed solvent cooled to 0°C, a cloudy precipitate occurred.

The mixture was centrifuged (at 9000 rpm at 0°C for 2 min), and the solution was then decanted. The resulting solid was washed again with a small amount of diethyl ether cooled to 0°C and then dried under reduced pressure. The solid (peptide) obtained was used in the next cyclization reaction. The cyclization reaction of the peptide was performed by dissolving the peptide in water/acetonitrile (1/1) to a final concentration of 2.5 mM based on the number of moles of the solid-phase resin; adding 10 equivalents of triethylamine; stirring the mixture at room temperature for 4 hours; and then adding acetic acid for quenching the reaction. The resulting reaction solution was concentrated under reduced pressure by using Genevac HT-12.

The mixture obtained was purified using the following conditions (column: Waters XSelect (registered trademark) CSH Prep C18 5 µm 30 × 150 mm; mobile phase: A = 50 mM TEAA (in water), B = acetonitrile; temperature: 50°C; gradient (%B): 6.0-2.2% over 0.1 min, 2.2-2.2% over 4.9 min, 2.2-6.0% over 1 min, 6.0-31.6% over 3 min, 31.6-36.7% over 9 min, 36.7-60% over 1 min; flow rate: 44-9 mL/min over 0.1 min, 9-9 mL/min over 4.9 min, 9-44 mL/min over 1 min, then 44 mL/min). After lyophilization, purification was performed again under the same conditions. After lyophilization, the product was dissolved in 0.1 TFA-containing water/acetonitrile (a mixture at a volume ratio of 50/50) and lyophilized again.

The purity of the target product was calculated from the area ratio of LC/MS (UV wavelength: 225 nm) chromatogram under the analytical conditions. The purity of the target product was 98.3%.

Analytical conditions: retention time = 5.32 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 1251.85 (M+2H)²⁺

### Example 10-7: Synthesis of Myostatin_99_v478 (SEQ ID NO: 337)

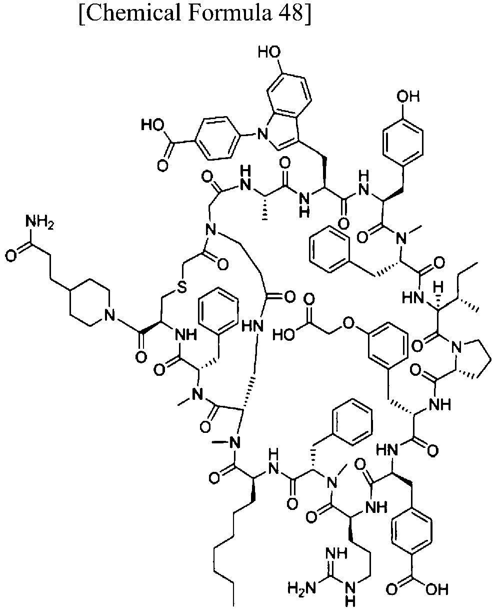

The target peptide was synthesized by the general procedure described above while using Sieber amide resin (0.60 mmol/g, 0.42 g; Watanabe Chemical Industries, LTD.) and removing the Fmoc group at first. At that time, CEM's Liberty Blue was used as a solid-phase synthesizer for synthesis according to the manufacturer's instruction. For the introduction of each residue, 0.21 M Fmoc-AA (in DMF)/1 M DIC (in DMF)/0.5 M Oxyma pure (in DMF) (4.2 equivalents/8 equivalents/4 equivalents) were used per equivalent of resin. The reaction was carried out once at 90°C for 3 min. Meanwhile, the reaction was carried out twice for residues 4 and 6 at 90°C for 10 min. The reaction was carried out twice for residues 10 and 15 at 50°C for 15 min. The reaction was carried out twice for residues 12 and 13 at 75°C for 30 min. Fmoc-CeG(allyl)-OH was used for the reaction of residue 1 and Fmoc-MeDap(alloc)-OH for the reaction of residue 13. In addition, the Fmoc was removed by a reaction once with a 10% pyrrolidine-containing DMF solution at 90°C for 1 min. Meanwhile, the reaction was carried out twice for residues 4, 6, 10, 12, 13, and 15 at 25°C for 1 min. The resulting solid-phase resin was suspended in DCM, 5 equivalents of Fmoc-OSu was added, shaken at 25°C for 1 hour, and then washed with DMF.

The resin obtained was suspended in 2% HFIP-containing DCM, and 0.2 equivalents of Pd(PPh₃)₄ and 10 equivalents of phenylsilane were added. The mixture was shaken at 25°C for 60 min, and washed with DCM and then DMF in this order. The resulting resin was suspended in DMF to a final concentration of 25 mM based on the number of moles of the solid-phase resin, and 1.2 equivalents of PyAOP and 5 equivalents of DIEA were added. The mixture was then stirred at room temperature for 60 min. After washing with DMF, the Fmoc group was removed by shaking with a 10% pyrrolidine-containing DMF solution for 10 min at room temperature. The resulting resin was suspended in DMF, and 5.2 equivalents of chloroacetic acid, 5 equivalents of HATU, and 10 equivalents of DIEA were added. The mixture was stirred at 25°C for 30 min. In this way, the chloroacetyl group was introduced.

For the deprotection of side chains and the cleavage from the solid-phase resin, the resin obtained after the chloroacetyl group introduction step was first washed five times with DMF and three times with methylene chloride, then washed with diethyl ether, and then dried under reduced pressure. The reaction agent cocktail (15 mL; a mixture of TFA/H₂O/TIS/DODT/thioanisole at a volume ratio of 70/2.5/2.5/5/20) was then added to a reaction vessel containing the solid-phase resin, and the vessel was shaken at room temperature for 60 min. The reaction solution was collected by filtration through a frit. The solid-phase resin remaining in the reaction vessel was shaken again with the cocktail for cleavage, and the solution component was collected from the frit and mixed with the above filtrate. When the filtrate was added to an excess of diethyl ether/hexane (1/1) mixed solvent cooled to 0°C, a cloudy precipitate occurred.

The mixture was centrifuged (at 9000 rpm at 0°C for 2 min), and the solution was then decanted. The resulting solid was washed again with a small amount of diethyl ether cooled to 0°C and then dried under reduced pressure. The solid (peptide) obtained was used in the next cyclization reaction. The cyclization reaction of the peptide was performed by dissolving the peptide in water/acetonitrile (1/1) to a final concentration of 5 mM based on the number of moles of the solid-phase resin; adding 10 equivalents of triethylamine; stirring the mixture at room temperature for 60 min; and then adding acetic acid for quenching the reaction. The resulting reaction solution was concentrated under reduced pressure by using Genevac EZ-II Elite.

The resulting mixture was purified using the following conditions (column: Waters XSelect (registered trademark) C18 5 µm 19 × 150 mm; mobile phase: A = 0.1% TFA (in water), B = 0.1% TFA (in MeCN); temperature: 40°C; gradient (%B): 36-36% over 3 min, 36-41% over 8 min, 41-60% over 1 min; flow rate: 20-20 mL/min over 1 min, 20-120 mL/min over 2 min, then 120 ml/min).

The purity of the target product was calculated from the area ratio of LC/MS (UV wavelength: 225 nm) chromatogram under the analytical conditions. The purity of the target product was 98.7%.

Analytical conditions: retention time = 5.18 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 1243.60 (M+2H)²⁺

### Example 10-8: Synthesis of Myostatin_99_v489 (SEQ ID NO: 348)

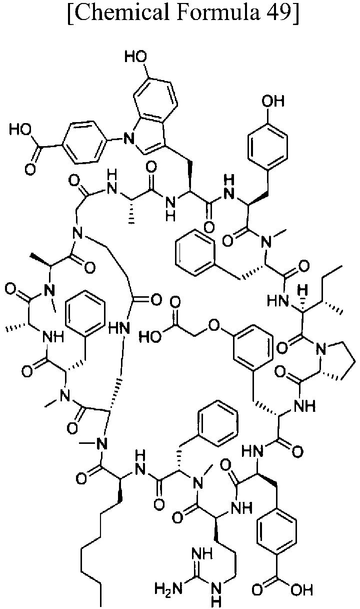

H-A-Trt(2-Cl) resin (Watanabe Chemical Industries, LTD.; 1.08 mmol/g, 0.12 g) was used to synthesize the target peptide. At that time, CEM's Liberty Blue was used as a solid-phase synthesizer for synthesis according to the manufacturer's instruction. For the introduction of each residue, 0.21 M Fmoc-AA (in DMF)/1 M DIC (in DMF)/0.5 M Oxyma pure (in DMF) (4.2 equivalents/8 equivalents/4 equivalents) were used per equivalent of resin.

The Fmoc group was deprotected using a DMF solution containing 10% pyrrolidine. Further, the peptide was elongated according to the conditions listed in Table 17 below.

**[Table 17]**

| Solid-phase synthesis introduction order | Residue number | Fmoc amino acid | Coupling reaction conditions | Fmoc deprotection conditions |
|---|---|---|---|---|
| 1 | 1 | Fmoc-CeG(allyl)-OH | 50°C, 20 min, 1 time | 25°C, 5 min, 2 times |
| 2 | 16 | Fmoc-MeA-OH | 50°C, 20 min, 2 times | 25°C, 5 min, 2 times |
| 3 | 15 | Fmoc-da-OH | 50°C, 20 min, 2 times | 25°C, 5 min, 2 times |
| 4 | 14 | Fmoc-MeF-OH | 50°C, 20 min, 1 time | 25°C, 5 min, 2 times |
| 5 | 13 | Fmoc-MeDap(alloc)-OH | 50°C, 20 min, 2 times | 25°C, 5 min, 2 times |
| 6 | 12 | Fmoc-Ano-OH | 50°C, 20 min, 2 times | 25°C, 5 min, 2 times |
| 7 | 11 | Fmoc-MeF-OH | 50°C, 20 min, 1 time | 25°C, 5 min, 2 times |
| 8 | 10 | Fmoc-R(Pbf)-OH | 50°C, 20 min, 2 times | 25°C, 5 min, 2 times |
| 9 | 9 | Fmoc-F4COO(tBu)-OH | 50°C, 20 min, 1 time | 25°C, 5 min, 2 times |
| 10 | 8 | Fmoc-F3aao(tBu)-OH | 50°C, 20 min, 1 time | 25°C, 5 min, 2 times |
| 11 | 7 | Fmoc-dp-OH | 50°C, 20 min, 2 times | 25°C, 5 min, 2 times |
| 12 | 6 | Fmoc-I-OH | 50°C, 20 min, 2 times | 25°C, 5 min, 2 times |
| 13 | 5 | Fmoc-MeF-OH | 50°C, 20 min, 1 time | 25°C, 5 min, 2 times |
| 14 | 4 | Fmoc-Y(tBu)-OH | 50°C, 20 min, 2 times | 25°C, 5 min, 2 times |
| 15 | 3 | Fmoc-W6H(Boc)1 Ph4COO(tBu)-OH | 50°C, 20 min, 1 time | - |

The resulting solid-phase resin was suspended in DCM, 5 equivalents of Fmoc-OSu was added, shaken at 25°C for 1 hour, and then washed with DMF. The resin obtained was suspended in DCM, and 0.2 equivalents of Pd(PPh₃)₄ and 20 equivalents of phenylsilane were added. The resulting mixture was shaken at 25°C for 60 min, and washed with DCM and then DMF in this order. The resulting resin was suspended in DMF to a final concentration of 3.5 mM based on the number of moles of the solid-phase resin, and 1.01 equivalents of PyAOP and 5 equivalents of DIEA were added. The mixture was then stirred at room temperature for 20 min. After washing with DMF, the Fmoc group was removed by shaking with a 10% pyrrolidine-containing DMF solution for 30 min at room temperature.

For the cleavage from the solid-phase resin, the resin obtained was first washed five times with DMF and three times with methylene chloride, then washed with diethyl ether, and then dried under reduced pressure. The reaction agent cocktail (10 mL; a mixture of HFIP/DCM at a volume ratio of 20/80) was then added to a reaction vessel containing the solid-phase resin, and the vessel was shaken at room temperature for 60 min. The reaction solution was collected by filtration through a frit. The solid-phase resin remaining in the reaction vessel was shaken again with the cocktail for cleavage, and the solution component was collected from the frit and mixed with the above filtrate. The filtrate obtained was concentrated under reduced pressure. When diethyl ether was then added, a white precipitate occurred. The resulting precipitate was centrifuged (at 9000 rpm at 0°C for 2 min), and the solution was then decanted. The resulting solid was washed again with a small amount of diethyl ether cooled to 0°C and then dried under reduced pressure.

The solid (peptide) obtained was used in the next cyclization reaction. The peptide was dissolved in DMF to a final concentration of 7 mM based on the number of moles of the solid-phase resin; 1.1 equivalents of HATU and 5 equivalents of DIEA were added; the mixture was stirred at room temperature for 30 min; and acetic acid was then added for quenching the reaction. The resulting reaction solution was concentrated under reduced pressure by using Genevac HT-12. The resulting mixture was dissolved in a reaction agent cocktail (12 mL; a mixture of TFA/H₂O/TIS/thioanisole at a volume ratio of 70/2.5/2.5/25) and shaken at 25°C for 35 min. The white precipitate caused by the addition of diethyl ether was centrifuged (at 9000 rpm at 0°C for 2 min), and the solution was then decanted. The resulting solid was washed again with a small amount of diethyl ether cooled to 0°C and then dried under reduced pressure.

The crude product obtained was purified using the following conditions (column: Waters XSelect (registered trademark) CSH Prep C18 5 µm 30 × 150 mm; mobile phase: A = 50 mM TEAA (in water), B = acetonitrile; temperature: 50°C; gradient (%B): 11.1-7.8% over 0.1 min, 7.8-7.8% over 4.9 min, 7.8-11.1% over 1 min, 11.1-36.7% over 3 min, 36.7-41.8% over 9 min, 41.8-60% over 1 min; flow rate: 44-9 mL/min over 0.1 min, 9-9 mL/min over 4.9 min, 9-44 mL/min over 1 min, then 44 mL/min). After lyophilization, the product was dissolved in 0.1 TFA-containing water/acetonitrile (a mixture at a volume ratio of 50/50) and lyophilized again.

The purity of the target product was calculated from the area ratio of LC/MS (UV wavelength: 225 nm) chromatogram under the analytical conditions. The purity of the target product was 94.1%.

Analytical conditions: retention time = 5.77 min; column: Kinetex EVO C18 2.6 µm 2.1 × 150 mm, 100 Å; mobile phase: A = 0.025% TFA (in water), B = 0.025% TFA (in MeCN); temperature: 60°C; gradient (% B conc): 20-60% over 7.15 min, then 60-95% over 0.3 min, then 95-95% over 1.55 min; flow rate: 0.5 mL/min.

ESI-MS(+): observed m/z = 1171.75 (M+2H)²⁺

### Example 11: Analysis of Peptide

The target peptides were synthesized according to the common methods described above and the procedures in Examples 1, 2, and 10.

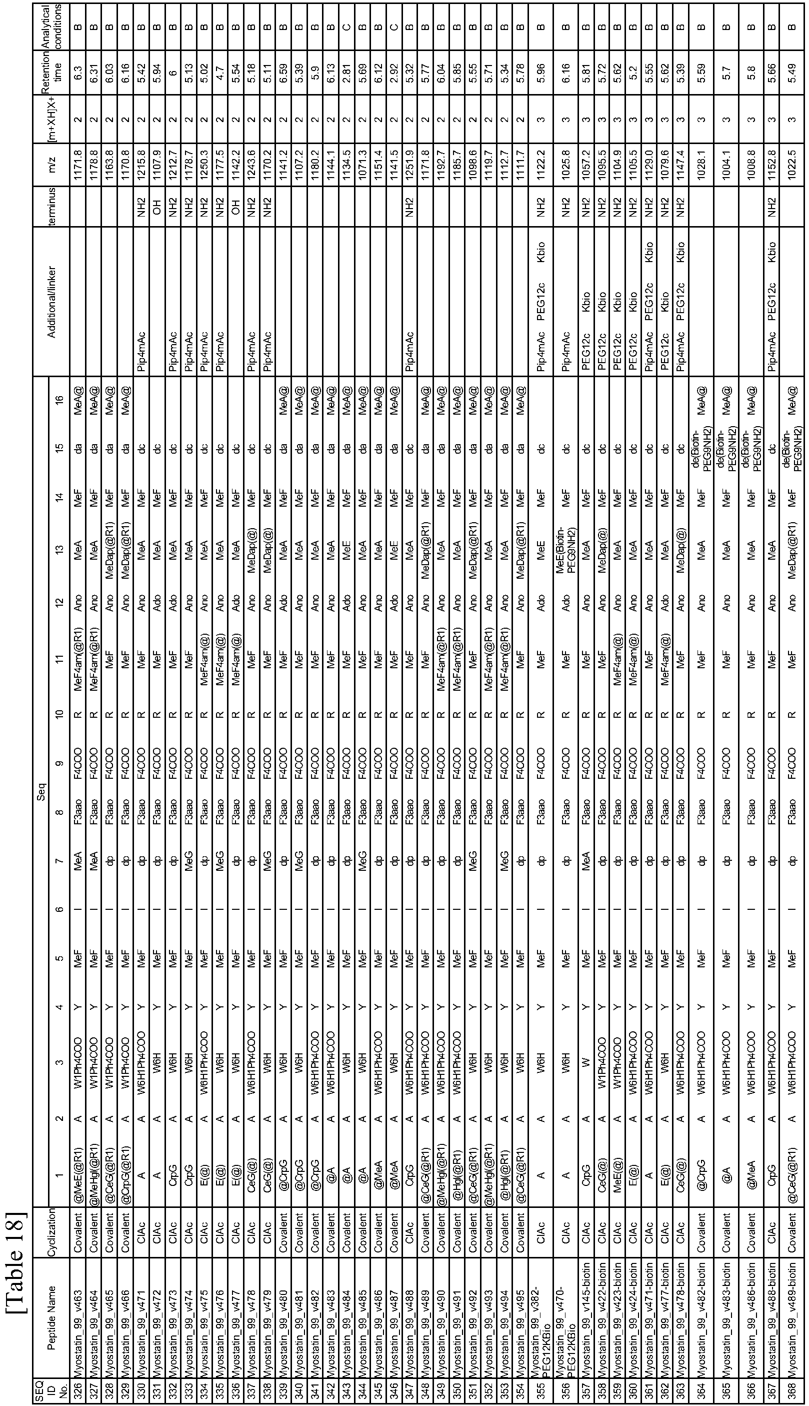

Table 18 lists the target peptides, analytical conditions, and retention times, and ESI-MS(+) observed values. The "terminus" indicates the C-terminal functional group (in the tables, "-OH" or "OH" indicates COOH and "-NH2" or "NH2" indicates CO(NH₂)), and the blank case indicates that no functional group is present at the C-terminus. With respect to "Cyclization" in the tables, entries with the description "ClAc" represent that cyclization was achieved by linking the amino acid at position 1 into which a chloroacetyl group was introduced with Cys present at the C-terminus, whereas the description "covalent" indicates that the N-terminal amino group of the amino acid at position 1 is linked to the C-terminal carboxyl group of the amino acid at position 15 or 16. In addition, the "Additional/Linker" in the tables indicates a linker or additional amino acid(s) that is not included in the cyclic structure. For example, in Myostatin_99_v31 (SEQ ID NO: 4), the amino acid sequence X1-X15 forms a cyclic structure, and an amino acid sequence G-de-G-dk is further added as a linker from the dc of the C-terminal X15.

In the peptides of SEQ ID NOs: 326-329, 334-338, 348-354, 358-360, 362-366, and 368, the side chains of amino acid residues with (@), (@R1) indicate that the side chains of the amino acid residues marked with (@) and (@R1) are bonded together to form a crosslinking structure, and the N-terminal amino group and the C-terminal carboxy group of the amino acid residues marked with @ are bonded together to form a cyclic structure. For example, in Myostatin_99_v463 (SEQ ID NO: 326), the N-terminal amino group (@MeE) of MeE at position 1 is bonded to the C-terminal carboxy group (MeA@) of MeA at position 16 to form a cyclic structure, and the side-chain carboxy group (MeE(@R1)) of the MeE at position 1 is bonded to the side-chain amide group (MeF4am(@R1)) of MeF4am at position 11 to form a crosslinking structure. In addition, in Myostatin_99_v475 (SEQ ID NO: 334), E at position 1 and dc at position 15 are bonded to form a cyclic structure, and the side-chain amide group (E(@)) of the E at position 1 and the side-chain amide group (MeF4am(@)) of MeF4am at position 11 are also bonded to form a crosslinking structure.

Further, the following describes SEQ ID NOs: 355-368. In Myostatin_99_v382-PEG12KBio (SEQ ID NO: 355), A at position 1 is bonded to dc at position 15 to form a cyclic structure; an additional linker (Additional/Linker), namely Pip4 mcAc-PEG12c-K is attached from the side chain of the dc at position 15; further, the carboxy group of biotin is attached to the side chain terminal NH₂ of the terminal K; and furthermore, the OH of the carboxy group of the terminal K is replaced by NH₂ (CONH₂).

### Example 12: Evaluation of In Vitro Activity by Using Reporter Gene Assay for Myostatin Signaling in HepG2 Cell Line

In this Example, the *in vitro* myostatin inhibitory effects of the peptides synthesized in Examples 10 and 11 were evaluated according to the Evaluation Procedure (1) in Example 3.

Table 19 shows the results.

**[Table 19]**

| SEQ ID No. | Peptide Name | IC50 (Luciferase) Myostatin HepG2 (nM) | IC50 (Luciferase) GDP-11 HepG2 (nM) | Selectivity | Method |
|---|---|---|---|---|---|
| 326 | Myostatin_99_v463 | 0.05 | 2.15 | 39.4 | D |
| 327 | Myostatin_99_v464 | 0.04 | 2.99 | 82.1 | D |
| 328 | Myostatin_99_v465 | 0.03 | 31.02 | 1121.1 | D |
| 329 | Myostatin_99_v466 | 0.07 | 32.66 | 501.9 | D |
| 330 | Myostatin_99_v471 | 0.09 | 15.24 | 169.1 | D |
| 331 | Myostatin_99_v472 | 1.51 | 83.83 | 55.4 | D |
| 332 | Myostatin_99_v473 | 0.37 | 108.80 | 290.7 | D |
| 333 | Myostatin_99_v474 | 1.23 | 120.30 | 98.1 | D |
| 334 | Myostatin_99_v475 | 0.04 | 1.78 | 44.9 | D |
| 335 | Myostatin_99_v476 | 0.22 | 25.31 | 114.3 | D |
| 336 | Myostatin_99_v477 | 0.14 | 25.69 | 181.8 | D |
| 337 | Myostatin_99_v478 | 0.04 | 25.52 | 575.7 | D |
| 338 | Myostatin_99_v479 | 0.88 | 47.49 | 54.1 | D |
| 339 | Myostatin_99_v480 | 0.36 | 172.20 | 472.7 | D |
| 340 | Myostatin_99_v481 | 0.58 | 64.04 | 110.8 | D |
| 341 | Myostatin_99_v482 | 0.11 | 90.17 | 814.5 | D |
| 342 | Myostatin_99_v483 | 0.14 | 59.94 | 424.2 | D |
| 343 | Myostatin_99_v484 | 0.43 | 240.40 | 560.2 | D |
| 344 | Myostatin_99_v485 | 2.11 | 123.90 | 58.6 | D |
| 345 | Myostatin_99_v486 | 0.14 | 70.82 | 516.6 | D |
| 346 | Myostatin_99_v487 | 0.48 | 123.90 | 259.9 | D |
| 347 | Myostatin_99_v488 | 0.18 | 53.16 | 294.4 | D |
| 348 | Myostatin_99_v489 | 0.12 | 45.41 | 365.6 | D |
| 349 | Myostatin_99_v490 | 0.70 | 112.00 | 160.4 | D |
| 350 | Myostatin_99_v491 | 0.77 | 82.22 | 106.9 | D |
| 351 | Myostatin_99_v492 | 0.42 | 78.61 | 187.6 | D |
| 352 | Myostatin_99_v493 | 0.46 | 84.09 | 184.0 | D |
| 353 | Myostatin_99_v494 | 0.46 | 63.86 | 139.7 | D |
| 354 | Myostatin_99_v495 | 0.32 | 246.60 | 774.3 | D |

Table 19 has demonstrated that all peptides examined in this Example exhibited inhibitory activity against myostatin. The GDF-11 inhibitory activity of the peptides examined in this Example was lower than the myostatin inhibitory activity.

### Reference Examples: To Synthesize Novel Non-Natural Amino Acid

The Reference Examples show examples of synthesis of various non-natural amino acids.

### Reference Example 1: Synthesis of Fmoc-W1Ph4COO(tBu)-OH

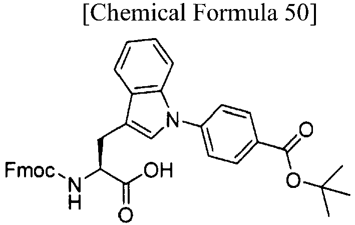

First, tert-butyl 4-iodobenzoate (25.0 g, 82.2 mmol; CAS: 120363-13-5) was dissolved in DMF (82 mL), and 1H-indole (10.6 g, 90.4 mmol; CAS: 120-72-9), copper(I) iodide (3.1 g, 16.4 mmol; CAS: 7681-65-4), and cesium carbonate (40.3 g, 123 mmol; CAS: 534-17-8) were added at room temperature. The mixture was stirred at 120°C for 1 hour. The mixture was diluted with 0.1 M hydrochloric acid aqueous solution and extracted twice with ethyl acetate. The combined organic layer was dried over sodium sulfate, filtered, and then concentrated under reduced pressure. The resulting residue was purified by silica gel column chromatography (petroleum ether/ethyl acetate = 100/50 to 0/100).

At room temperature, a portion of the resulting product (20.5 g, 69.9 mmol) was dissolved in DMF (233 mL), and N-iodosuccinimide (18.9 g, 83.9 mmol; CAS: 516-12-1) was added. The mixture was stirred for 10 min. The reaction mixture was cooled on ice, admixed with an aqueous sodium thiosulfate solution, and extracted twice with ethyl acetate. The combined organic layer was washed with saturated sodium bicarbonate water and saturated brine, dried over sodium sulfate, filtered, and then concentrated under reduced pressure. The resulting residue was purified by silica gel column chromatography (petroleum ether/ethyl acetate = 90/10 to 80/20) to afford tert-butyl 4-(3-iodo-1H-indol-1-yl)-benzoate.

At room temperature, zinc (1.30 g, 19.9 mmol; CAS: 7440-66-6) was suspended in DMF (13 mL), and 1,2-dibromoethane (0.057 mL, 0.665 mmol; CAS: 106-93-4) and trimethylsilyl chloride (0.088 mL, 0.665 mmol; CAS: 75-77-4) were added. The mixture was stirred at 50°C for 30 min. After the reaction mixture was cooled to room temperature, methyl (R)-2--((((9H-fluoren-9-yl)methoxy)carbonyl)amino)-3-iodopropanoate (3.00 g, 6.65 mmol; CAS: 156017-42-4) was added. The mixture was stirred at room temperature for 1 hour to prepare an organozinc reagent.

To a DMF solution (13 mL) containing tert-butyl 4-(3-iodo-1H-indol-1-yl)-benzoate (2.79 g, 6.65 mmol) were added Pd₂(dba)₃·CHCl₃ (0.344 g, 0.332 mmol; CAS: 52522-40-4) and SPhos (0.546 g, 1.33 mmol; CAS: 657408-07-6) at room temperature. The prepared organozinc reagent was then added. After stirring at 60°C for 2 hours, the reaction mixture was diluted with ethyl acetate, and saturated sodium bicarbonate water was added at 0°C. After the solid was filtered, the filtrate was extracted twice with ethyl acetate. The combined organic layer was dried over sodium sulfate, filtered, and then concentrated under reduced pressure. The resulting residue was purified by silica gel column chromatography (petroleum ether/ethyl acetate = 97/3 to 20/80).

A portion of the product obtained (2.01 g, 3.26 mmol) was dissolved in isopropanol (48.9 mL), and calcium chloride (5.79 g, 52.1 mmol; CAS: 10043-52-4) cooled on ice was added. Next, an aqueous solution (16.3 mL) containing lithium hydroxide (0.312 g, 13.0 mmol; CAS: 1310-65-2) was added dropwise. After completion of the dropping, the reaction mixture was stirred at room temperature for 16 hours. The reaction mixture was cooled to 0°C, adjusted to pH = 4 by adding 1 M hydrochloric acid aqueous solution, and extracted with ethyl acetate. The combined organic layer was washed with saturated brine, dried over sodium sulfate, filtered, and then concentrated under reduced pressure. The resulting residue was purified by silica gel column chromatography (dichloromethane/methanol = 95/5 to 80/20). The title substance was thus obtained.

ESI-MS(+): observed m/z = 603.3 (M+H)⁺

### Reference Example 2: Synthesis of Fmoc-W1Ph4OMe-OH

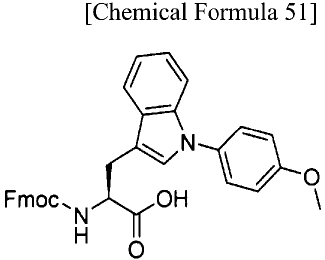

W1Ph4OMe was obtained by substantially the same synthetic procedure as for W1Ph4COO(tBu) in Reference Example 1. However, 1-iodo-4-methoxybenzene was used as a starting material instead of *tert*-butyl 4-iodobenzoate.

ESI-MS(+): observed m/z = 533.2 (M+H)⁺

### Reference Example 3: Synthesis of Fmoc-Hgl-OtBu

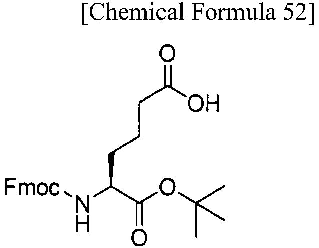

First, (S)-2-((((9H-fuoren-9-yl)methoxy)carbonyl)amino)-6-(allyloxy)-6-oxohexanoic acid (2.00 g, 4.72 mmol; CAS: 133464-45-6) was dissolved in dichloromethane (47.2 mL) and THF (4.72 mL), and *tert*-butyl 2,2,2-trichloroacetimidate (2.06 g, 9.45 mmol; CAS: 98946-18-0) was added at room temperature. The reaction mixture was stirred at room temperature for 12 hours, and then stirred at 45°C for 20 hours. After the reaction mixture was concentrated under reduced pressure, the solid was filtered and washed with dichloromethane (50 mL). The filtrate was concentrated under reduced pressure, and the resulting residue was then purified by silica gel column chromatography (petroleum ether/ethyl acetate = 80/20).

The resulting 6-allyl-1-(*tert*-butyl)(S)-2-((((9H-fluoren-9-yl)methoxy)carbonyl)amino)hexanedioate (2.26 g, 4.72 mmol) was dissolved in dichloromethane (23.6 mL) and THF (23.6 mL). Next, phenylsilane (1.16 mL, 9.44 mmol; CAS: 694-53-1) and tetrakis(triphenylphosphine)palladium (0.273 g, 0.236 mmol; CAS: 14221- 01-3) were added at 0°C.

The reaction mixture was stirred at 0°C for 1 hour, and then stirred at room temperature for 24 hours. The reaction mixture was concentrated under reduced pressure, and the resulting residue was then purified by silica gel column chromatography (petroleum ether/ethyl acetate = 100/0 to 80/20 to 30/70). The title substance was thus obtained.

ESI-MS(+): observed m/z = 384.3 (M+H-C4H8)⁺

### Reference Example 4: Synthesis of Fmoc-dhgl-OtBu

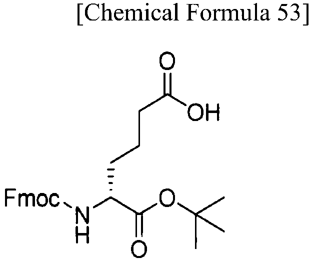

First, D-α-aminoadipic acid (CAS: 7620-28-2) was used as a starting material, and the procedure described in Angew. Chem. Int. Ed., 2016, 55, 1192-1195 was used to afford (R)-2-((((9H-fluoren-9-yl)methoxy)carbonyl)amino)-6-(allyloxy)-6-oxohexanoic acid.

The synthesis of dhgl-O(tBu) was carried out in substantially the same manner as that of Hgl-O(tBu). However, instead of (S)-2-((((9H-fluoren-9-yl)methoxy)carbonyl)amino)-6-(allyloxy)-6-oxohexanoic acid, (R)-2-(9H-fluoren-9-yl)methoxy)carbonyl)amino)-6-(allyloxy)-6-oxohexanoic acid obtained above was used.

ESI-MS(+): observed m/z = 384.1 (M+H-C4H8)⁺

### Reference Example 5: Synthesis of Fmoc-MeHgl-OtBu

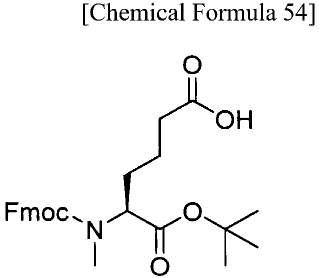

MeHgl-O(tBu) was obtained by substantially the same synthetic procedure as of Hgl-O(tBu). However, instead of (S)-2-((((9H-fluoren-9-yl)methoxy)carbonyl)amino)-6-(allyloxy)-6-oxohexanoic acid, (S)-2-(9H-fluoren-9-yl)methoxy)carbonyl)(methyl)amino)-6-(allyloxy)-6-oxohexanoic acid was used as a starting material.

ESI-MS(+): observed m/z = 398.3 (M+H-C4H8)⁺

### Reference Example 6: Synthesis of Fmoc-Medhgl-OtBu

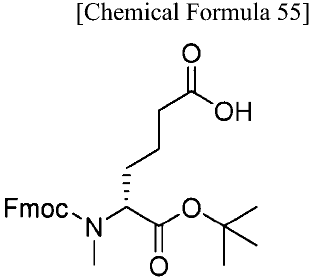

The procedure described in US18/109702 was used to afford (R)-2-((((9H-fluoren-9-yl)methoxy)carbonyl)(methyl)amino)-6-(allyloxy)-6-oxohexanoic acid. However, instead of (S)-2-((((9H-fluoren-9-yl)methoxy)carbonyl)amino)-6-(allyloxy)-6-oxohexanoic acid, (R)-2-(9H-fluoren-9-yl)methoxy)carbonyl)amino)-6-(allyloxy)-6-oxohexanoic acid was used as a starting material.

Medhgl-O(tBu) was obtained by substantially the same synthetic procedure as of Hgl-O(tBu). However, instead of (S)-2-((((9H-fluoren-9-yl)methoxy)carbonyl)amino)-6-(allyloxy)-6-oxohexanoic acid, (R)-2-(9H-fluoren-9-yl)methoxy)carbonyl)(methyl)amino)-6-(allyloxy)-6-oxohexanoic acid obtained above was used.

ESI-MS(+): observed m/z = 398.1 (M+H-C4H8)

### Reference Example 7: Synthesis of Fmoc-W6H(PMB)1Ph4COO(tBu)-OH

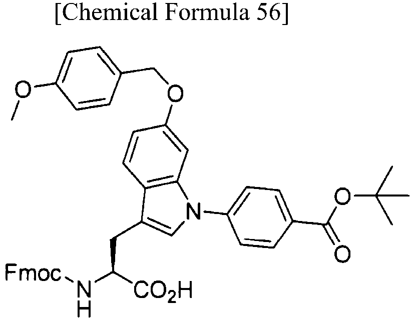

To a DMF solution (225 mL) containing 1H-indole-6-ol (15.0 g, 113 mmol; CAS: 2380-86-1) were added cesium carbonate (73.4 g, 225 mmol; CAS: 534-17-8) and 4-methoxybenzylchloride (16.9 mL, 124 mmol; CAS: 824-94-2). The mixture was stirred at room temperature for 24 hours. The reaction solution was diluted with ethyl acetate and saturated brine, and the solid was filtered. The filtrate was extracted twice with ethyl acetate. The combined organic layer was washed three times with saturated brine and dried over sodium sulfate. After filtration and concentration under reduced pressure, the obtained solid was suspended in dichloromethane and filtered out.

To a DMF solution (13 mL) containing a portion of the obtained product (3.7 g, 14.5 mmol) were added 4-iodobenzoic acid tert-butyl ester (4.0 g, 13.2 mmol; CAS: 120363-13-5), cesium carbonate (6.5 g, 19.8 mmol), and copper iodide (0.50 g, 2.6 mmol; CAS: 7681-65-4). The mixture was stirred at 120°C for 7 hours. The reaction solution was diluted with ethyl acetate and saturated brine, and filtered through Celite. The filtrate was extracted with ethyl acetate, washed twice with saturated brine, and dried over sodium sulfate. After filtration and concentration under reduced pressure, the resulting residue was purified by silica gel column chromatography (heptane/ethyl acetate = 97/3 to 80/20).

A portion of the product obtained (5.6 g, 12.9 mmol) was dissolved in DMF (431 mL), and N-iodosuccinimide (2.9 g, 12.8 mmol; CAS: 516-12-1) was added. The mixture was stirred at room temperature for 3 hours. The reaction solution was cooled on ice, quenched with a sodium thiosulfate aqueous solution, and extracted with ethyl acetate. The resulting organic layer was washed sequentially with saturated sodium bicarbonate water and saturated brine, and dried over sodium sulfate. The resulting material was filtered and concentrated under reduced pressure. The resulting residue was purified by silica gel column chromatography (heptane/ethyl acetate = 90/10 to 50/50) to afford 4-(3-iodo-6-((4-methoxybenzyl)oxy)-1H-indol-1-yl)benzoic acid *tert*-butyl ester.

At room temperature, zinc (2.0 g, 31 mmol; CAS: 7440-66-6) was suspended in DMF (26 mL), and iodine (2.6 g, 10.4 mmol; CAS: 7790-99-0) was added. The mixture was stirred at room temperature for 20 min. To the reaction solution was added methyl (R)-2-((((9H-fluoren-9-yl)methoxy)carbonyl)amino)-3-iodopropanoate (5.6 g, 12.5 mmol; CAS: 156017-42-4). The mixture was stirred at room temperature for 3 hours to prepare an organozinc reagent.

To a DMF solution (26 mL) containing 4-(3-iodo-6-((4-methoxybenzyl)oxy)-1H-indol-1-yl)benzoic acid *tert*-butyl ester (5.7 g, 10.4 mmol) were added Pd₂(dba)₃·CHCl₃ (0.54 g, 0.52 mmol; CAS: 52522-40-4) and SPhos (0.85 g, 2.1 mmol; CAS: 657408-07-6) at room temperature. The prepared organozinc reagent was then added. The reaction solution was stirred at 70°C for 3 hours. The reaction solution was diluted with ethyl acetate and saturated brine, and filtered through Celite. The filtrate was extracted with ethyl acetate. The resulting organic layer was washed sequentially with saturated sodium bicarbonate water and saturated brine, and dried over sodium sulfate. After filtration and concentration under reduced pressure, the resulting residue was purified by silica gel column chromatography (hexane/ethyl acetate = 85/15 to 45/55).

A portion of the product obtained (5.0 g, 6.7 mmol) was dissolved in tetrahydrofuran (34 mL) and isopropanol (16.7 mL), and an aqueous solution (11.7 mL) containing calcium chloride (11.9 g, 107 mmol; CAS: 10043-52-4) was added under ice cold conditions. An aqueous solution (5.0 mL) containing lithium hydroxide (0.64 g, 26.8 mmol; CAS: 1310-65-2) was then added dropwise. After completion of the dropping, the reaction mixture was stirred at room temperature for 2 days. The pH was adjusted to 3 by adding 1 M hydrochloric acid. The reaction solution was extracted with ethyl acetate, and the resulting organic layer was washed with saturated brine and dried over sodium sulfate. After filtration and concentration under reduced pressure, the resulting residue was purified by silica gel column chromatography (heptane/ethyl acetate = 65/35 to 0/100, then dichloromethane/methanol = 100/0 to 90/10). Fractions containing the target product were concentrated and then purified by silica gel column chromatography (ethyl acetate/methanol = 100/0 to 90/10). The title substance was thus obtained.

ESI-MS(+): observed m/z = 739.3 (M+H)⁺

### Reference Example 8: Synthesis of Fmoc-W7Ph3C-OH

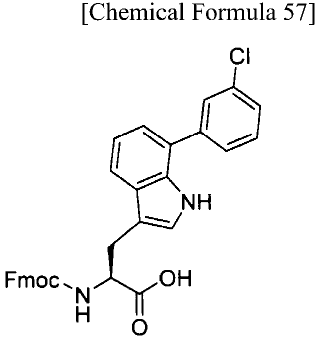

First, 7-bromo-1H-indole (19.6 g, 100 mmol; CAS: 51417-51-7), (3-chlorophenyl)boronic acid (18.8 g, 120 mmol; CAS: 63503-60-6), Pd(PPh₃)₄ (5.8 g, 5.0 mmol; CAS: 14221-01-3), and tripotassium phosphate (63.7 g, 300 mmol; CAS: 7778-53-2) were dissolved in 1,4-dioxane (450 mL) and water (50 mL). The mixture was stirred at 100°C for 75 min. The reaction solution was diluted with water and extracted twice with ethyl acetate. The combined organic layer was washed with water and saturated brine and then dried over sodium sulfate. After filtration and concentration under reduced pressure, the resulting residue was purified by silica gel column chromatography (hexane/ethyl acetate = 90/10 to 70/30).

To a DMF solution (80 mL) containing a portion of the product obtained (6.8 g, 30 mmol) were added potassium hydroxide (4.2 g, 75 mmol) and then a DMF solution (20 mL) containing iodine (7.6 g, 30 mmol; CAS: 7553-56-2) cooled on ice. The mixture was stirred at room temperature for 30 min. The reaction solution was diluted with ethyl acetate, washed with saturated brine, and dried over sodium sulfate. After filtration and concentration under reduced pressure, the resulting residue was purified by silica gel column chromatography (heptane/ethyl acetate = 90/10 to80/20) to afford 7-(3-chlorophenyl)-3-iodo-1H-indole.

At room temperature, zinc (3.9 g, 60 mmol) was suspended in DMF (50 mL), and iodine (1.5 g, 6.0 mmol) was added. The mixture was then stirred at 60°C for 30 min. To the reaction solution was added methyl (R)-2-((((9H-fluoren-9-yl)methoxy)carbonyl)amino)-3-iodopropanoate (10.8 g, 24.0 mmol; CAS: 156017-42-4). The mixture was stirred at room temperature for 90 min to prepare an organozinc reagent.

Next, 7-(3-chlorophenyl)-3-iodo-1H-indole (7.1 g, 20 mmol), Pd₂(dba)₃·CHCl₃ (1.0 g, 1.0 mmol; CAS: 52522-40-4), and SPhos (1.6 g, 4.0 mmol; CAS: 657408-07-6) were added at room temperature. The mixture was stirred at 60°C for 30 min. The prepared organozinc reagent was then added, and the mixture was stirred at 60°C for 1 hour. The reaction solution was diluted with ethyl acetate and quenched by adding 0.5 M hydrochloric acid under ice-cold conditions. After stirring for 5 min under ice-cold conditions, the mixture was filtered through Celite and washed with ethyl acetate. The filtrate was extracted with ethyl acetate, and the resulting organic layer was subsequently washed with saturated brine and then dried over sodium sulfate. After filtration and concentration under reduced pressure, the resulting material was purified by silica gel column chromatography (hexane/ethyl acetate = 90/10 to 40/60).

A portion of the resulting product (4.4 g, 8.0 mmol) was dissolved in isopropanol (120 mL), calcium chloride (14.2 g, 128 mmol; CAS: 10043-52-4) was added at 0°C, and an aqueous solution (40 mL) containing lithium hydroxide (0.77 g, 31.9 mmol; CAS: 1310-65-2) was added dropwise. After completion of the dropping, the reaction mixture was stirred at room temperature for 30 min. The reaction mixture was cooled to 0°C, and adjusted to pH = 3 to 4 by adding 1 M hydrochloric acid (30 mL). The reaction solution was extracted three times with ethyl acetate. The combined organic layer was washed with water and then saturated brine, and dried over sodium sulfate. After filtration and concentration under reduced pressure, the resulting residue was purified by silica gel column chromatography (heptane/ethyl acetate = 30/70 to 80/20). The title substance was thus obtained.

ESI-MS(+): observed m/z = 537.2 (M+H)⁺

### Reference Example 9: Synthesis of Fmoc-pBph2aao(tBu)-OH

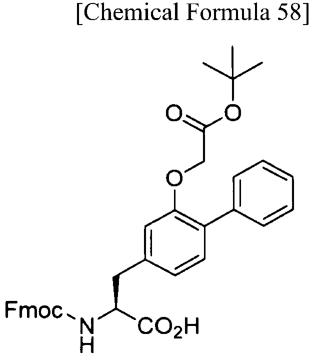

First, 4-bromo-[1,1'-biphenyl]-2-ol (4.5 g, 18.0 mmol; CAS: 1679324 -76-5) was dissolved in acetone (90 mL), and cesium carbonate (8.8 g, 27.0 mmol; CAS: 534-17-8) and tert-butyl 2-bromoacetate (4.0 mL, 27.0 mmol; CAS: 5292-43-3) were added. The mixture was then stirred at room temperature for 19 hours. The mixture was concentrated under reduced pressure and quenched with saturated sodium bicarbonate water. The mixture was extracted with ethyl acetate and washed with saturated brine. The resulting material was dried over sodium sulfate, filtered, and then concentrated under reduced pressure. The resulting residue was purified by silica gel column chromatography (heptane/ethyl acetate = 97/3 to 90/10) to afford *tert*-butyl 2-((4-bromo-[1,1'-biphenyl]-2-yl)oxy)acetate.

At room temperature, zinc (3.6 g, 55 mmol) was suspended in DMF (45 mL), and iodine (4.6 g, 18.2 mmol) was added. The mixture was then stirred at room temperature for 30 min. To the reaction solution was added methyl (R)-2-((((9H-fluoren-9-yl)methoxy)carbonyl)amino)-3-iodopropanoate (5.6 g, 12.5 mmol; CAS: 156017-42-4). The mixture was stirred at room temperature for 3.5 hours to prepare an organozinc reagent. To a DMF solution (45 mL) containing *tert*-butyl 2-((4-bromo-[1,1'-biphenyl]-2-yl)oxy)acetate (6.6 g, 18.2 mmol) were added Pd₂(dba)₃·CHCl₃ (0.94 g, 0.91 mmol; CAS: 52522-40-4) and SPhos (1.5 g, 3.6 mmol; CAS: 657408-07-6) at room temperature. The prepared organozinc reagent was then added. The reaction solution was stirred at 70°C for 3 hours. The reaction solution was diluted with ethyl acetate and saturated brine, and filtered through Celite. The filtrate was extracted with ethyl acetate. The resulting organic layer was washed sequentially with saturated sodium bicarbonate water and saturated brine, and dried over sodium sulfate. After filtration and concentration under reduced pressure, the resulting residue was purified by silica gel column chromatography (hexane/ethyl acetate = 90/10 to 40/60).

A portion of the product obtained (2.9 g, 4.7 mmol) was dissolved in tetrahydrofuran (24 mL) and isopropanol (11.9 mL), and an aqueous solution (8.3 mL) containing calcium chloride (6.3 g, 56.9 mmol; CAS: 10043-52-4) was added under ice cold conditions. An aqueous solution (3.6 mL) containing lithium hydroxide (0.34 g, 14.2 mmol; CAS: 1310-65-2) was then added dropwise. After completion of the dropping, the reaction mixture was stirred at room temperature for 16 hours. The pH was adjusted to 3 by adding 1 M hydrochloric acid. The reaction solution was extracted with ethyl acetate, and the resulting organic layer was washed with saturated brine and dried over sodium sulfate. After filtration and concentration under reduced pressure, the resulting residue was purified by silica gel column chromatography (heptane/ethyl acetate = 80/20 to 60/40, then dichloromethane/methanol = 95/5 to 80/20). The title substance was thus obtained.

ESI-MS(+): observed m/z = 538.3 (M+H)⁺

### Reference Example 10: Synthesis of Fmoc-W6H(Boc)1Ph4COO(tBu)-OH

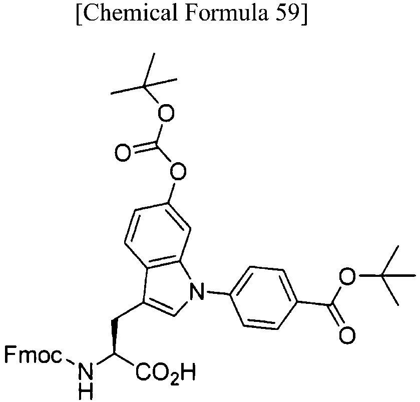

To a DMSO solution (700 mL) containing 1H-indole-6-ol (35.0 g, 263 mmol; CAS: 2380-86-1) were added *tert*-butyl 4-iodobenzoate (88 g, 289 mmol; CAS: 120363-13-5), copper(I) bromide (1.9 g, 13.1 mmol; CAS: 7787-70 -4), potassium carbonate (109 g, 789 mmol; CAS: 534-17-8), α-D-galacturonic acid hydrate (5.6 g, 26.3 mmol; CAS: 91510-62-2) at room temperature. The mixture was then stirred at 100°C overnight. The mixture was filtered and the solid was washed with ethyl acetate. The filtrate was combined and washed with saturated ammonium chloride aqueous solution and saturated brine. The organic layer was dried over sodium sulfate, filtered, and then concentrated under reduced pressure.

The resulting residue was purified by silica gel column chromatography (petroleum ether/ethyl acetate = 80/20). A portion of the resulting product (13.7 g, 44.3 mmol) was dissolved in acetonitrile (140 mL), and DMAP (0.54 g, 4.43 mmol) and Boc2O (9.7 g, 44.3 mmol) were then added. The mixture was stirred at room temperature for 2 hours. The reaction was quenched with water and extracted with ethyl acetate. The combined organic layer was washed with saturated brine. The organic layer was dried over sodium sulfate, filtered, and then concentrated under reduced pressure.

The resulting residue was purified by silica gel column chromatography (petroleum ether/ethyl acetate = 83/17). A portion of the resulting mixture (16.2 g, 39.6 mmol) was dissolved in DMF (160 mL), and N-iodosuccinimide (9.8 g, 43.5 mmol; CAS: 516-12-1) was added under ice-cold conditions. The mixture was then stirred for 2 hours. The mixture was quenched with saturated sodium bicarbonate water under ice-cold conditions and then diluted with water. The resulting mixture was extracted with ethyl acetate. The combined organic layer was washed with saturated brine. The organic layer was dried over sodium sulfate, filtered, and then concentrated under reduced pressure. The resulting residue was purified by silica gel column chromatography (petroleum ether/ethyl acetate = 95/5) to afford *tert*-butyl 4-(6-((*tert*-butoxycarbonyl)oxy)-3-iodoindol-1-yl-benzoate.

At room temperature, zinc (7.0 g, 106 mmol; CAS: 7440-66-6) was suspended in DMF (400 mL), and iodine (2.7 g, 10.6 mmol; CAS: 7790-99-0) was added. The mixture was stirred at room temperature for 20 min. To the reaction solution was added methyl (R)-2-((((9H-fluoren-9-yl)methoxy)carbonyl)amino)-3-iodopropanoate (19 g, 42.6 mmol; CAS: 156017-42-4). The mixture was stirred at room temperature for 1 hour.

To the resulting mixture were added *tert*-butyl 4-(6-((*tert*-butoxycarbonyl)oxy)-3-iodoindol-1-yl-benzoate (19 g, 36 mmol), Pd₂(dba)₃·CHCl₃ (1.82 g, 1.8 mmol; CAS: 52522-40-4), and SPhos (2.91 g, 7.1 mmol; CAS: 657408-07-6) at room temperature. The mixture was then stirred at 50°C for 3 hours. The reaction solution was brought to room temperature and the reaction was quenched with water. The mixture was filtered and the solid was washed with ethyl acetate.

The resulting filtrate was combined and extracted with ethyl acetate. The combined organic layer was washed with saturated brine and dried over sodium sulfate. After filtration and concentration under reduced pressure, the resulting residue was purified by silica gel column chromatography (petroleum ether/ethyl acetate = 67/33).

A mixture containing a portion of the product obtained and another lot (40 g, 55 mmol) was dissolved in isopropanol (900 mL), and an aqueous solution (240 mL) containing calcium chloride (96.9 g, 873 mmol; CAS: 10043-52-4) was added under ice-cold conditions. An aqueous solution (60 mL) containing lithium hydroxide (5.24 g, 218 mmol; CAS: 1310-65-2) was then added dropwise. After completion of the dropping, the reaction mixture was stirred at room temperature overnight. A sodium dihydrogen phosphate aqueous solution was added and the pH was adjusted to 4. The reaction solution was extracted with ethyl acetate, and the combined organic layer was washed with saturated brine and then dried over sodium sulfate. After filtration and concentration under reduced pressure, the resulting residue was purified by reversed-phase silica gel column chromatography (C18; gradient: H₂O containing 10 nM ammonium bicarbonate/acetonitrile = 95/5 to 0/100 over 40 min). The title substance was thus obtained.

ESI-MS(+): observed m/z = 741.4 (M+Na)⁺

### Reference Example 11: Synthesis of Fmoc-W6H(Boc,Boc)-OH

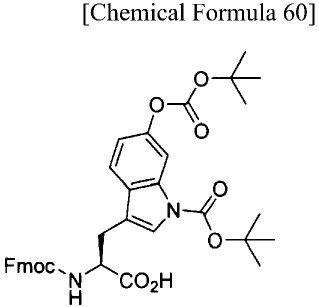

To a DMF solution (65 mL) containing tert-butyl(1H-indol-6-yl)carbonate (4.6 g, 19.5 mmol; CAS: 873779-75-0) was added N-iodosuccinimide (4.4 g, 19.5 mmol; CAS: 516-12-1). The mixture was then stirred at 25°C for 2 hours. The mixture was cooled on ice, quenched with a sodium thiosulfate aqueous solution, and extracted twice with ethyl acetate. The combined organic layer was washed sequentially with aqueous sodium bicarbonate and saturated brine, dried over sodium sulfate, filtered, and then concentrated under reduced pressure. The resulting residue was purified by column chromatography (20% dichloromethane-containing heptane/20% dichloromethane-containing ethyl acetate = 100/0 to 70/30).

To a DMF solution (48 mL) containing a portion of the obtained product (5.2 g, 14.5 mmol) were added di-*tert*-butyl bicarbonate (3.8 g, 17.3 mmol; CAS: 24424-99-5) and 4-dimethylaminopyridine (0.18 g, 1.45 mmol; CAS: 1122-58-3). The mixture was then stirred at 25°C for 3 hours. The mixture was diluted with saturated brine and extracted with ethyl acetate. The organic layer was washed with saturated brine, dried over sodium sulfate, filtered, and then concentrated under reduced pressure. The resulting residue was purified by column chromatography (heptane/ethyl acetate = 95/5 to 80/20) to afford *tert*-butyl-6-((*tert*-butoxycarbonyl)oxy)-3-iodo-1H-indol-1-carboxylate.

Zinc (2.9 g, 43.8 mmol; CAS: 7440-66-6) was suspended in DMF (37 mL), and iodine (3.7 g, 14.6 mmol; CAS: 7553-56-2) was added. The mixture was then stirred at 25°C for 30 min. To the suspension was added methyl (R)-2-((((9H-fluoren-9-yl)methoxy)carbonyl)amino)-3-iodopropanoate (7.9 g, 17.5 mmol; CAS: 156017-42-4). The mixture was stirred at 25°C for 2 hours to prepare an organozinc reagent.

To a DMF solution containing tert-butyl-6-((*tert*-butoxycarbonyl)oxy)-3-iodo-1H-indol-1-carboxylate (6.7 g, 14.6 mmol) were added Pd₂(dba)₃·CHCl₃ (0.76 g, 0.73 mmol; CAS: 52522-40-4) and SPhos (1.2 g, 2.9 mmol; CAS: 657408-07-6) at 25°C. The prepared organozinc reagent was added and the mixture was then stirred at 70°C for 2 hours. The mixture was brought to room temperature, diluted with ethyl acetate and saturated brine, and filtered through Celite. The filtrate was extracted with ethyl acetate, and washed sequentially with aqueous sodium bicarbonate, and saturated brine. The organic layer was dried over sodium sulfate, filtered, and then concentrated under reduced pressure. The resulting residue was purified by column chromatography (heptane/ethyl acetate = 90/10 to 60/40).

To a THF/2-propanol solution (95 mL; 2/1) containing a portion of the product obtained (8.3 g, 12.7 mmol) was added an aqueous solution (22 mL) containing calcium chloride (16.9 g, 152 mmol; CAS: 10043-52-4) under ice cold conditions. An aqueous solution (9.5 mL) containing lithium hydroxide (0.91 g, 38.1 mmol; CAS: 1310-65-2) was added, and the mixture was then stirred at 25°C overnight. Next, 1 N hydrochloric acid (38 mL) was added to the reaction solution, and the mixture was extracted with ethyl acetate and then washed with saturated brine. The organic layer was dried over sodium sulfate, filtered, and then concentrated under reduced pressure. The resulting residue was purified by column chromatography (dichloromethane/methanol = 95/5 to 90/10). The title substance was thus obtained.

ESI-MS(+): observed m/z = 531.2 (M-C8H16+H)⁺

### Reference Example 12: Synthesis of Fmoc-dcaa-OtBu

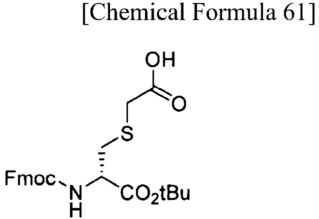

To a THF solution containing (((9H-fluoren-9-yl)methoxy)carbonyl)-D-cysteine (14.0 g, 40.8 mmol; CAS: 157355-80-1) were added an aqueous solution (102 mL) containing sodium bicarbonate (6.9 g, 82.0 mmol) and allyl 2-chloroacetate (5.2 mL, 44.9 mmol; CAS: 2916 -14-5) under ice-cold conditions. The mixture was then stirred at 25°C for 4 hours. The pH was adjusted to 4 by adding 1 M hydrochloric acid, and the mixture was extracted twice with ethyl acetate. The combined organic layer was washed with saturated brine, filtered, and then concentrated under reduced pressure.

The resulting residue was purified by silica gel column chromatography (heptane/ethyl acetate = 90/10 to 20/80). To a dichloromethane solution (102 mL) containing a portion of the product obtained (9.0 g, 20.4 mmol) was added O-*tert*-butyl-N,N'-diisopropylisourea (20.4 g, 102 mmol; CAS: 71432-55-8) under ice-cold conditions. The mixture was then stirred at 40°C for 6 hours. The resulting solid was filtered off and washed with ethyl acetate. The filtrate was washed twice with saturated sodium bicarbonate water, dried over magnesium sulfate, and concentrated.

The resulting residue was purified by column chromatography (hexane/ethyl acetate = 80/20 to 30/70). To a dichloromethane solution (44 mL) containing a portion of the product obtained (4.4 g, 8.8 mmol) were added phenylsilane (2.2 mL, 17.7 mmol) and Pd(PPh₃)₄ (2.0 g, 1.8 mmol) under ice-cold conditions. The mixture was then stirred at 25°C for 4 hours. The reaction solution was concentrated under reduced pressure, and was then purified by silica gel column chromatography (heptane/ethyl acetate = 90/10 to 40/60). The title substance was thus obtained.

ESI-MS(+): observed m/z = 402.0 (M-C4H8+H)⁺

## Claims

1. A peptide comprising the following amino acid sequence:
X1-X2-X3-X4-X5-X6-X7-X8-X9-X10-X11-X12-X13-X14-X15 (SEQ ID NO: 2); or a pharmaceutically acceptable salt thereof,
wherein
X1 is any amino acid or peptoid;
X2 is any amino acid;
X3 is an amino acid having an optionally substituted aromatic ring in a side chain;
X4 is an amino acid having an optionally substituted aromatic ring in a side chain;
X5 is an amino acid having an optionally substituted aromatic ring or optionally substituted cycloalkyl in a side chain;
X6 is an amino acid having an alkyl group in a side chain;
X7 is any secondary amino acid, N-alkylamino acid, or peptoid;
X8 is any amino acid;
X9 is an amino acid having an optionally substituted aromatic ring or an optionally substituted alkyl group or cycloalkyl group in a side chain;
X10 is R;
X11 is an amino acid having an optionally substituted aromatic ring or an optionally substituted alkyl group or cycloalkyl group in a side chain;
X12 is an optionally substituted aliphatic amino acid or an amino acid having an optionally substituted alkyl chain in a side chain;
X13 is any secondary amino acid, N-alkylamino acid, or peptoid;
X14 is an amino acid having an optionally substituted aromatic ring in a side chain; and
X15 is any amino acid.

2. The peptide or pharmaceutically acceptable salt thereof according to claim 1, wherein the following one or two or more requirements are satisfied:
X1 is an amino acid having an optionally substituted alkyl group in a side chain or a peptoid having an optionally substituted alkyl group in a side chain attached to amide nitrogen;
X3 is optionally substituted F or W;
X4 is optionally substituted F or Y, or naphthylalanine;
X5 is optionally substituted F, Y, or naphthylalanine, or optionally substituted cyclohexylalanine;
X6 is an amino acid having a branched alkyl group, or an amino acid having a cycloalkyl group, wherein a carbon atom contained therein is optionally replaced by an oxygen atom;
X8 is an amino acid having an optionally substituted aromatic ring or an optionally substituted alkyl group or cycloalkyl in a side chain;
X9 is optionally substituted F or Y, or an amino acid containing an optionally substituted heterocycloalkyl group in a side chain, or alanine;
X12 is an aliphatic amino acid containing three or more carbon atoms in a side chain, or an amino acid having an optionally substituted alkyl chain in a side chain;
X13 is an N-alkylamino acid;
X14 is optionally substituted F or Y; and
X15 is an amino acid having a sulfur (S) element at a side chain terminus.

3. The peptide or pharmaceutically acceptable salt thereof according to claim 1, wherein the following one or two or more requirements are satisfied:
X1 is an amino acid having an optionally substituted alkyl group in a side chain or a peptoid having an optionally substituted alkyl group in a side chain attached to amide nitrogen;
X3 is optionally substituted F or W;
X4 is optionally substituted F or Y, or naphthylalanine;
X5 is optionally substituted F, Y, or naphthylalanine, or optionally substituted cyclohexylalanine;
X6 is an amino acid having a branched alkyl group, or an amino acid having a cycloalkyl group, wherein a carbon atom contained therein is optionally replaced by an oxygen atom;
X8 is an amino acid having an optionally substituted aromatic ring or an optionally substituted alkyl group or cycloalkyl in a side chain;
X9 is optionally substituted F or Y, or an amino acid containing an optionally substituted heterocycloalkyl group in a side chain, or alanine;
X12 is an aliphatic amino acid containing three or more carbon atoms in a side chain, or an amino acid having an optionally substituted alkyl chain in a side chain;
X13 is an N-alkylamino acid;
X14 is optionally substituted F or Y; and
X15 is an aliphatic amino acid, and is further attached to an aliphatic amino acid or proline as X16.

4. The peptide or pharmaceutically acceptable salt thereof according to claim 1, wherein the following one or two or more requirements are satisfied:
X1 is A, MeA, Dap, ApG, CrpG, CmG, MeD, N, CrmG, MeopG, MeeG, AcapG, A4paa, KCOpipzaa, CeG, CrbG, K, KMe, Hly, E, Hgl, Orn, Dab, MeE, D, or MeHgl;
X2 is R, Dap, K, E, A, da, S, G, de, dkCOpipzaa, ddab, or dorn;
X3 is W, W7N, F44Py, Bph, W6N, F41dMeCmm4Pyz, W1Bn, W6H, W1Ph, Bph4ms, W1aa, W7Ph3C, W1Ph4OMe, W1Ph4COO, F42Py, or W6H1Ph4COO;
X4 is Y, 3Py6NH2, YBn, Yae, Nal1, Yph, or mBph;
X5 is MeF, MeY, MeF3Me, F, MeCha, MeYap, MeA1Ac4pip, MemBph, MeNal1, MeNal2, MeF4OMe, or MeF4C;
X6 is I, Gcpe, or TMe;
X7 is MeG, MeA, Meda, dp, EtG, or PrG;
X8 is Y, F4COO, K, 4Py, F, F3aao, F3OMe, A4paa, KCOpipzaa, Bph2C, Bph2OMe, or pBph2aao;
X9 is Y, F4COO, 3Py, 4Py, F4aao, F4CON, F3CON, F4F, or F4OMe;
X11 is MeF, Me4Py, Me3Py, F, Cha, MeCha, MeK, MeKMe, MeF4am, MeD, MeE, MeF4COO, or F4am;
X12 is L, Ahp, Ano, Ado, Ade, Aoc, SPent, HseBu, AhpOMe, HsePr, or Aun;
X13 is MeA, MeE, MeKCOpipzaa, MeK, MeDap, or MeDab;
X14 is MeF, MeF3Et, Me4Py, Me3Py, or MemBph; and
X15 is C, MeC, dc, Medc, dhcy, Hcy, or de.

5. The peptide or pharmaceutically acceptable salt thereof according to claim 1, wherein the following one or two or more requirements are satisfied:
X1 is A, MeA, Dap, ApG, CrpG, CmG, MeD, N, CrmG, MeopG, MeeG, AcapG, A4paa, KCOpipzaa, CeG, CrbG, K, KMe, Hly, E, Hgl, Orn, Dab, MeE, D, or MeHgl;
X2 is R, Dap, K, E, A, da, S, G, de, dkCOpipzaa, ddab, or dorn;
X3 is W, W7N, F44Py, Bph, W6N, F41dMeCmm4Pyz, W1Bn, W6H, W1Ph, Bph4ms, W1aa, W7Ph3C, W1Ph4OMe, F42Py, W1Ph4COO, or W6H1Ph4COO;
X4 is Y, 3Py6NH2, YBn, Yae, Nal1, Yph, or mBph;
X5 is MeF, MeY, MeF3Me, F, MeCha, MeYap, MeA1Ac4pip, MemBph, MeNal1, MeNal2, MeF4OMe, or MeF4C;
X6 is I, Gcpe, or TMe;
X7 is MeG, MeA, Meda, dp, EtG, or PrG;
X8 is Y, F4COO, K, 4Py, F, F3aao, F3OMe, A4paa, KCOpipzaa, Bph2C, Bph2OMe, or pBph2aao;
X9 is Y, F4COO, 3Py, 4Py, F4aao, F4CON, F3CON, F4F, or F4OMe;
X11 is MeF, Me4Py, Me3Py, F, Cha, MeCha, MeK, MeKMe, MeF4am, MeD, MeE, MeF4COO, or F4am;
X12 is L, Ahp, Ano, Ado, Ade, Aoc, SPent, HseBu, AhpOMe, HsePr, or Aun;
X13 is MeA, MeE, MeKCOpipzaa, MeK, MeDap, or MeDab;
X14 is MeF, MeF3Et, Me4Py, Me3Py, or MemBph; and
X15 is Aeoac, Ape, dhgl, Hgl, MeHgl, Medhgl, MeAeoac, or MeApe, or X15 is G, da, A, or de and is attached to MeA, Meda, G, da, MeG, P, or A as X16.

6. The peptide or pharmaceutically acceptable salt thereof according to claim 1, wherein the following one or two requirements are satisfied:
X1 is A, MeA, Dap, ApG, CrpG, CmG, MeD, N, CrmG, MeopG, MeeG, AcapG, A4paa, KCOpipzaa, CeG, CrbG, K, KMe, Hly, E, Hgl, Orn, Dab, MeE, D, or MeHgl; and
X15 is Aeoac, Ape, dhgl, Hgl, MeHgl, Medhgl, MeAeoac, or MeApe, or X15 is G, da, A, or de and is attached to MeA, Meda, G, da, MeG, P, or A as X16.

7. The peptide or pharmaceutically acceptable salt thereof according to claim 1, the peptide comprising or consisting of an amino acid sequence set forth in any one of SEQ ID NOs: 2 to 368.

8. A peptide comprising the following amino acid sequence:
A-R-W-Y-MeF-I-MeG-Y-Y-R-MeF-L-MeA-MeF-C (SEQ ID NO: 1)
or
an amino acid sequence having a substitution, addition, deletion, or insertion in 1 to 14 amino acid residues selected from the group consisting of amino acid residues at positions 1, 2, 3, 4, 5, 6, 7, 8, 9, 11, 12, 13, 14, and 15 in the above amino acid sequence; or a pharmaceutically acceptable salt thereof.

9. The peptide or pharmaceutically acceptable salt thereof according to any one of claims 1 to 8, wherein the peptide is a cyclic peptide.

10. The peptide or pharmaceutically acceptable salt thereof according to any one of claims 1, 2, 4, 6, 7, and 8, which has a cyclic structure in which a chloroacetylated amino acid is bonded to a cysteine residue included in the peptide.

11. The peptide or pharmaceutically acceptable salt thereof according to any one of claims 1, 3, 5, 6, and 7, which has a cyclic structure in which an amino group of an amino acid residue at position 1 included in the peptide is bonded to a carboxy group of an amino acid residue at position 15.

12. The peptide or pharmaceutically acceptable salt thereof according to any one of claims 1, 3, 5, 6, and 7, which has a cyclic structure in which an amino group of an amino acid residue at position 1 included in the peptide is bonded to a carboxy group of an amino acid residue at position 16.

13. The peptide or pharmaceutically acceptable salt thereof according to claim 9, wherein the peptide further comprises a crosslinking structure.

14. The peptide or pharmaceutically acceptable salt thereof according to claim 9, which has a crosslinking structure between any of X1, X2, or X3 and X11 or X13.

15. The peptide or pharmaceutically acceptable salt thereof according to claim 12, the peptide comprising or consisting of an amino acid sequence set forth in any one of SEQ ID NOs: 238-290, 326-329, 334-338, 348-354, 358-360, 362-366, and 368.

16. The peptide or pharmaceutically acceptable salt thereof according to any one of claims 1 to 8, further comprising an additional amino acid residue.

17. The peptide or pharmaceutically acceptable salt thereof according to any one of claims 1 to 8, comprising a linker at a C-terminus thereof.

18. A pharmaceutical composition comprising the peptide or pharmaceutically acceptable salt thereof according to any one of claims 1 to 8.

19. The pharmaceutical composition according to claim 18, having myostatin inhibitory activity.

20. The pharmaceutical composition according to claim 18, for preventing or treating a myostatin-related disease or a disease or symptom associated with reduced muscle function.

21. A method of inhibiting myostatin signaling, comprising using the peptide or pharmaceutically acceptable salt thereof according to any one of claims 1 to 8.

22. A diagnostic composition comprising the peptide or pharmaceutically acceptable salt thereof according to any one of claims 1 to 8, for diagnosing a myostatin-related disease or a disease or symptom associated with reduced muscle function.

23. A method of testing a peptide or a pharmaceutically acceptable salt thereof, comprising testing at least one of
a) solubility in a solvent,
b) myostatin inhibitory activity,
c) cell and/or tissue toxicity, or
d) toxicity to an experimental animal,
wherein the peptide or pharmaceutically acceptable salt thereof is the peptide or pharmaceutically acceptable salt thereof according to any one of claims 1 to 8.
